# EUROPÄISCHE PATENTSCHRIFT

(11) **EP 0 352 218 B1**
(45) Veröffentlichungstag und Bekanntmachung des Hinweises auf die Patenterteilung: **22.02.1995**
(21) Anmeldenummer: 89730165.1
(22) Anmeldetag: 18.07.1989
(51) Int. Cl.: C07D 471/08, C07D 498/08, C07D 255/04, C07D 513/08, C07D 519/00, C07F 5/00, C07F 13/00, C07F 15/00, A61K 49/00, A61K 51/00

(54) **5- oder 6-Ring-enthaltende makrocyclische Polyaza-Verbindungen, Verfahren zu ihrer Herstellung und diese enthaltende pharmazeutische Mittel**
Macrocyclic polyaza compounds containing rings with 5 or 6 members, methods for their preparation and pharmaceutical compositions containing same
Composés polyaza macrocycliques, leur procédé de préparation et compositions pharmaceutiques les contenant

(30) Priorität: 20.07.1988 DE 3825040
(43) Veröffentlichungstag der Anmeldung: 24.01.1990
(73) Patentinhaber: SCHERING AKTIENGESELLSCHAFT, 13342 Berlin (DE)
(72) Erfinder: Platzek, Johannes, Dr., D-1000 Berlin 33 (DE); Radüchel, Bernd, Dr., D-1000 Berlin 28 (DE); Gries, Heinz, Dr., D-1000 Berlin 31 (DE); Weinmann, Hanns-Joachim, Dr., D-1000 Berlin 38 (DE); Speck, Ulrich, Prof., D-1000 Berlin 28 (DE)

(56) Entgegenhaltungen:
- EP-A- 0 071 564
- EP-A- 0 203 047
- DE-A- 3 401 052
- TETRAHEDRON, Band 37, 1981, Seiten 767-772; H. STETTER et al.: "DarstellungundKomplexbildung von Polyazacycloalkan-N-Essigsäuren"

## Beschreibung

Die Erfindung betrifft den in den Patentansprüchen gekennzeichneten Gegenstand d.h. 5- oder 6-Ring enthaltende makrocyclische Polyaza-Komplexbildner,-Komplexe und -Komplexsalze, diese Verbindungen enthaltende Mittel, ihre Verwendung als Diagnostika und Therapeutika sowie Verfahren zur Herstellung dieser Verbindungen und Mittel.

Metallkomplexe sind schon zu Beginn der 50er Jahre als Kontrastmittel für die Radiologie in Betracht gezogen worden. Die damals eingesetzten Verbindungen waren aber derart toxisch, daß eine Verwendung beim Menschen nicht in Betracht kam. Es war daher durchaus überraschend, daß sich bestimmte Komplexsalze als ausreichend verträglich erwiesen haben, so daß eine routinemäßige Anwendung am Menschen für diagnostische Zwecke in Erwägung gezogen werden konnte. Als erster Vertreter dieser Substanzklasse hat sich das in der europäischen Patentanmeldung mit der Publikationsnummer 71564 beschriebene Dimegluminsalz des Gd DTPA (Gadolinium-III-Komplex der Diethylentriaminpentaessigsäure) als Kontrastmittel für die Kernspintomographie bisher in der klinischen Prüfung an über 7.000 Patienten sehr gut bewährt. Der Schwerpunkt der Anwendung liegt bei Erkrankungen des Zentralnervensystems.

Ein wesentlicher Grund für die gute Verträglichkeit von Gd DTPA in der klinischen Anwendung liegt in der hohen Wirksamkeit bei der Kernspintomographie, insbesondere bei vielen Hirntumoren. Wegen seiner guten Wirksamkeit kann Gd DTPA mit 0,1 mmol/kg Körpergewicht sehr viel niedriger dosiert werden als beispielsweise Röntgenkontrastmittel in vielen Röntgenuntersuchungen.

Als weiterer Vertreter der Komplexsalze hat sich das in der deutschen Patentanmeldung 34 01 052 beschriebene Megluminsalz des Gd DOTA (Gadolinium-III-Komplex der 1,4,7,10-Tetraazacyclododecan-tetraessigsäure) für diagnostische Zwecke bewährt.

Nun besteht aber der Wunsch, Chelate auch höher dosiert einzusetzen. Das ist besonders zum Nachweis bestimmter Erkrankungen außerhalb des Zentralnervensystems mit Hilfe der Kernspintomographie (NMR-Diagnostik), ganz besonders aber bei der Verwendung von Chelaten als Röntgenkontrastmittel, der Fall.

Um dabei die Volumenbelastung des Körpers möglichst gering zu halten, ist es notwendig hochkonzentrierte Chelatlösungen zu verwenden. Die bisher bekannten Chelate sind vor allem wegen ihrer zu hohen Osmolalität dafür wenig geeignet.

Es besteht daher ein Bedarf an Chelaten, die eine geringere Osmolalität als die vorbekannten Chelate aufweisen. Gleichzeitig müssen jedoch die Voraussetzungen für die Anwendung dieser Verbindungen am Menschen bezüglich des Abstands zwischen der wirksamen und der im Tierversuch toxischen Dosis (die therapeutische Breite), der Organspezifität, der Stabilität, der kontrastverstärkenden Wirkung, der Verträglichkeit sowie der Löslichkeit der Komplexverbindungen erfüllt sein.

Der Erfindung liegt somit die Aufgabe zugrunde, diese Verbindungen und Mittel zur Verfügung zu stellen, sowie ein möglichst einfaches Verfahren zu ihrer Herstellung zu schaffen.

Diese Aufgabe wird durch die vorliegende Erfindung erfüllt.

Die erfindungsgemäßen Komplexverbindungen und die aus ihnen bereiteten Lösungen erfüllen die genannten Anforderungen in überraschender Weise. Sie besitzen eine verringerte Osmolalität sowie eine günstigere therapeutische Breite und/oder Stabilität und Lagerfähigkeit der chemischen Bestandteile der Lösung und/oder Organspezifität und/oder kontrastverstärkende Wirkung (z.B. Relaxivität) und/oder Verträglichkeit (z.B. geringere kardiovaskuläre oder allergieartige Nebenwirkungen) als die bisher gebräuchlichen Diagnostika.

Auch ohne spezifische Maßnahmen erlaubt ihre Pharmakokinetik die Verbesserung der Diagnose zahlreicher Erkrankungen. Die Komplexe werden zum größten Teil unverändert und rasch wieder ausgeschieden, so daß insbesondere auch im Falle der Verwendung relativ toxischer Metallionen trotz hoher Dosierung keine schädlichen Wirkungen beobachtet werden.

Die praktische Anwendung der neuen Komplexe und Komplexbildner wird auch durch deren günstige chemische Stabilität erleichtert.

Ein weiterer wesentlicher Vorteil der beschriebenen Komplexe und Komplexbildner ist deren außerordentliche chemische Vielseitigkeit. Neben dem Zentralatom lassen sich die Eigenschaften durch die Wahl vielfältiger Substituenten, des 5- oder 6-Rings im Makrocyclus und/oder der Salzbildner den Anforderungen an Wirksamkeit, Pharmakokinetik. Verträglichkeit, Löslichkeit, Handhabbarkeit usw. anpassen. So kann z.B. eine in der Diagnostik und Therapie sehr erwünschte Spezifität der Verbindungen für Strukturen im Organismus, für bestimmte biochemische Substanzen, für Stoffwechselvorgänge, für Zustände der Gewebe oder Körperflüssigkeiten, erzielt werden.

Die erfindungsgemäßen makrocyclischen Verbindungen werden durch die allgemeine Formel I gekennzeichnet: 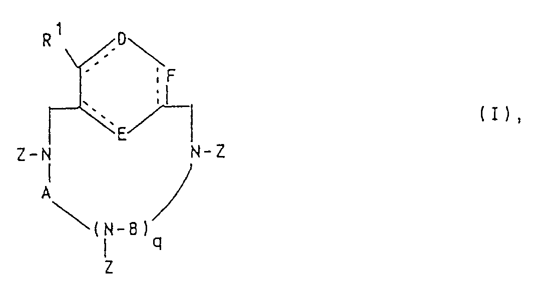
worin
- ....: für eine Einfach- oder Doppelbindung.
- q: für die Ziffern 0-5,
- A und B: , die gleich oder verschieden sind, jeweils für eine geradkettige oder verzweigte Alkylengruppe mit 2 bis 6 Kohlenstoffatomen.
- D: für ein Stickstoff-, Sauerstoffatom, die Gruppe =C=O, =NR², -CHR³ - oder =CR³-,
- E: für ein Stickstoff-, Schwefel-, Sauerstoffatom,
die 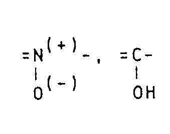 oder >NR⁴-Gruppe,
- F: für (-CHR⁸-)ₙ oder (=CR⁸)ₙ,
- R¹: für ein Wasserstoff- oder Halogenatom oder eine C₁-C₆-Alkylgruppe,
- Z: für ein Wasserstoffatom oder die Gruppe -CH₂COOY mit Y in der Bedeutung eines Wasserstoffatoms und/oder eines Metallionenäquivalents eines Elements der Ordnungszahlen 21-29, 31, 32, 37-39, 42-44, 49 oder 57-83,
- R²: für ein Wasserstoffatom oder eine C₁-C₆-Alkylgruppe,
- R³: für ein Wasserstoff- oder Halogenatom, eine Phenyl- oder eine C₁-C₆-Alkylgruppe, die gegebenenfalls durch eine oder mehrere Phenyl- und/oder Hydroxygruppe(n) substituiert ist, den Rest OR⁵, den Substituenten 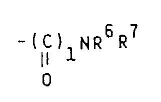 oder den Substitutenten G,
- R⁴: für eine Hydroxygruppe, für R² oder eine gegebenenfalls hydroxylierte oder carboxylierte C₁-C₆-Alkylgruppe,
- R⁵: für einen gegebenenfalls durch 1 bis 3 Hydroxygruppe(n) substituierten C₁-C₆-Alkylrest,
- R⁶ und R⁷: unabhängig voneinander für Wasserstoffatome, für den Rest R⁵, für gegebenenfalls durch 1 bis 3 Hydroxygruppen substituierte Phenyl- oder Benzylreste oder R⁶ und R⁷ gemeinsam mit dem Stickstoffatom für einen gesättigten oder ungesättigten, gegebenenfalls ein weiteres Stickstoff-, Sauerstoff-, Schwefelatom oder eine Carbonylgruppe enthaltenden 5- oder 6-Ring, der gegebenenfalls substituiert ist durch 1 bis 3 Reste R⁵, oder einer der Substituenten R⁶ oder R⁷ für den Rest 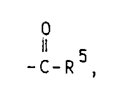
- R⁸: für R¹ oder G,
- l: für die Ziffern 0 oder 1,
- n: für die Ziffern 0 oder 1,
- G: für einen über eine direkte Bindung, eine Bis(carbonylamino)gruppe (-NH-CO-CO-NH-) oder über eine C₁-C₂₀-Alkylengruppe, die gegebenenfalls an den Enden Carbonyl-(>CO) oder Carbonylamino(-NH-CO)-gruppen oder Sauerstoffatome trägt und gegebenenfalls ein oder mehrere Sauerstoffatom(e), Z-, acyl- oder hydroxyacylsubstituierte Iminogruppen oder ein bis zwei C-C-Doppel- und/oder C-C-Dreifachbindungen enthält, gebundenen zweiten Makrocyclus der allgemeinen Formel II 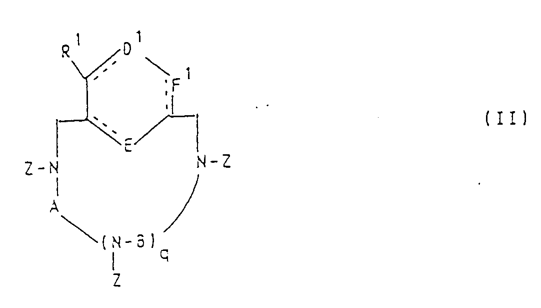 stehen,
worin
- D¹: die gleiche Bedeutung wie D hat, mit der Ausnahme, daß D¹ nicht den Substituenten G enthält, oder für den Rest 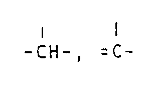 oder 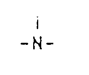 steht un
- F¹: die gleiche Bedeutung wie F hat, mit der Ausnahme, daß F¹ nicht den Substituenten G enthält, oder für den Rest 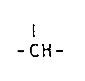 oder 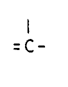 steht,
sowie deren Salze mit anorganischen und/oder organischen Basen, Aminosäuren oder Aminosäureamiden,
mit den Maßgaben, daß mindestens zwei der Substituenten Z für den Rest -CH₂COOY stehen, daß die makrocyclische Verbindung der allgemeinen Formel I nicht mehr als einen Rest G enthält und daß die allgemeine Formel I nicht für 3,6,9,12,18-Pentaazabicyclo[12.3.1]octadeca-1(18),14,16-trien-N-tetraessigsäure oder 3,6,9,15-Tetraazabicyclo[9.3.1]pentadeca-1(15),11,13-trien-N-triessigsäure oder deren Cu-, Pb-, Co- und Sr-Komplexe steht.

Steht n für die Ziffer 0 und soll der so gebildete 5-Ring ungesättigt sein, dann befinden sich die Doppelbindungen zwischen den Positionen 2,3 und 4,5 des 5-Rings.

Verbindungen der allgemeinen Formel I mit Y in der Bedeutung von Wasserstoff werden als Komplexbildner und mit mindestens zwei der Substituenten Y in der Bedeutung eines Metallionenäquivalents als Metallkomplexe bezeichnet.

Das Element der oben genannten Ordnungszahl, welches das Zentralion des physiologisch verträglichen Komplexsalzes bildet, kann für den angestrebten Verwendungszweck des erfindungsgemäßen diagnostischen Mittels selbstverständlich auch radioaktiv sein.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan (II)-, Eisen(II)-, Cobalt(II)-, Nickel(II)-, Kupfer(II)-, Praseodym(III)-, Neodym(III)-, Samarium(III)-und Ytterbium(III)-ion. Wegen ihres sehr starken magnetischen Moments sind besonders bevorzugt das Gadolinium(III)-, Terbium (III)-, Dysprosium(III)-, Holmium(III)-, Erbium(III)- und Eisen(III)-ion.

Für die Verwendung der erfindungsgemäßen Mittel in der Nuklearmedizin muß das Zentralion radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium und Iridium.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Als Alkylsubstituenten R¹, R², R³ und R⁴ kommen geradkettige oder verzweigte Kohlenwasserstoffe mit bis zu 6, bevorzugt bis zu 4, Kohlenstoffatomen, die im Falle von R³ gegebenenfalls durch eine oder mehrere, bevorzugt 1 bis 3, Phenyl- und/oder Hydroxygruppen, im Falle von R⁴ gegebenenfalls durch eine oder mehrere, bevorzugt 1 bis 3, Hydroxy- oder Carboxylgruppen und im Falle von R⁵ gegebenenfalls durch eine oder mehrere, bevorzugt 1 bis 3, Hydroxygruppen substituiert sind, in Frage.

Als gegebenenfalls substituierte Alkylgruppen seien beispielsweise die Methyl-, Hydroxymethyl-, Ethyl-, 2-Hydroxyethyl-, 2-Hydroxy-1-(hydroxymethyl)-ethyl-, 1-[Hydroxymethyl)-ethyl-, Propyl-, Isopropyl-, 2- und 3-Hydroxypropyl, 2,3-Dihydroxypropyl-, n-, sek.- und tert.-Butyl-, 2-, 3-, und 4-Hydroxybutyl-, 2- und 3-Hydroxy-isobutyl, Pentyl-, 2-,3- und 4-Hydroxy-2-methylbutyl-, 2,3,4-Trihydroxybutyl-, 1,2,4-Trihydroxybutyl-, Cyclopentyl-, Cyclohexyl-, 2,3,4,5,6-Pentahydroxyhexyl-, Benzyl-, Carboxymethyl- und Carboxyethylgruppe genannt.

Als in R¹ und R³ enthaltenes Halogenatom seien Fluor, Chlor, Brom und Jod genannt.

Der durch R⁶ und R⁷ unter Einschluß des Stickstoffatoms gebildete heterocyclische 5- oder 6-Ring kann gesättigt, ungesättigt und/oder substituiert sein und gegebenenfalls ein Stickstoff-, Sauerstoff-, Schwefelatom oder eine Carbonylgruppe enthalten.

Als geeignete Heterocyclen seien beispielsweise genannt: der Pyrrolidinyl-, Piperidyl-, Pyrazolidinyl-, Pyrrolinyl-, Pyrazolinyl-, Piperazinyl-, Morpholinyl-, Imidazolidinyl-, Oxazolidinyl-, Thiazolidinyl-, Pyrrolidonyl-Ring.

Als Alkylengruppe A und B kommen geradkettige oder verzweigte Ketten mit 2 bis 6 Kohlenstoffatomen, vorzugsweise die Ethylen-, Methylethylen- und die Propylengruppe in Frage.

Die Alkylenkette, an die der zweite Makrocyclus II gebunden ist, trägt an den Enden gegebenenfalls Carbonyl-(CO), Carbonylamino-(NH-CO)-gruppen oder Sauerstoffatome und enthält 1-20 Kohlenstoffatome. Sie kann durch ein oder mehrere Sauerstoffatom(e), Z-, acyl- oder hydroxyacyl-substituierte Iminogruppen oder ein bis zwei C-C-Doppel- und/oder C-C-Dreifachbindungen unterbrochen sein. Die beiden Makrocyclen können aber auch durch eine direkte Bindung verknüpft sein. Als gegebenenfalls hydroxylierte Acylgruppen kommen dabei Acyl-Reste mit bis zu 10 Kohlenstoffatomen in Frage. Beispielhaft genannt seien der Acetyl-, Propionyl-, Butyryl-, Benzoyl- und Hydroxyacetyl-Rest.

Die Alkylenkette kann gerad- oder verzweigtkettig, gesättigt oder ungesättigt und gegebenenfalls wie beschrieben unterbrochen sein. Sie kann bis zu 4 Sauerstoffatome und/oder bis zu 3 Carboxymethyliminogruppen enthalten.

Beispiele für die Alkylenkette sind:
-(CH₂)₂-, -CH₂-O-CH₂-, -(CH₂)₄-, -(CH₂-CH₂-O-CH₂-CH₂)-, -(CH₂-O-CH₂)₂-, -(CH₂-O-CH₂)₃-, -CH₂-CH₂-(O-CH₂-CH₂)₃-, -CH₂-CH₂-(O-CH₂-CH₂)₄-, 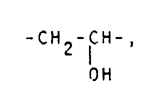 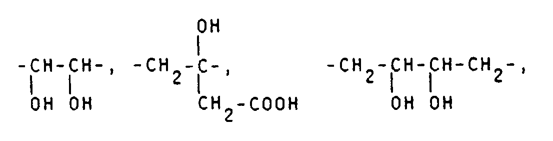
-C≡C-C≡C-, 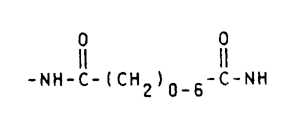 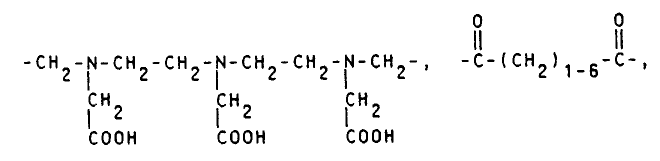
-O-(CH₂)₁₋₆-O-, 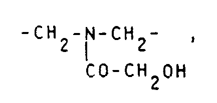
-(CH₂)₂CH=CH-(CH₂)₂-.

Wenn nicht alle aciden Wasserstoffatome durch das Zentralion substituiert werden, können ein, mehrere oder alle verbleibenden Wasserstoffatom(e) durch Kationen anorganischer und/oder organischer Basen oder Aminosäuren ersetzt sein. Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins.

Die Herstellung der erfindungsgemäßen makrocyclischen Komplexe der allgemeinen Formel I erfolgt dadurch, daß man in an sich bekannter Weise Verbindungen der allgemeinen Formel I' 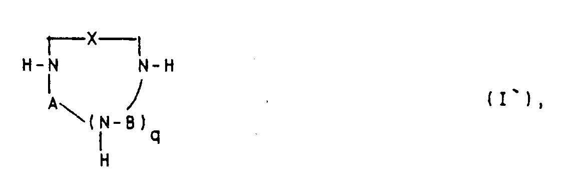
worin
X für 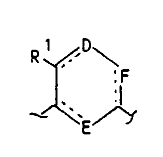
oder für einen in den gewünschten Ring umzuwandelnden 5- oder 6-Ring steht,
mit einer Halogenverbindung III

HalCH₂COOY' (III),

worin Hal für Chlor, Brom oder Jod und
- Y': für ein Wasserstoffatom oder eine Säureschutzgruppe steht,
alkyliert und anschließend, gegebenenfalls nach Umwandlung von X in den gewünschten 5- oder 6-Ring des Endprodukts sowie gegebenenfalls nach Abspaltung der Schutzgruppen Y', gewünschtenfalls die so erhaltenen Komplexbildner der allgemeinen Formel I mit Y in der Bedeutung von Wasserstoff in an sich bekannter Weise mit mindestens einem Metalloxid oder Metallsalz eines Elements der Ordnungszahlen 21-29, 31, 32, 37-39, 42-44, 49 oder 57-83 umsetzt und anschließend, falls gewünscht, noch vorhandene acide Wasserstoffatome durch Kationen anorganischer und/oder organischer Basen, Aminosäuren oder Aminosäureamide substituiert.

Als Säureschutzgruppen Y' kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen in Frage.
Die Abspaltung der Schutzgruppen Y', die vor oder nach Umwandlung von X in den gewünschten 5- oder 6-Ring des Endprodukts durchgeführt werden kann, erfolgt nach den dem Fachmann bekannten verfahren, beispielsweise durch Hydrolyse, Hydrogenolyse, alkalische Verseifung der Ester mit Alkali in wäßrig-alkoholischer Lösung bei Temperaturen von 0 bis 50°C oder im Fall von z.B. tert.-Butylestern mit Hilfe von Trifluoressigsäure.

Die Alkylierung der Edukte I' mit den Halogenverbindungen der allgemeinen Formel III erfolgt in polaren aprotischen Lösungsmitteln wie zum Beispiel Dimethylformamid, Acetonitril, Dimethylsulfoxid, wäßriges Tetrahydrofuran oder Hexamethylphosphorsäuretriamid in Gegenwart eines Säurefängers, wie zum Beispiel tertiäres Amin (zum Beispiel Triäthylamin, Trimethylamin, N,N-Dimethylaminopyridin, 1,5 Diazabicyclo [4.3.0] nonen-5[DBN], 1,5 Diazabicyclo [5.4.0] undecen-5 (DBU) Alkali-, Erdalkalicarbonat, -hydrogencarbonat oder -hydroxid (zum Beispiel Natrium-, Magnesium-, Calcium-, Barium-, Kalium-carbonat, -hydroxid und -hydrogencarbonat) bei Temperaturen zwischen -10°C und 120°C, vorzugsweise zwischen 0°C und 50°C.

Die Umwandlung einer Vorstufe des im Endprodukt enthaltenen gewünschten 5- oder 6-gliedrigen Ringes erfolgt nach den dem Fachmann bekannten Methoden Beispielhaft erwähnt seien die Hydrierung von z.B. Pyridin-[Advan. Catal. 14, 203 (1963)], Pyrrol-[M. Freifelder, Practical Catalytic Hydrogenation, 577, Wiley-Interscience, New York-London-Sydney-Toronto 1971], Furan-[US-3,194,818], Pyrimidin- [J. Med.Chem. 15, 291 (1972)], Deoxygenierung von Nitroxid- [E. Klingsberg, The Chemistry of Heterocyclic Compounds, Volume 14, part 2, Interscience Publishers New York, p. 120 (1961)] -ringen, Umwandlungen und Einführung von funktionellen Gruppen am 5- oder 6-Ring, z.B. Freisetzung von phenolischen Hydroxygruppen [J. Org. Chem. 53, 5 (1988)], Einführung von Halogensubstituenten [E. Klingsberg, The Chemistry of Heterocyclic Compounds, Volume 14, Part 2, Interscience Publishers New York, p. 341 [1961), Houben-Weyl, Methoden der organischen Chemie, Band V/3, 651 (1962)], Austausch von Heteroatomen, z.B. Umwandlung eines Furans in ein Pyrrol (US-2,478,456).

Die Funktionalisierung von 4-Chlorpyridinderivaten (z.B. Azidaustausch) im Phasentransferverfahren unter Verwendung von 18-Krone-6 oder Tetrabutylammoniumbromid als Katalysator ist in "Phase Transfer Reactions" (Fluka Compendium Vol. 2; Walter E. Keller, Georg Thieme Verlag Stuttgart, New York) beschrieben.

Die Herstellung von Amiden, d.h. von Verbindungen der allgemeinen Formel I, worin R³ für 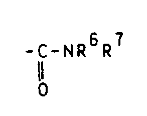
steht, erfolgt durch Umsetzung von aktivierten Säurederivaten (z.B. gemischtes Anhydrid, Säurechlorid) mit primären oder sekundären Aminen der allgemeinen Formel 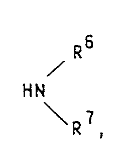
worin R⁶ und R⁷ die oben genannte Bedeutung haben.

Als geeignete Amine seien beispielsweise genannt:
Dimethylamin, Diethylamin, Di-n-propylamin, Diisopropylamin, Di-n-butylamin, Diisobutylamin, Di-sek.butylamin, N-Methyl-n-propylamin, Dioctylamin, Dicyclohexylamin, N-Ethylcyclohexylamin, Diisopropenylamin, Benzylamin, Anilin, 4-Methoxyanilin, 4-Dimethylaminoanilin, 3,5-Dimethoxyanilin, Morpholin, Pyrrolidin, Piperidin, N-Methylpiperazin, N-Ethylpiperazin, N-(2-Hydroxyethyl)piperazin, N-(Hydroxymethyl)piperazin, 2-(2-Hydroxymethyl)-piperidin, 4-(2-Hydroxyethyl)-piperidin, 2-Hydroxymethylpiperidin, 4-Hydroxymethylpiperidin, 2-Hydroxymethyl-pyrrolidin, 3-Hydroxy-piperidin, 4-Hydroxypiperidin, 3-Hydroxy-pyrrolidin, 4-Piperidon, 3-Pyrrolin, 2,6-Dimethylpiperidin, 2,6-Dimethylmorphoiin, Pyrazolin, Imidazolin, Oxazolidin, Thiazolidin, 2,3-Dihydroxypropylamin, N-Methyl-2,3-dihydroxypropylamin, 2-Hydroxy-1-(hydroxymethyl)-ethylamin, N,N-Bis-(2-hydroxyethyl)-amin, N-Methyl-2,3,4,5,6-pentahydroxyhexylamin, 6-Amino-2,2-dimethyl-1,3-dioxepin-5-ol, 2-Hydroxyethylamin, 2-Amino-1,3-propandiol, Diethanolamin, Ethanolamin.

Die Polyhydroxyalkylamine können vorteilhafterweise auch in geschützter Form zur Reaktion eingesetzt werden, zum Beispiel als 0-Acylderivate oder als Ketale. Dies gilt besonders dann, wenn diese Derivate bequemer und billiger herstellbar sind als die Polyhydroxyalkylamine selbst. Ein typisches Beispiel ist das 2-Amino-1-(2,2-dimethyl-1,3-dioxolan-4-yl)-ethanol, das Acetonid des 1-Amino-2,3,4-trihydroxybutans, hergestellt nach DE-OS 31 50 917.

Die nachträgliche Entfernung der Schutzgruppen ist problemlos und kann zum Beispiel durch Behandlung mit einem sauren Ionenaustauscher in wäßrig-ethanolischer Lösung erfolgen.

Die Synthese von Verbindungen mit mehr als einem Ring erfolgt nach literaturbekannten Verfahren, zum Beispiel über eine Additions/Eliminierungs-Reaktion eines Amins mit einer Carbonylverbindung (zum Beispiel Säurechlorid, gemischtes Anhydrid, aktivierter Ester, Aldehyd); zweier aminsubstituierter Ringe mit einer Dicarbonylverbindung (zum Beispiel Oxalylchlorid, Glutardialdehyd); zweier p-Nitrosubstituierter Nitroxide mit Bisalkoholaten [ vergl. E. Klingsberg, The Chemistry of Heterocyclic Compounds, Interscience Publishers New York, p. 514 (1961)]; zweier Ringe, die je eine nukleophile Gruppe aufweisen, mit einer zwei Fluchtgruppen tragenden Alkylenverbindung oder im Falle terminaler Acetylene durch oxidative Kupplung (Cadiot, Chodkiewicz in Viehe "Acetylenes", 597-647, Marcel Dekker, New York, 1969).

Die die Ringe verknüpfende Kette kann anschließend durch Folgereaktionen modifiziert werden (zum Beispiel Hydrierung).

Die Synthese der zu alkylierenden Edukte der allgemeinen Formel I' erfolgt durch Cyclisierung zweier Reaktanten, von denen der eine den Substituenten X, d.h. den gewünschten 5- oder 6-gliedrigen Ring des Endprodukts oder eine in diesen umzuwandelnde Vorstufe enthält.

Die Cyclisierung wird nach literaturbekannten Methoden, (zum Beispiel Org. Synth. 58,86 (1978), Makrocyclic Polyether Syntheses, Springer Verlag Berlin, Heidelberg, New York 1982, Coord.Chem.Rev. 3,3 (1968), Ann. Chem. 1976,916), J. Org. Chem. 49,110[1984]) durchgeführt: einer der beiden Reaktanten trägt am Kettenende zwei Fluchtgruppen, der andere zwei Stickstoffatome, die nukleophil diese Fluchtgruppen verdrängen. Als Beispiel seien genannt die Umsetzung von endständigen, den Substituenten X und gegebenenfalls ein bis fünf Stickstoffatom(e) enthaltenden, Dichlor-, Dibrom-, Dimesyloxy-, Ditosyloxy- oder Dialkoxycarbonylalkylenverbindungen mit endständigen, gegebenenfalls ein bis fünf zusätzliche Stickstoffatom(e) in der Alkylenkette enthaltenden Diazaalkylenverbindungen. Der Substituent X kann stattdessen auch in dem zweiten Reaktanten, d.h. dem mit den endständigen nukleophilen Stickstoffatomen, enthalten sein. Die Stickstoffatome sind gegebenenfalls geschützt, zum Beispiel als Tosylate oder Trifluoracetate und werden vor der nachfolgenden Alkylierungsreaktion nach literaturbekannten Verfahren freigesetzt (die Tosylate z.B. mit Mineralsäuren, Alkalimetallen in flüssigem Ammoniak, Bromwasserstoffsäure und Phenol, RedAl^{(R)}, Lithiumaluminiumhydrid, Natrium-Amalgam, vgl. z.B. Liebigs Ann. Chem. 1977, 1344, Tetrahedron Letters 1976, 3477; die Trifluoracetate z.B. mit Mineralsäuren oder Ammoniak in Methanol, vgl. z.B. Tetrahedron Letters 1967, 289).

Zur Herstellung von an den Stickstoffatomen unterschiedlich (Wasserstoff oder die Gruppe CH₂COOY) substituierten Makrocyclen können diese Atome in den Edukten mit unterschiedlichen Schutzgruppen, z.B. mit Tosylat- und Benzylgruppen, versehen werden. Letztere werden dann ebenfalls nach literaturbekannten Methoden (bevorzugt durch Hydrierung, z.B. EP-Patentanmeldung 232751) entfernt.

Werden Diester in die Cyclisierungsreaktion eingesetzt, so müssen die so erhaltenen Diketoverbindungen nach dem Fachmann bekannten Verfahren, zum Beispiel mit Diboran, reduziert werden.

Es können auch entsprechend substituierte endständige Bisaldehyde mit den jeweils gewünschten endständigen Bisaminen cyclisiert werden; die Reduktion der so erhaltenen Schiffschen Basen erfolgt nach literaturbekannten Methoden, z.B. durch katalytische Hydrierung [Helv. Chim. Acta 61,1376 (1978)].

Die Herstellung der als Ausgangssubstanzen für die Cyclisierung benötigten Amine erfolgt analog literaturbekannten Methoden.

Ausgehend von einer N-geschützten Aminosäure erhält man durch Umsetzung mit einem partiell geschützten Diamin (zum Beispiel nach der Carbodiimidmethode), Abspaltung der Schutzgruppen und Diboranreduktion ein Triamin.

Die Umsetzung eines aus Aminosäuren erhältlichen Diamins (Eur. J. Med. Chem.-Chim. Ther. 21,333 (1986)) mit der doppelten molaren Menge einer N-geschützten ω-Aminosäure liefert ein Tetramin nach geeigneter Aufarbeitung.

Die gewünschten Diamine können auch durch Gabriel-Reaktion aus z.B. den entsprechenden Tosylaten oder Halogeniden hergestellt werden [vgl. z.B. Inorg. Chem. 25, 4781 (1986)].

In beiden Fällen ist die Anzahl der Kohlenstoffatome zwischen den N-Atomen durch die Art der als Kopplungspartner eingesetzten Diamine bzw. Aminosäuren bestimmbar.

Die so erhaltenen Verbindungen der allgemeinen Formel I mit Y in der Bedeutung eines Wasserstoffatoms stellen Komplexbildner dar. Sie können isoliert und gereinigt werden oder ohne Isolierung in Metallkomplexe der allgemeinen Formel I mit mindestens zwei der Substituenten Y in der Bedeutung eines Metallionenäquivalents überführt werden.

Die Herstellung der erfindungsgemäßen Metallkomplexe erfolgt in der Weise, wie sie in der Patentschrift DE-OS 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21-29, 31, 32, 37-39, 42-44, 49, 57-83 in Wasser und/oder einem niederen Alkohol (wie Methanol, Ethanol oder Isopropanol) löst oder suspendiert und mit der Lösung oder Suspension der äquivalenten Menge der komplexbildenden Säure der allgemeinen Formel I mit Y in der Bedeutung eines Wasserstoffatoms umsetzt, vorzugsweise bei Temperaturen zwischen 40 und 100°C, und anschließend, falls gewünscht, vorhandene acide Wasserstoffatome von Säuregruppen durch Kationen anorganischer und/oder organischer Basen, Aminosäuren oder Aminosäureamide substituiert.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl und N,N-Dimethylglucamin, sowie basischer Aminosäuren, wie zum Beispiel Lysin, Arginin und Ornithin.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol. Ethanol, Isopropanol und anderen), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man die komplexbildende Säure in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Im Falle der Verwendung von Radioisotope enthaltenden Komplexverbindungen kann deren Herstellung nach den in "Radiotracers for Medical Applications", Volume 1 CRC-Press, Boca Raton, Florida beschriebenen Methoden vorgenommen werden.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 1»Mol - 1 Mol/l des Komplexsalzes und werden in der Regel in Mengen von 0,001 - 5 mMol/kg dosiert. Sie sind zur enteralen und parenteralen Applikation bestimmt.
Die erfindungsgemäßen Komplexverbindungen kommen zur Anwendung
1. für die NMR-und Röntgen-Diagnostik in Form ihrer Komplexe mit den Ionen der Elemente mit den Ordnungszahlen 21-29, 42, 44 und 57-83;
2. für die Radiodiagnostik und Radiotherapie in Form ihrer Komplexe mit den Radioigotopen der Elemente mit den Ordnungszahlen 27, 29, 31, 32, 37-39, 43, 49, 62, 64, 70 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100 bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,001 - 5 mMol/kg, vorzugsweise 0,005 - 0,5 mMol/kg, dosiert. Details der Anwendung werden zum Beispiel in H.J. Weinmann et al., Am J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts- Reagenzien und als shift-Reagenzien für die in-vivo NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁶⁸Ga verwendet. (Heiss, W.D., Phelps, M.E., Positron Emission Tomography of Brain, Springer Verlag Berlin, Heidelberg, New York 1983).

Die erfindungsgemäßen Verbindungen können auch in der Radioimmuno- oder Strahlentherapie verwendet werden. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Geeignete β-emittierende Ionen sind, zum Beispiel ⁴⁶Sc, ⁴⁷Sc, ⁴⁸Sc, ⁷²Ga und ⁷³Ga. Geeignete geringe Halbwertszeiten aufweisende α-emittierende Ionen sind zum Beispiel ²¹¹Bi, ²¹²Bi, ²¹³Bi und ²¹⁴Bi, wobei ²¹²Bi bevor zugt ist. Ein geeignetes Photonen- und Elektronenemittierendes Ion ist ¹⁵⁸Gd, das aus ¹⁵⁷Gd durch Neutroneneinfang erhalten werden kann.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.V. appliziert.

Details der Anwendung von Radiotherapeutika werden z. B. in R.W. Kozak et al., TIBTEC, Oktober 1986, 262 diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 -5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler - "Einführung in die Röntgendiagnostik", G. Thieme, Stuttgart, New York (1977) diskutiert.

Insgesamt ist es gelungen, neue Komplexbildner, Metallkomplexe und Metallkomplexsalze zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstandes.

### BEISPIEL 1

### a) 3,6,9-Tris(p-tolylsulfonyl)-3,6,9,15-tetraazabicyclo[9.3.1]pentadeca-1(15),-11,13-trien

Zu 121,9 g (200 mol) N,N',N''-Tris(p-tolylsulfonyl)-diethylentriamin-N,N''-dinatriumsalz in 1600 ml Dimethylformamid wird bei 100°C eine Lösung aus 35,2 g (200 mol) 2,6-Bis-(chlormethyl)-pyridin (gelöst in 700 ml Dimethylformamid) innerhalb 3 Stunden zugetropft. Man läßt über Nacht bei 100°C rühren. In die heiße Lösung tropft man 2 l Wasser und läßt auf 0°C abkühlen. Der Niederschlag wird abgesaugt und mit Wasser gewaschen. Nach Trocknung im Vakuum (60°C) wird aus Acetonitril umkristallisiert. Man erhält 92,3 g ( 69% der Theorie) der Titelverbindung als farbloses Pulver.

| | | | | | |
|---|---|---|---|---|---|
| Analyse: Ber.: | C 57,46 | H 5,43 | N 8,38 | O 14,35 | S 14,38 |
| Gef. : | C 51,39 | H 5,48 | N 8,35 | | S 14.34 |

### b) 3,6,9,15-Tetraazabicyclo[9.3.1]pentadeca-1(15),11,13-trien Tetrahydrosulfat

90,3 g (135 mmol) der Titelverbindung aus Beispiel 1a werden in 270 ml konz. Schwefelsäure eingetragen und 48 Stunden bei 100°C gerührt. Man kühlt auf 0°C und tropft 1,35 l absoluten Ether hinzu. Der Niederschlag wird abgesaugt und in 800 ml Methanol ausgerührt. Nach Filtrieren und Einengen trocknet man im Vakuum bei 50°C.

Ausbeute: 42,6 g (52,7% der Theorie) eines an der Luft zerlaufenden Feststoffes.

| | | | | | |
|---|---|---|---|---|---|
| Analyse: Ber.: | C 22,07 | H 4,38 | N 9,36 | O 42,76 | S 21,43 |
| Gef.: | C 22,10 | H 4,42 | N 9,31 | | S 21.40 |

### c) 3,6,9,15-Tetraazabicyclo[9.3.1]pentadeca-1(15),11,13-trien

40,0 g (66,8 mmol) der Titelverbindung aus Beispiel 1c werden in 100 ml Wasser gelöst und mit 32%iger Natronlauge auf pH 11 eingestellt. Man extrahiert 8 mal mit 150 ml Methylyenchlorid und trocknet über Magnesiumsulfat. Nach Eindampfen im Vakuum erhält man 9,79 g (71% der Theorie) eines gelblichen Pulvers.

| | | | |
|---|---|---|---|
| Analyse: Ber.: | C 64,04 | H 8,79 | N 27,16 |
| Gef.: | C 63,91 | H 8,85 | N 26,98 |

### d) 3,6,9-Tris-(carboxymethyl)-3,6,9,15-tetraazabicyclo[9.3.1]pentadeca-1(15),-11,13-trien

33,1 g (55,1 mmol) der Titelverbindung aus Beispiel 1c werden in 170 ml Wasser mit 6 n Kalilauge auf pH 8,5 eingestellt. Zu dieser Lösung gibt man 20,84 g (220,5 mmol) Chloressigsäure, stellt mit 6 n Kalilauge auf pH 9,5 und erwärmt auf 45°C. Man rührt 12 Stunden bei dieser Temperatur und hält den pH-Wert zwischen 9,5-10 durch Zugabe von 6 n Kalilauge. Nach Abkühlen auf Raumtemperatur stellt man mit konz. Salzsäure auf pH 2 und dampft zur Trockne ein. Der Rückstand wird mit 300 ml Ethanol/50ml Aceton extrahiert, der Feststoff abfiltriert und das Filtrat im Vakuum eingedampft. Der Rückstand wird in wenig Wasser gelöst und auf eine Kationenaustauschersäule (IR 120) gegeben. Nach Spülung mit Wasser wird der Ligand mit 0,5 n wäßriger Ammoniak-Lösung eluiert. Die Fraktionen werden eingedampft, mit wenig Wasser aufgenommen und über eine Anionenaustauschersäule (IRA 67) gegeben. Man wäscht zuerst mit Wasser und eluiert dann mit 0,5 n Ameisensäure. Man dampft im Vakuum ein und löst den Rückstand in wenig heißem Ethanol. Durch vorsichtige Zugabe von Aceton und Kühlung im Eisbad kristallisiert die Titelverbindung aus.

Ausbeute: 12,37 g (59% der Theorie) einer stark hygroskopischen Verbindung.

| | | | | |
|---|---|---|---|---|
| Analyse: Ber.: | C 53,67 | H 6,36 | N 14,13 | O 25,24 |
| Gef.: | C 53,55 | H 6,43 | N 14,65 | |

### e) Gadolinium-Komplex von 3,6,9-Tris-(carboxymethyl)-3,6,9,15-tetraazabicyclo-[9.3.1]pentadeca-1(15),11,13-trien

5,0 g (13,14 mmol) der Titelverbindung aus Beispiel 1d werden in 20 ml entionisiertem Wasser gelöst und 2,38 g (6,57 mmol) Gadoliniumoxid zugesetzt. Man rührt 3 Stunden bei 90°C. Die Lösung wird filtriert und das Filtrat gefriergetrocknet.

Ausbeute: 7,74 g ( 100% der Theorie) eines weißen amorphen Pulvers, das laut Analyse 9,31% Wasser enthält.

| | | | | | |
|---|---|---|---|---|---|
| Analyse: Ber.: | C 38,19 | H 3,96 | N 10,46 | O 17,98 | Gd 29,41 |
| Gef.: | C 38,11 | H 4,05 | N 10,38 | | Gd 29,32 |

Es wurde folgende Relaxivität im Plasma gemessen (die Messungen der Relaxationszeiten T₁ erfolgten in einem Minispec p20 (Bruker) bei 0,46 Tesla (=20MHz) bei 40°C):
T₁-Relaxivität: 7,64 (L/mmol sec)
Im Vergleich: Dimegluminsalz des Gadoliniumkomplexes der
Diethylentriaminpentaessigsäure (Gd-DTPA): T₁-Relaxivität: 4,95 (L/m mol sec)
Osmolalität einer 0,5 molaren Lösung bei 37°C: 0,55 (Osml/kg Wasser)
Zum Vergleich Dimegluminsalz von Gd-DTPA: 1,1 (Osml/kg Wasser).

In analoger Weise erhält man mit Eisen-III-oxid, Fe₂O₃, den Eisen-III-Komplex von 3,6,9-Tris(carboxymethyl)-3,6,9,15-tetraazabicyclo-[9.3.1]-pentadeca-1(15),11,13-trien als braunes Pulver.

| Analyse (bezogen auf wasserfreie Substanz) | | | | |
|---|---|---|---|---|
| Ber.: | C 47,13 | H 4,89 | N 12,83 | Fe 12,89 |
| Gef.: | C 47,04 | H 4,96 | N 12,84 | Fe 12,81 |

T₁-Relaxivität (L/mMol.sec), 40°C, Wasser, 20 MHZ: 0,49

### f) Dysprosium-Komplex von 3,6,9-Tris-(carboxymethyl)-3,6,9,15-tetraazabicyclo-[9.3.1]pentadeca-1(15),11,13-trien

Verwendet man anstelle von Gadoliniumoxid im Beispiel 1e Dysprosiumoxid so erhält man die Titelverbindung praktisch in quantitativer Ausbeute.

| | | | | | |
|---|---|---|---|---|---|
| Analyse: Ber.: | C 37,82 | H 3,92 | N 10,38 | O 17,78 | Dy 30,10 |
| Gef.: | C 37,87 | H 3,98 | N 10,24 | | Dy 30,02 |

### g) Ytterbium-Komplex von 3,6,9-Tris-(carboxymethyl)-3,6,9,15-tetraazabicyclo-[9.3.1]pentadeca-1(15),11,13-trien

Verwendet man anstelle von Gadoliniumoxid im Beispiel 1e Ytterbiumoxid so erhält man die Titelverbindung praktisch in quantitativer Ausbeute.

| | | | | | |
|---|---|---|---|---|---|
| Analyse: Ber.: | C 37,09 | H 3,85 | N 10,18 | O 17,44 | Yb 31,44 |
| Gef.: | C 37,13 | H 3,94 | N 10,09 | | Yb 31,37 |

### h) Megluminsalz des Mangan (II)-Komplexes von 3,6,9-Tris-(carboxymethyl)-3,6,9,15-tetraazabicyclo-[9.3.1]pentadeca-1(15),-11,13-trien

3,0 g (7,89 mmol) der Titelverbindung aus Beispiel 1d werden in 20 ml entionisiertem Wasser gelöst und 907 mg (7,89 mmol) Mangan (II)-carbonat zugegeben. Man rührt 2 Stunden bei 80°C. Die Lösung wird filtriert und das Filtrat mit einer 1 molaren N-Methylglucaminlösung auf pH 7,2 gestellt. Anschließend wird gefriergetrocknet.
Ausbeute: 5,56 g (100% der Theorie) eines leicht rosa scheinenden amorphen Pulvers, das laut Analyse 12,2% Wasser enthält.

| | | | | | |
|---|---|---|---|---|---|
| Analyse: Ber.: | C 45,86 | H 6,25 | N 11,14 | O 28,00 | Mn 8,74 |
| Gef.: | C 45,98 | H 6,21 | N 11,08 | | Mn 8,68 |

### BEISPIEL 2

### a) 3,6,9-Tris(carboxymethyl)-3,6,9,15-tetraazabicyclo[9.3.1]pentadecan

6,0 g (15,77 mmol) der Titelverbindung aus Beispiel 1d werden in 200 ml 5%iger Salzsäure gelöst und in einem Autoklaven über einem Rhodium-Katalysator (5% Rh/C) bei 30 Bar, 45°C hydriert. Nach 4 Stunden filtriert man vom Katalysator ab und dampft im Vakuum ein. Der Rückstand wird wie in Beispiel 1d beschrieben an Ionenaustauschern gereinigt. Kristallisation aus Methanol/Aceton ergibt 4,75 g (78% der Theorie) einer extrem hygroskopischen Verbindung.

| | | | | |
|---|---|---|---|---|
| Analyse: Ber.: | C 52,83 | H 7,83 | N 14,50 | O 24,84 |
| Gef.: | C 52,94 | H 1,89 | N 14,37 | |

### b) Gadolinium-Komplex von 3,6,9-Tris(carboxymethyl)-3,6,9,15-tetraazabicyclo-[9.3.1]pentadecan

Zu 4,3 g (11,13 mmol)) der Titelverbindung aus Beispiel 2a in 20 ml entionisiertem Wasser gibt man 2,02 g (5,57 mmol) Gadoliniumoxid und rührt 3 Stunden bei 90°C. Die Lösung wird filtriert und das Filtrat gefriergetrocknet. Man erhält 6,5 g (100% der Theorie) eines weißen, flockigen Pulvers, das laut Analyse 10,3% Wasser enthält.

| | | | | | |
|---|---|---|---|---|---|
| Analyse: Ber.: | C 31,76 | H 5,03 | N 10,36 | O 17,76 | Gd 29,08 |
| Gef.: | C 37,63 | H 5,12 | N 10,33 | | Gd 28,97 |

### BEISPIEL 3

### a) 3,6,9-Tris(acetyl)-3,6,9,15-tetraazabicyclo[9.3.1]pentadeca-1(15),11,13-trien

15,8 g (76,6 mmol) der Titelverbindung aus Beispiel 1c, 42,7 ml Triethylamin (306,4 mmol) und 50 mg Dimethylaminopyridin (DMAP) werden in 300 ml absolutem Methylenchlorid gelöst. Man gibt 28,9 ml (306,4 mmol) Essigsäureanhydrid zu und rührt über Nacht bei Raumtemperatur. Das Solvens wird im Vakuum eingedampft und der Rückstand mit 200 ml 3%iger Natriumcarbonatlösung aufgenommen. Man extrahiert 2 mal mit 150 ml Methylenchlorid. Nach Trocknen der organischen Phase über Magnesiumsulfat dampft man im Vakuum ein. Der Rückstand wird aus Ether/Essigester umkristallisiert. Man erhält 23,93 g (94% der Theorie) der Titelverbindung als weiße Blättchen.

| | | | | |
|---|---|---|---|---|
| Analyse: Ber.: | C 61,42 | H 7,28 | N 16,86 | 0 14,44 |
| Gef.: | C 61,48 | H 7,37 | N 16,80 | |

### b) 3,6,9-Tris(acetyl)-3,6,9,15-tetraazabicyclo[9.3.1]pentadeca-1(15),11,13-trien-15-N-oxid

22,5 g (67,7 mmol) der Titelverbindung aus Beispiel 3a werden in 100 ml Eisessig gelöst. Man fügt 7,7 ml einer 30%igen Wasserstoffperoxidlösung zu und erwärmt 4 Stunden auf 70°C. Dann werden weitere 3,9 ml 30%iger Wasserstoffperoxidlösung zugesetzt und eine weitere Stunde bei 70°C gerührt. Man engt im Vakuum auf ein Drittel ein und versetzt vorsichtig mit gesättigter Natriumcarbonatlösung bis zur alkalischen Reaktion. Man extrahiert zweimal mit 250 ml Methylenchlorid und trocknet dann die organischen Phasen über Magnesiumsulfat. Nach Eindampfen im Vakuum und Kristallisation aus Ether/Essigester erhält man 18,63 g (79% der Theorie) der Titelverbindung als kristallines Pulver.

| | | | | |
|---|---|---|---|---|
| Analyse: Ber.: | C 58,60 | H 6,94 | N 16,08 | O 16,07 |
| Gef.: | C 58,47 | H 6,88 | N 16,14 | |

### c) 13-Nitro-3,6,9-tris(acetyl)-3,6,9,15-tetraazabicyclo[9.3.1]pentadeca-1(15), 11,13-trien-15-N-oxid

17g (48,8 mmol) der Titelverbindung aus Beispiel 3b werden in 40 ml 90%iger Schwefelsaure gelost und auf 60°C erwarmt. Zu dieser Losung tropft man 14 ml konz. Salpetersaure (d=1,36) und rührt 3 Stunden bei 60°C. Es wird auf Eis gegossen, der Niederschlag filtriert und mit viel Wasser gewaschen. Nach Trocknung im Vakuum erhalt man ein orangenes Pulver, das aus Aceton umkristallisiert wird.
Ausbeute: 9,2 g (48% der Theorie) gelbe Rhomben.

| | | | | |
|---|---|---|---|---|
| Analyse: Ber.: | C 51,90 | H 5,89 | N 17,80 | O 24,40 |
| Gef.: | C 52,01 | H 5,76 | N 17,64 | |

### d) 13,13'-Ethylendioxy-bis[3,6,9-tris(acetyl)-3,6,9,15-tetraazabicyclo[9.3.1]-pentadeca-1(15),11,13-trien-15-N-oxid]

Eine frisch zubereitete Losung von Ethylenglycol-dinatrium in Dimethylformamid (hergestellt aus 620 mg Ethandiol und 600 mg Natriumhydrid [80%ige Suspension in Parafinol] in 15 ml wasserfreiem Dimethylformamid) wird innerhalb 10 Minuten zu einer 50°C warmen Losung aus 8 g (20.34 mmol) der Titelverbindung aus Beispiel 3c zugetropft und über Nacht bei dieser Temperatur geruhrt. Man gibt 10 ml Wasser zu und dampft im Vakuum ein. Der Rückstand wird an Kieselgel chromatographiert (Laufmittel: Methanol/32%ige wäßrige Ammoniaklosung = 10/1). Nach Kristallisation aus Ether/Essigester erhält man 3,15 g (41% der Theorie) eines gelblichen kristallinen Pulvers.

| | | | | |
|---|---|---|---|---|
| Analyse: Ber.: | C 57,28 | H 6,68 | N 14,85 | O 21,19 |
| Gef.: | C 57.40 | H 6,61 | N 14,79 | |

### e) 13,13'-Ethylendioxy-bis[3,6,9-tris(acetyl)-3,6,9,15-tetraazabicyclo[9.3.1]-pentadeca-1(15),11,13-trien]

3 g (3,97 mmol) der Titelverbindung aus Beispiel 3d werden in 100 ml Ethanol gelöst, 1 ml konz. Salzsäure zugesetzt und über Pd/C hydriert. Man filtriert vom Katalysator ab, dampft im Vakuum ein und nimmt den Rückstand mit 50 ml 3%iger Natriumcarbonat-Lösung auf. Es wird zweimal mit 100 ml Methylenchlorid extrahiert. Nach Trocknen der organischen Phasen über Magnesiumsulfat zieht man das Solvens im Vakuum ab und kristallisiert aus Ether/Essigester.
Ausbeute: 2,87 g (87% der Theorie) weiße Blättchen

| | | | | |
|---|---|---|---|---|
| Analyse: Ber.: | C 59,81 | H 6,97 | N 15,50 | O 17,71 |
| Gef.: | C 59,70 | H 6,91 | N 15,39 | |

### f) 13,13'-Ethylendioxy-bis[3,6,9,15-tetraazabicyclo[9.3.1]pentadeca-1(15),11,-13-trien] 2,5 g (3,46 mmol) der Titelverbindung aus Beispiel 3e und 4,66 g (41,5 mmol) Kalium-tert.-butylat werden in 40 ml Dioxan unter Stickstoff gelöst und über Nacht unter Rückfluß erhitzt. Man dampft im Vakuum ein, nimmt den Rückstand mit 50 ml Wasser auf und bringt mit 2 n Natronlauge auf pH 10. Nach 6 maliger Extraktion mit je 80 ml Methylenchlorid trocknet man über Magnesiumsulfat und zieht das Solvens im Vakuum ab. Ausbeute: 1,55 g (95% der Theorie) eines blaßgelben Öls, das beim Stehenlassen kristallisiert.

| | | | | |
|---|---|---|---|---|
| Analyse: Ber.: | C 61,25 | H 8,14 | N 23,81 | O 6,80 |
| Gef.: | C 61,17 | H 8,20 | N 23,93 | |

### g) 13,13'-Ethylendioxy-bis[3,6,9-tris(carboxymethyl)-3,6,9,15-tetraazabicyclo-[9.3.1]pentadeca-1(15),11,13-trien]

1,4 g (2,97 mmol) der Titelverbindung aus Beispiel 3f werden in 20 ml Wasser gelöst und 2,25 g (23,8 mmol) Chloressigsäure zugefügt. Man stellt mit 6 n Kalilauge auf pH 9,5. Es wird 12 Stunden bei 45°C gerührt und der pH-Wert durch Zugabe von 6 n Kalilauge zwischen 9,5-10 gehalten. Man stellt mit konz. Salzsäure auf pH 2 und reinigt an Ionenaustauschern wie in Beispiel 1d beschrieben. Kristallisation aus Ethanol/Aceton liefert 1,3 g (57% der Theorie) der Titelverbindung als stark hygroskopischen Feststoff.

| | | | | |
|---|---|---|---|---|
| Analyse: Ber.: | C 52,80 | H 6,16 | N 13,69 | O 27,35 |
| Gef.: | C 52,67 | H 6,07 | N 13,75 | |

### h) Gadoliniumkomplex von 13,13'-Ethylendioxy-bis[3,6,9-tris(carboxymethyl)-3,6,9,15-tetraazabicyclo[9.3.1]pentadeca-1(15),11,13-trien]

1,2 g (1,47 mmol) der Titelverbindung aus Beispiel 3g werden in 8 ml entionisiertem Wasser gelöst und 533 mg (1,47 mmol) Gadoliniumoxid zugefügt. Man rührt 3 Stunden bei 90°C. Die Lösung wird filtriert und das Filtrat gefriergetrocknet.
Ausbeute: 1,85 g (100% der Theorie) eines amorphen Pulvers, das laut Analyse 11,3% Wasser enthält.

| | | | | | |
|---|---|---|---|---|---|
| Analyse: Ber.: | C 38,45 | H 3,93 | N 9,94 | O 19,87 | Gd 27,90 |
| Gef.: | C 38,60 | H 3,98 | N 10,03 | | Gd 27,79 |

### BEISPIEL 4

### a) 13-Ethinyl-3,6,9-tris(acetyl)-3,6,9,15-tetraazabicyclo[9.3.1]pentadeca-1-(15),11,13-trien-15N-oxid

10 g (25,42 mmol) der Titelverbindung aus Beispiel 3c werden in 200 ml Dimethoxyethan (DME) unter Stickstoff gelöst Man gibt 1,22 g (25,42 mmol) Natriumacetylid (18%ige Suspension in Xylol/Leichtmineralöl) zu und rührt über Nacht bei Raumtemperatur Es werden 10 ml Wasser zugesetzt und zur Trockne eingedampft. Der Rückstand wird an Kieselgel chromatographiert (Laufmittel: Methanol/Aceton = 1:1), Kristallisation aus Ether/Essigester ergibt 5,02 g (53% der Theorie) der Titelverbindung als hellgelbes Pulver,

| | | | | |
|---|---|---|---|---|
| Analyse: Ber.: | C 61,27 | H 6,49 | N 15,05 | O 17,19 |
| Gef.: | C 61,31 | H 6,55 | N 14,94 | |

### b) 13,13'-(1,3-Butadiin-1,4-diyl)-bis([3,6,9-tris(acetyl)-3,6,9,15-tetraazabicyclo[9.3.1]pentadeca-1(15),11,13-trien]-15-N-oxid)

4,75 g (12,75 mmol) der Titelverbindung aus Beispiel 4a werden in 200 ml Pyridin gelöst und mit 2,52 g (25,5 mmol) Kupfer (I)-Chlorid versetzt. Die Lösung wird mit Sauerstoff gesättigt und anschließend zwei Tage bei Raumtemperatur gerührt. Dabei muß gewährleistet sein, daß ständig eine Sauerstoffatmosphäre aufrecht erhalten bleibt. Nach Einengen der Lösung im Vakuum wird der Rückstand an Kieselgel chromatographiert (Laufmittel: Methanol/Aceton = 1/2). Kristallisation aus Ether/Essigester ergibt 2,1 g (57% der Theorie) eines schwach gelblichen Pulvers.

| | | | | |
|---|---|---|---|---|
| Analyse: Ber.: | C 61,27 | H 6,49 | N 15,05 | O 17,19 |
| Gef.: | C 61,31 | H 6,55 | N 14,94 | |

### c) 13,13'-(1,3-Butadiin-1,4-diyl)-bis[3,6,9-tris(acetyl)3,6,9,15-tetraazabicyclo[9.3.1]pentadeca-1(15),11,13-trien]

2,5 g (3,37 mmol) der Titelverbindung aus Beispiel 4b werden in 50 ml Eisessig gelöst und auf 60°C erwärmt. Man setzt 1,88 g (33,65 mmol) Eisenpulver zu und rührt 2 Stunden bei 60°C. Man filtriert vom Feststoff ab und dampft das Filtrat zur Trockne ein. Der Rückstand wird mit 100 ml 3%iger Natriumcarbonat-Lösung aufgenommen und dreimal mit 100 ml Chloroform extrahiert. Nach Trocknen über Magnesiumsulfat zieht man das Solvens im Vakuum ab und kristallisiert aus Ether/Aceton.

Ausbeute: 2,08 g (87% der Theorie) farbloses Pulver

| | | | | |
|---|---|---|---|---|
| Analyse: Ber.: | C 64,21 | H 6,52 | N 15,77 | O 13,51 |
| | C 64,31 | H 6,60 | N 15,68 | |

### d) 13,13'-(1,3-Butadiin-1,4-diyl)-bis(3,6,9,15-tetraazabicyclo[9.3.1]pentadeca-1(15),11,13-trien]

1,9 g (2,67 mmol) der Titelverbindung aus Beispiel 4c werden in 20 ml Dioxan unter Stickstoff gelöst. Man setzt 2,4 g (21,38 mmol) Kalium-tert.-butylat zu und kocht über Nacht unter Rückfluß. Das Solvens wird im Vakuum abgezogen und der Rückstand mit 20 ml Wasser aufgenommen. Man stellt mit 2 n Natronlauge auf pH 10 und extrahiert sechsmal mit 60 ml Methylenchlorid. Nach Trocknen der organischen Phasen über Magnesiumsulfat wird im Vakuum eingedampft.

Ausbeute: 1,09 g (89% der Theorie) eines blaßgelben Öls.

| | | | |
|---|---|---|---|
| Analyse: Ber.: | C 68,09 | H 7,47 | N 24,44 |
| Gef.: | C 68,18 | H 7,54 | N 24,51 |

### BEISPIEL 5

### a) 13-Chlor-3,6,9-tris(acetyl)-3,6,9,15-tetraazabicyclo[9.3.1]pentadeca-1(15),11,13-trien-15-N-oxid

7,3 g (18,56 mmol) der Titelverbindung aus Beispiel 3c werden in 50 ml Acetylchlorid 4 Stunden auf 50°C erhitzt. Man engt im Vakuum ein und nimmt den Rückstand in 200 ml 3%iger Natriumcarbonat-Lösung auf. Es wird dreimal mit 100 ml Chloroform extrahiert und über Magnesiumsulfat getrocknet. Nach Entfernen des Solvens im Vakuum wird aus Ether/Essigester umkristallisiert.

Ausbeute: 6,18 g (87% der Theorie) eines farblosen kristallinen Pulvers.

| | | | | | |
|---|---|---|---|---|---|
| Analyse: Ber.: | C 53,33 | H 6,05 | N 14,64 | O 16,72 | Cl 9,26 |
| Gef.: | C 53,48 | H 5,98 | N 14,71 | | Cl 9,20 |

### b) 13-Chlor-3,6,9-tris(acetyl)-3,6,9,15-tetraazabicyclo[9.3.1]pentadeca-1(15),11,13-trien

6,0 g (15,67 mmol) der Titelverbindung aus Beispiel 5a werden in 300 ml Ethanol gelöst. Man fügt 1 ml konz. Salzsäure hinzu und hydriert über Pd/C. Nach beendeter Wasserstoffaufnahme filtriert man vom Katalysator ab und dampft im Vakuum ein. Der Rückstand wird mit 100 ml 3%iger Natriumcarbonat-Lösung aufgenommen und zweimal mit 100 ml Chloroform extrahiert. Die organischen Phasen werden über Magnesiumsulfat getrocknet und im Vakuum eingedampft. Kristallisation des Rückstandes aus Ether/Essigester ergibt 5,34 g (93% der Theorie) der Titelverbindung als farbloses Pulver.

| | | | | | |
|---|---|---|---|---|---|
| Analyse: Ber.: | C 55,66 | H 6,32 | N 15,27 | O 13,08 | Cl 9,66 |
| Gef.: | C 55,57 | H 6,38 | N 15,31 | | Cl 9,59 |

### e) 13,13'-(1,3-Butadiin-1,4-diyl)-bis[3,6,9-tris(carboxymethyl)-3,6,9,15-tetraazabicyclo[9.3.1]pentadeca-1(15),11,13-trien]

1,0 g (2,18 mmol) der Titelverbindung aus Beispiel 4d werden in 15 ml Wasser gelöst und 1,65 g (17,44 mmol) Chloressigsäure zugesetzt. Man stellt mit 6 n Kalilauge auf pH 9,5 und rührt 12 Stunden bei 45°C. Dabei wird der pH-Wert durch Zugabe von 6 n Kalilauge zwischen 9,5-10 gehalten. Nach Ansäuern mit konz. Salzsäure wird wie in 1d beschrieben an Ionenaustauschern gereinigt. Kristallisation aus Methanol/Aceton ergibt 1,01 g (61% der Theorie) eines stark hygroskopischen Feststoffes.

| | | | | |
|---|---|---|---|---|
| Analyse: Ber.: | C 56,57 | H 5,75 | N 13,89 | O 23,79 |
| Gef.: | C 56,64 | H 5,81 | N 13,79 | |

### f) Gadoliniumkomplex von 13,13'-(1,3-Butadiin-1,4-diyl)-bis[3,6,9-tris(carboxymethyl)-3,6,9,15-tetraazabicyclo[9.3.1]pentadeca-1(15),11,13-trien]

900 mg (1,116 mmol) der Titelverbindung aus Beispiel 4e werden in 8 ml Wasser gelöst und 404 mg (1,116 mmol) Gadoliniumoxid zugefügt. Nach 3 stündigem Rühren bei 90°C wird die Lösung filtriert und das Filtrat gefriergetrocknet.

Ausbeute: 1,35 g (100% der Theorie) eines weißen amorphen Pulvers, das laut Analyse 8,9% Wasser enthält.

| | | | | | |
|---|---|---|---|---|---|
| Analyse: Ber.: | C 40,92 | H 3,62 | N 10,05 | O 17,22 | Gd 28,20 |
| Gef.: | C 40,81 | H 3,65 | N 10,18 | | Gd 28,11 |

### c) 13-Chlor-3,6,9,15-tetraazabicyclo[9.3.1]pentadeca-1(15),11,13-trien

5,1 g (13,9 mmol) der Titelverbindung aus Beispiel 5b werden in 50 ml Dioxan unter Stickstoff gelöst. Man gibt 6,24 g (55,6 mmol) Kalium-tert.-butylat zu und kocht über Nacht unter Rückfluß. Man arbeitet wie in Beispiel 4d beschrieben auf.
Ausbeute: 3,01 g (90% der Theorie) eines leicht gelblichen Öls, das nach kurzer Zeit kristallisiert.

| | | | | |
|---|---|---|---|---|
| Analyse: Ber.: | C 54,88 | H 7,12 | N 23,28 | Cl 14,73 |
| Gef.: | C 54,93 | H 7,06 | N 23,41 | Cl 14,81 |

### d) 13-Chlor-3,6,9-tris(carboxymethyl)-3,6,9,15-tetraazabicyclo[9.3.1]pentadeca-1(15),11,13-trien

2,38 g (11,65 mmol) der Titelverbindung aus Beispiel 5c werden in 30 ml Wasser gelöst und 4,4 g (46,6 mmol) Chloressigsäure zugesetzt. Man stellt mit 6 n Kalilauge auf pH 9,5. Es wird 12 Stunden bei 45°C gerührt und der pH-Wert durch Zugabe von 6 n Kalilauge zwischen 9,5 - 10 gehalten. Nach Aufarbeitung wie in Beispiel 1d beschrieben und Kristallisation aus Methanol/Aceton erhält man 3,23 g (67% der Theorie) der Titelverbindung als einen an der Luft zerfließenden Feststoff.

| | | | | | |
|---|---|---|---|---|---|
| Analyse: Ber.: | C 49,22 | H 5,59 | N 13,51 | O 23,14 | Cl 8,55 |
| Gef.: | C 49,31 | H 5,65 | N 13,60 | | Cl 8,49 |

### e) Gadolinium-Komplex von 13-Chlor-3,6,9-tris(carboxymethyl)-3,6,9,15-tetraazabicyclo[9.3.1]pentadeca-1(15),11,13-trien

3,23 g (7,78 mmol) der Titelverbindung aus Beispiel 5d werden in 20 ml entionisiertem Wasser gelöst und 1,41 g (3,89 mmol) Gadoliniumoxid zugefügt. Man rührt 3 Stunden bei 90°C. Die Lösung wird filtriert und das Filtrat gefriergetrocknet.
Ausbeute: 4,9 g (100% der Theorie) eines weißen amorphen Pulvers, das laut Analyse 11,9% Wasser enthält.

| | | | | | | |
|---|---|---|---|---|---|---|
| Analyse: Ber.: | C 35,88 | H 3,54 | N 9,85 | O 16,87 | Cl 6,23 | Gd 27,63 |
| Gef.: | C 35,94 | H 3,57 | N 10,01 | | Cl 6,17 | Gd 27,56 |

T₁-Relaxivität (L/mmol·sec), 40°C, Wasser, 20 MHZ: 5,44

### BEISPIEL 6

### a) 3,6,9,15-Tetraazabicyclo[9.3.1]pentadeca-1(15),11,13-trien-15-N-oxid

4,5 g (12,9 mmol) der Titelverbindung aus Beispiel 3b werden in 40 ml Dioxan unter Stickstoff gelöst. Man fügt 5,8 g (51,7 mmol) Kalium-tert.-butylat zu und kocht über Nacht unter Rückfluß. Nach Aufarbeitung wie in Beispiel 3f beschrieben erhält man 2,61 g (91% der Theorie) eines zartgelben Öls, das beim Stehenlassen kristallisiert.

| | | | | |
|---|---|---|---|---|
| Analyse: Ber.: | C 59,43 | H 8,16 | N 25,20 | O 7,20 |
| Gef.: | C 59,37 | H 8,21 | N 25,13 | |

### b) 3,6,9-tris(carboxymethyl)-3,6,9,15-tetraazabicyclo[9.3.1]pentadeca-1(15),-11,13-trien-15-N-oxid

2,4 g (10,77 mmol) der Titelverbindung aus Beispiel 6a werden in 30 ml Wasser gelöst und 4,1 g (43,4 mmol) Chloressigsäure zugefügt. Man stellt mit 6 n Kalilauge auf pH 9,5. Es wird 12 Stunden bei 45°C gerührt und der pH-Wert unter Zugabe von 6 n Kalilauge zwischen 9,5-10 gehalten. Nach Aufarbeitung wie in Beispiel 1d beschrieben ergibt die Kristallisation aus Ethanol/Aceton 2,7 g (63% der Theorie) eines stark hygroskopischen Pulvers.

| | | | | |
|---|---|---|---|---|
| Analyse: Ber.: | C 51,51 | H 6,10 | N 14,14 | O 28,26 |
| Gef.: | C 51,63 | H 6,01 | N 14,08 | |

### c) Gadolinium-Komplex von 3,6,9-tris(carboxymethyl)3,6,9,15-tetraazabicyclo-[9.3.1]pentadeca-1(15),11,13-trien-15-N-oxid

2,1 g (5,3 mmol) der Titelverbindung aus Beispiel 6b werden in 15 ml entionisiertem Wasser gelöst und 935 mg (2,65 mmol) Gadoliniumoxid zugefügt. Man rührt 3 Stunden bei 90°C. Die Lösung wird filtriert und das Filtrat gefriergetrocknet. Ausbeute: 3,2 g (100% der Theorie) eines amorphen Pulvers, das laut Analyse
10,7% Wasser enthält.

| | | | | | |
|---|---|---|---|---|---|
| Analyse: Ber.: | C 37,08 | H 3,84 | N 10,18 | O 20,34 | Gd 28,56 |
| Gef.: | C 37,18 | H 3,79 | N 10,21 | | Gd 28,48 |

### BEISPIEL 7

### a) 3,6,9-Tris(p-tolylsulfonyl)-3,6,9-triaza-14-oxa-bicyclo[9.2.1]tetradeca-1-(13),11-dien

Zu 121,9 g (200 mmol) N,N',N''-Tris(p-tolylsulfonyl)-diethylentriamin-N,N''-dinatriumsalz in 1600 ml Dimethylformamid wird bei 100°C eine Lösung von 33 g (200 mmol) 2,5-Bis(chlormethyl)-furan in 700 ml Dimethylformamid innerhalb 3 Stunden zugetropft. Man läßt über Nacht bei 100°C rühren. In die heiße Lösung werden 2 Liter Wasser getropft. Man kühlt auf 0°C. Der Niederschlag wird mit viel Wasser gewaschen und im Vakuum getrocknet (60°C). Nach Kristallisation aus Acetonitril erhält man 88,14 g (67% der Theorie) eines weißen Pulvers.

| | | | | | |
|---|---|---|---|---|---|
| Analyse: Ber.: | C 56,60 | H 5,36 | N 6,39 | O 17,03 | S 14,62 |
| Gef.: | C 56,52 | H 5,42 | N 6,30 | | S 14,60 |

### b) 3,6,9-Triaza-14-oxa-bicyclo[9.2.1]tetradeca-1(13),11-dien

30 g (45,61 mmol) der Titelverbindung aus Beispiel 7a werden in 500 ml flüssigem Ammoniak bei -40°C suspendiert. Dann werden innerhalb 30 Minuten 10,49 g (456,1 mmol) Natrium zugefügt und 3 Stunden bei -40°C gerührt. Man zerstört den Überschuß Natrium durch vorsichtige Zugabe von Ethanol (Entfärbung) und läßt Ammoniak abdampfen. Der Rückstand wird mit 100 ml Wasser aufgenommen und mit 6 n Natronlauge auf pH 11 eingestellt. Dann extrahiert man sechsmal mit 150 ml Methylenchlorid, trocknet die organische Phase über Magnesiumsulfat und dampft im Vakuum ein. Der Rückstand wird an Kieselgel chromatographiert (Laufmittel: Acetonitril/Wasser/32%ige Ammoniaklösung = 10/3/1).
Ausbeute: 3,95 g (45% der Theorie) eines blaßgelben Öls.

| | | | | |
|---|---|---|---|---|
| Analyse: Ber.: | C 61,51 | H 8,78 | N 21,52 | O 8,19 |
| Gef.: | C 61,43 | H 8,85 | N 21,47 | |

### c) 3,6,9-Tris(ethoxycarbonylmethyl)-3,6,9-triaza-14-oxa-bicyclo[9.2.1]tetradeca-1(13),11-dien

Zu einer Mischung aus 3,7 g (18,95 mmol) der Titelverbindung aus Beispiel 7b und 6,03 g (56,85 mmol) wasserfreiem Natriumcarbonat in 150 ml Dimethylformamid werden bei 50°C innerhalb 10 Minuten 7 ml (62,5 mmol) Bromessigsäureethylester langsam zugetropft. Man rührt 4 Stunden bei 50°C. Das Solvens wird im Vakuum abgezogen und der Rückstand in 200 ml Methylenchlorid ausgerührt. Man filtriert vom Feststoff ab und dampft das Filtrat ein. Das zurückbleibende Öl wird an Kieselgel chromatographiert (Laufmittel: Methylenchlorid/Ethanol = 12/1).
Ausbeute: 6,1 g (71% der Theorie) eines gelblichen Öls, das langsam erstarrt.

| | | | | |
|---|---|---|---|---|
| Analyse: Ber.: | C 58,26 | H 7,78 | N 9,27 | O 24,69 |
| Gef.: | C 58,17 | H 7,88 | N 9,19 | |

### d) Gadolinium-Komplex von 3,6,9-Tris(carboxymethyl)-3,6,9-triaza-14-oxa-bicyclo[9.2.1]tetradeca-1(13),11-dien

5,0 g (11,02 mmol) der Titelverbindung aus Beispiel 7c werden in 80 ml Ethanol gelöst und bei 60°C 36 ml 1 n Natronlauge langsam zugetropft. Es wird 30 Minuten unter Rückfluß erhitzt. Man dampft zur Trockne ein, nimmt mit 20 ml Wasser auf und bringt mit 2 n Salzsäure vorsichtig auf pH 6,5. Nach Zugabe von 2,0 g (5,57 mmol) Gadoliniumoxid wird 3 Stunden bei 90°C gerührt. Die Lösung wird filtriert und das Filtrat zuerst über eine kurze Kationenaustauschersäule (IR-120), dann über eine kurze Anionenaustauschersäule (IRA-67) gegeben. Das Eluat wird gefriergetrocknet.
Ausbeute: 4,9 g (78% der Theorie) eines amorphen Pulvers, das laut Analyse 9,7% Wasser enthält.

| | | | | | |
|---|---|---|---|---|---|
| Analyse: | C 36,70 | H 3,85 | N 8,03 | O 21,39 | Gd 30,03 |
| | C 36,51 | H 3,81 | N 8,11 | | Gd 29,91 |

### BEISPIEL 8

### Gadolinium-Komplex von 3,6,9-Tris(carboxymethyl)-3,6,9,14-tetraaza-bicyclo-[9.2.1]tetradeca-1(13),11-dien

10,0 g (22,04 mmol) der Titelverbindung aus Beispiel 7c werden in 150 ml Ethanol gelöst und bei 60°C 80 ml 1 n Natronlauge zugetropft. Man kocht eine Stunde unter Rückfluß und dampft dann zur Trockne ein. Der Rückstand wird in einen Schüttelautoklaven überführt und 3,54 g (66,12 mmol) Ammoniumchlorid zugefügt. Nach Zugabe von Vanadiumoxid-Katalysator (US-Patent 2 478 456, Chem. Abstr. 44, 665(1950)) werden 100 ml Ammoniak einkondensiert. Man erhitzt 12 Stunden auf 200°C. Nach Abdampfen des Ammoniaks wird der Rückstand an Kieselgel chromatographiert (Laufmittel: Dioxan/Wasser/32%ige wäßrige Ammoniaklösung = 6/2/1). Eindampfen ergibt ca.5,19 g (56% der Theorie) des extrem hygroskopischen Ammoniumsalzes, das sofort weiter umgesetzt wird. Man löst in 25 ml entionisiertem Wasser und stellt mit 2 n Salzsäure auf pH 6,5. Es werden 2,24 g (6,17 mmol) Gadoliniumoxid zugefügt und 3 Stunden bei 90°C unter Stickstoff gerührt. Man setzt 1 g Aktivkohle zu und rührt eine weitere Stunde bei 90°C. Die Lösung wird filtriert und das Filtrat zuerst über eine kurze Kationenaustauschersäule (IR-120), dann über eine kurze Anionenaustauschersäule (IRA-67) gegeben. Das Eluat wird gefriergetrocknet.
Ausbeute: 6,0 g (47% der Theorie bezogen auf die Titelverbindung aus Beispiel 7c) eines amorphen Pulvers, das laut Analyse 11,1% Wasser enthält.

| | | | | | |
|---|---|---|---|---|---|
| Analyse: Ber.: | C 36,84 | H 3,87 | N 10,74 | O 18,40 | Gd 30,15 |
| Gef.: | C 36,75 | H 3,91 | N 10,68 | | Gd 30,04 |

### BEISPIEL 9

### a) 15-Methoxy-3,6,9-Tris(p-tolylsulfonyl)-3,6,9-triaza-bicyclo[9.3.1]-pentadeca-1(15),11,13-trien

182,85 g (300 mmol) N,N',N''-Tris(p-tolylsulfonyl)-diethylentriamin-N'N''-dinatriumsalz werden in 2,4 Liter Dimethylformamid gelöst und auf 100°C erwärmt. Hierzu tropft man innerhalb von 3 Stunden eine Lösung aus 88,2 g (300 mmol) 2,6-Bis(brommethyl)-phenolmethylether in 1 Liter Dimethylformamid. Es wird über Nacht bei 100°C gerührt. In die heiße Lösung tropft man 3 Liter Wasser und kühlt auf 0°C ab. Der Niederschlag wird mit viel Wasser gewaschen und im Vakuum (60°C) getrocknet. Kristallisation aus Acetonitril ergibt 119,3 g (57% der Theorie) der Titelverbindung als leicht cremefarbenes Pulver.

| | | | | | |
|---|---|---|---|---|---|
| Analyse: Ber.: | C 58,51 | H 5,63 | N 6,02 | O 16,05 | S 13,78 |
| Gef.: | C 58,41 | H 5,68 | N 6,13 | | S 13,70 |

### b) 15-Hydroxy-3,6,9-triazabicyclo[9.3.1]-pentadeca-1(15),11,13-trien

Zu 100 g (143,3 mmol) der Titelverbindung aus Beispiel 9a in 2 Liter Dibutylether werden vorsichtig 21,75 g (573,2 mmol) Lithiumaluminiumhydrid zugegeben und über Nacht unter Rückfluß erhitzt. Man kühlt im Eisbad und zerstört überschüssiges Lithiumaluminiumhydrid mit Ethanol und anschließend mit Wasser. Es wird zur Trockne eingedampft, der Rückstand mit 1 Liter 2 n Natronlauge aufgenommen und zehnmal mit 200 ml Chloroform extrahiert. Nach Trocknen über Magnesiumsulfat wird im Vakuum eingeengt und der Rückstand an Kieselgel chromatographiert (Laufmittel: Methanol/32%ige wäßrige Ammoniaklösung = 10/1).
Ausbeute: 8,56 g (27% der Theorie) eines gelblichen Öls.

| | | | | |
|---|---|---|---|---|
| Analyse: Ber.: | C 65,13 | H 8,65 | N 18,99 | O 7,23 |
| Gef.: | C 65,18 | H 8,60 | N 19,10 | |

### c) 15-Hydroxy-3,6,9-tris(tert.-butoxycarbonylmethyl)-3,6,9-triazabicyclo[9.3.1] pentadeca-1(15),11,13-trien

8,3 g (37,50 mmol) der Titelverbindung aus Beispiel 9b werden in 250 ml Dimethylformamid gelöst und 15,55 g (112,5 mmol) wasserfreies Kaliumcarbonat zugesetzt. Hierzu tropft man innerhalb von 30 Minuten 16,3 ml (112,5 mmol) Bromessigsäure-tert.-butylester und rührt über Nacht bei Raumtemperatur. Man dampft das Lösungsmittel bis zur Trockne ab, nimmt mit 300 ml Wasser auf und extrahiert dreimal mit 150 ml Methylenchlorid. Die organischen Phasen werden über Magnesiumsulfat getrocknet und im Vakuum eingedampft.Das zurückbleibende Öl wird an Kieselgel chromatographiert (Laufmittel: Methylenchlorid/Methanol = 15/1).
Ausbeute: 13,32 g (63% der Theorie) der Titelverbindung als farbloses Öl.

| | | | | |
|---|---|---|---|---|
| Analyse: Ber.: | C 63,91 | H 8,76 | N 7,45 | O 19,87 |
| Gef.: | C 63,83 | H 8,85 | N 7,49 | |

### d) 15-Hydroxy-3,6,9-tris(carboxymethyl)-3,6,9-triazabicyclo[9.3.1]pentadeca-1-(15),11,13-trien

13,0 g (23,06 mmol) der Titelverbindung aus Beispiel 9c werden in 150 ml Trifluoressigsäure gelöst und über Nacht bei Raumtemperatur gerührt. Man engt im Vakuum ein. Der Rückstand wird mit wenig Wasser aufgenommen und wie in Beispiel 1d beschrieben an Ionenaustauschern gereinigt. Kristallisation aus Methanol/Aceton ergibt 6,5 g (71% der Theorie) der Titelverbindung als ein an der Luft zerfließendes Pulver.

| | | | | |
|---|---|---|---|---|
| Analyse: Ber.: | C 54,67 | H 6,37 | N 10,63 | O 28,33 |
| Gef.: | C 54,51 | H 6,30 | N 10,57 | |

### e) Gadolinium-Komplex von 15-Hydroxy-3,6,9-tris(carboxymethyl)3,6,9-triazabicyclo[9.3.1]pentadeca-1(15),11,13-trien

4,0 g (10,1 mmol) der Titelverbindung aus Beispiel 9d werden in 25 ml entionisiertem Wasser gelöst und 1,84 g (5,05 mmol) Gadoliniumoxid zugefügt. Man rührt 3 Stunden bei 90°C, setzt 1 g Aktivkohle zu und rührt eine weitere Stunde bei dieser Temperatur. Die Lösung wird filtriert und das Filtrat gefriergetrocknet. Man erhält 6,04 g (96% der Theorie) eines amorphen Pulvers, das laut Analyse 13,5 % Wasser enthält.

| | | | | | |
|---|---|---|---|---|---|
| Analyse: Ber.: | C 39,33 | H 4,04 | N 7,65 | O 20,38 | Gd 28,61 |
| Gef.: | C 39,41 | H 4,10 | N 7,58 | | Gd 28,51 |

### BEISPIEL 10

### a) 6-Benzyl-3,9-bis(p-tolylsulfonyl)-3,6,9,15-tetraazabicyclo[9.3.1]-pentadeca-1(15),11,13-trien

Zu 109,12 g (200 mmol) N,N''-Bis(p-tolylsulfonyl)-N'-benzyl-diethylentriamin-N,N''-dinatriumsalz in 1500 ml Dimethylformamid werden bei 100°C innerhalb 3 Stunden eine Lösung aus 35,2 g (200 mmol) 2,6-Bis(chlormethyl)pyridin (gelöst in 700 ml Dimethylformamid) getropft. Es wird über Nacht bei 100°C weitergerührt. In die heiße Lösung tropft man 2 Liter Wasser und kühlt auf 0°C ab. Der Niederschlag wird mehrmals mit Wasser gewaschen und im Vakuum getrocknet (60°C). Kristallisation aus Acetonitril/Ether ergibt 78,6 g (65% der Theorie) eines cremefarbenen Pulvers.

| | | | | | |
|---|---|---|---|---|---|
| Analyse: Ber.: | C 63,55 | H 6,00 | N 9,26 | O 10,58 | S 10,60 |
| Gef.: | C 63,48 | H 5,94 | N 9,18 | | S 10,63 |

### b) 6-Benzyl-3,6,9,15-tetraazabicyclo[9.3.1]pentadeca-1(15),11,13-trien

Zu 75 g (124 mmol) der Titelverbindung aus Beispiel 10a in 1,5 Liter Dibutylether werden vorsichtig 9,41 g (248 mmol) Lithiumaluminiumhydrid gegeben und über Nacht unter Rückfluß erhitzt. Nach Abkühlen im Eisbad wird der Überschuß Lithiumaluminiumhydrid mit Ethanol und Wasser zerstört. Es wird zur Trockne eingedampft, mit 500 ml Wasser aufgenommen und mit 6 n Kalilauge auf pH 11 gestellt. Man extrahiert sechsmal mit 100 ml Chloroform, trocknet über Magnesiumsulfat und engt im Vakuum ein. Chromatographie an Kieselgel (Laufmittel: Ethanol/32%ige wäßrige Ammoniaklösung = 12/1) ergibt 22,4 g (61% der Theorie) eines gelblichen Öls, das glasig erstarrt.

| | | | |
|---|---|---|---|
| Analyse: Ber.: | C 72,94 | H 8,16 | N 18,90 |
| Gef.: | C 72,75 | H 8,23 | N 18,81 |

### c) 6-Benzyl-3,9-bis(ethoxycarbonylmethyl)-3,6,9,15-tetraazabicyclo[9.3.1] pentadeca-1(15),11,13-trien

10 g (33,74 mnmol) der Titelverbindung aus Beispiel 10b werden in 300 ml Dimethylformamid gelöst. Man gibt 7,13 g (67,48 mmol) wasserfreies Natriumcarbonat hinzu und erwärmt auf 50°C. Dann werden innerhalb von 15 Minuten 8,3 ml (74,2 mmol) Bromessigsäureethylester zugetropft und über Nacht bei 50°C weitergerührt. Man dampft das Solvens im Vakuum ab, rührt den Rückstand zweimal mit 350 ml Methylenchlorid aus, filtriert und engt ein. Das zurückbleibende Öl wird an Kieselgel chromatographiert (Laufmittel: Methylenchlorid/Ethanol = 10/1).
Ausbeute: 13,12 g (83% der Theorie) der Titelverbindung als farbloses Öl.

| | | | | |
|---|---|---|---|---|
| Analyse: Ber.: | C 66,64 | H 7,74 | N 11,96 | O 13,66 |
| Gef.: | C 66,51 | H 7,81 | N 11,88 | |

### d) 3,9-Bis(carboxymethyl)-3,6,9,15-tetraazabicyclo[9.3.1]pentadeca-1(15),11,13-trien

12 g (25,6 mmol) der Titelverbindung aus Beispiel 10c werden in 100 ml Ethanol gelöst und auf 60°C erwärmt. Zu dieser Lösung tropft man 32 ml 2 n Natronlauge und kocht eine Stunde unter Rückfluß. Man dampft zur Trockne ein und löst den Rückstand in 200 ml 5%iger Essigsäure. Es wird über Pd/C hydriert. Nach beendeter Wasserstoffaufnahme filtriert man vom Katalysator ab, engt im Vakuum ein und reinigt wie in Beispiel 1d beschrieben an Ionenaustauschern. Kristallisation aus Ethanol/Aceton ergibt 6,52 g (79% der Theorie) eines stark hygroskopischen Feststoffes.

| | | | | |
|---|---|---|---|---|
| Analyse: Ber.: | C 55,88 | H 6,88 | N 17,38 | O 19,85 |
| Gef.: | C 55,79 | H 6,94 | N 17,27 | |

### e) Mangan (II)-Komplex von 3,9-Bis(carboxymethyl)-3,6,9,15-tetraazabicyclo-[9.3.1]pentadeca-1(15),11,13-trien

4 g (12,4 mmol) der Titelverbindung aus Beispiel 10d werden in 20 ml entionisiertem Wasser gelöst und 1,43 g (12,4 mmol) Mangan-(II)-carbonat zugefügt. Man erhitzt 2 Stunden auf 80°C. Die Lösung wird zuerst über eine kurze Kationenaustauschersäule (IR-120), anschließend über eine kurze Anionenaustauschersäule (IRA-67) gegeben. Das Eluat kocht man mit 1 g Aktivkohle eine Stunde unter Rückfluß und filtriert. Das Filtrat wird gefriergetrocknet. Man erhält 4,4 g (87% der Theorie) eines leicht rosa schimmernden amorphen Pulvers.

| | | | | | |
|---|---|---|---|---|---|
| Analyse: Ber.: | C 48,00 | H 5,37 | N 14,93 | O 17,05 | Mn 14,64 |
| Gef.: | C 47,93 | H 5,41 | N 14,87 | | Mn 14,58 |

### BEISPIEL 11

### a) 3,6-Bis(p-tolylsulfonyl)-3,6,12-triazabicyclo[6.3.1]dodeca-1(12),8,10-trien

Zu 82,48 g (0,2 mol) N,N'-Bis(p-tolylsulfonyl)-ethylendiamin-di-natriumsalz in 1600 ml Dimethylformamid wird bei 100°C eine Lösung von 35,2 g (0,2 mol) 2,6-Bis(chlormethyl)-pyridin (gelöst in 700 ml Dimethylformamid) innerhalb 3 Stunden zugetropft. Man läßt über Nacht bei 110°C weiterrühren. In die heiße Lösung werden 2 Liter Wasser getropft, der Niederschlag wird abgesaugt und mit viel Wasser gewaschen. Nach Trocknung im Vakuum (60°C) wird aus Acetonitril umkristallisiert.
Ausbeute: 67,9 g (72% der Theorie) eines cremefarbenen Pulvers.

| | | | | | |
|---|---|---|---|---|---|
| Analyse: Ber.: | C 58,58 | H 5,34 | N 8,91 | O 13,57 | S 13,60 |
| Gef.: | C 58,41 | H 5,37 | N 8,85 | | S 13,53 |

### b) 3,6,12-Triazabicyclo[6.3.1]dodeca-1(12),8,10-trien Trihydrosulfat

67,0 g (142 mmol) der Titelverbindung aus Beispiel 11a werden in 200 ml konz. Schwefelsäure eingetragen und 48 Stunden bei 100°C gerührt. Man kühlt auf 0°C und tropft 1 l absoluten Ether zu. Der Niederschlag wird abgesaugt und in 600 ml Methanol ausgerührt. Man filtriert und dampft anschließend zur Trockne ein. Trocknung im Vakuum (60°C) liefert 44,17 g (68% der Theorie) der Titelverbindung als kristallinen Feststoff.

| | | | | | |
|---|---|---|---|---|---|
| Analyse: Ber.: | C 23,63 | H 4,19 | N 9,19 | O 41,97 | S 21,03 |
| Gef.: | C 23,57 | H 4,24 | N 9,11 | | S 20,96 |

### c) 3,6,12-Triazabicyclo[6.3.1]dodeca-1(12),8,10-trien

42,0 g (91,8 mmol) der Titelverbindung aus Beispiel 11b werden in 100 ml Wasser gelöst und mit 32%iger Natronlauge auf pH 11 gestellt. Man extrahiert sechsmal mit 200 ml Methylenchlorid und trocknet die vereinigten Phasen über Magnesiumsulfat. Nach Eindampfen im Vakuum erhält man 11,24 g (75% der Theorie) eines leicht gelben Öls.

| | | | |
|---|---|---|---|
| Analyse: Ber.: | C 66,23 | H 8,03 | N 25,74 |
| Gef.: | C 66,17 | H 8,09 | N 25,67 |

### d) 3,6-Bis(carboxymethyl)-3,6,12-triazabicyclo[6.3.1]dodeca-1(12),8,10-trien

10 g (61,27 mmol) der Titelverbindung aus Beispiel 11c werden in 100 ml Wasser gelöst und 17,37 g (183,8 mmol) Chloressigsäure zugegeben. Man stellt durch Zugabe von 6 n Kalilauge auf pH 9,5 und erwärmt auf 45°C. Es wird 12 Stunden bei dieser Temperatur gerührt, wobei der pH-Wert durch Zugabe von 6 n Kalilauge zwischen 9,5 -10 gehalten wird. Man kühlt ab und reinigt wie in Beispiel 1d beschrieben an Ionenaustauschern. Kristallisation aus Ethanol/Aceton liefert 11,47 g (67% der Theorie) der Titelverbindung als kristallinen Feststoff.

| | | | | |
|---|---|---|---|---|
| Analyse: Ber.: | C 55,90 | H 6,14 | N 15,05 | O 22,92 |
| Gef.: | C 55,81 | H 6,19 | N 14,94 | |

### e) Mangan-Komplex von 3,6-Bis(carboxymethyl)-3,6,12-triazabicyclo[6.3.1]dodeca-1(12),8,10-trien

10,0 g (35,8 mmol) der Titelverbindung aus Beispiel 11d werden in 40 ml entionisiertem Wasser gelöst und 4,12 g (35,8 mmol) Mangan-II-carbonat zugesetzt. Man rührt 2 Stunden bei 80°C. Die Lösung wird über eine kurze Anionen- und Kationenaustauschersäule gegeben und das Eluat mit 1 g Aktivkohle 1 Stunde bei 80°C gerührt. Es wird filtriert und das Filtrat gefriergetrocknet.
Ausbeute: 12,7 g (96% der Theorie) der Titelverbindung als amorphes Pulver, das laut Analyse 11,3% Wasser enthält.

| | | | | | |
|---|---|---|---|---|---|
| Analyse: Ber.: | C 47,00 | H 4,55 | N 12,65 | O 19,26 | Mn 16,54 |
| Gef.: | C 46,95 | H 4,61 | N 12,58 | | Mn 16,48 |

### BEISPIEL 12

### Herstellung einer Losung vom Gadolinium-Komplex des 3,6,9-Tris(carboxymethyl)-3,6,9,15-tetraazabicyclo[9.3.1]pentadeca-1(15),11,13-triens

534,63 g (1 Mol) der in Beispiel 1e beschriebenen Verbindung wurde in 1200 ml Wasser pro injectione (p.i.) gelöst. Nach Zugabe von 24,62 g (50 mmol) Monohydrat des Calcium-Trinatriumsalzes der DTPA, CaNa₃DTPA, füllt man mit Wasser p.i. auf 2000 ml auf. Die Lösung wird abschließend ultrafiltriert, ampulliert und hitzesterilisiert und ist für die parenterale Anwendung gebrauchsfertig.

### BEISPIEL 13

### Herstellung einer Lösung des Megluminsalzes vom Mangan-II-Komplex des 3,6,9-Tris(carboxymethyl)-3,6,9,15-tetraazabicyclo[9.3.1]pentadeca-1(15),11,-13-triens

34,92 g (50 mmol) der in Beispiel 1h beschriebenen Verbindung (Wassergehalt 12,29%) werden in 65 ml Wasser (p.i.) gelöst. Nach Zugabe von 492 mg (1 mmol) Monohydrat des Calcium-trinatriumsalzes der DTPA, CaNa₃DTPA, füllt man mit Wasser p.i. auf 100 ml auf. Die Lösung wird abschließend ultrafiltriert, ampulliert und hitzesterilisiert und ist für die parenterale Anwendung gebrauchsfertig.

### BEISPIEL 14

Zusammensetzung eines Pulvers zur Herstellung einer Suspension für die orale Applikation:
4,000 g des in Beispiel 1e beschriebenen Gadolinium-Komplexes
3,895 g Mannitol
0,100 g Polyoxyethylenpolyoxypropylen-Polymeres
0.005 g Aromastoffe
8̅,̅0̅0̅0̅ g̅

### BEISPIEL 15

### Herstellung einer Lösung vom Indium-III-Komplex des 3,6,9-Tris(carboxymethyl)-3,6,9,15-tetraazabicyclo[9.3.1]pentadecans

Eine Lösung von 100 »g der in Beispiel 2a beschriebenen Verbindung in 5 ml eines Gemischs aus einer 150 mmolaren Kochsalz- und einer 150 mmolaren Natriumacetatlösung (pH 5,8) wird mit einer Lösung von 5 ml Indium-111-chlorid in 1 ml n-Salzsäure versetzt. Man bringt durch Zugabe von 0,1 n-Natronlauge auf pH 7,2, füllt die steril filtrierte Lösung in Multivials ab und lyophilisiert sie. Der Rückstand wird in physiologischer Kochsalzlösung aufgenommen und stellt dann ein für die Radiodiagnostik geeignetes Präparat dar.

In analoger Weise erhält man mit Yttrium-90-chlorid ein für die Radiotherapie geeignetes Präparat.

### BEISPIEL 16

### a) 3,6,9-Tris(p-tolylsulfonyl)-14-oxa-3,6,9-triazabicyclo[9.2.1]tetradecan

Zu einer Lösung von 28,19 g (64 mMol) 2,5-Bis(p-tosyloxymethyl)-tetrahydrofuran in 500 ml Dimethylformamid tropft man innerhalb von 2 Stunden bei 100 °C 39,01 g (64 mMol) N,N',N''-Tris(p-tolylsulfonyl)-diethylentriamin-N,N''-dinatriumsalz gelöst in 210 ml Dimethylformamid und rührt 5 Stunden bei 120 °C. In die heiße Lösung tropft man 700 ml Wasser und kühlt auf 0 °C ab. Der Niederschlag wird abgesaugt, mit Wasser gewaschen und im Vakuum bei 50° getrocknet. Nach dem Umkristallisieren aus Aceton erhält man 33,5 g der Titelverbindung als weißes Pulver, Schmelzpunkt 175 - 178 °C.

| | | | | |
|---|---|---|---|---|
| Analyse: Ber.: | C 56,26 | H 5,94 | N 6,35 | S 14,53 |
| Gef.: | C 56,01 | H 5,99 | N 6,28 | S 14,29 |

### b) 14-Oxa-3,6,9-triazabicyclo[9.2.1]tetradecan

30 g (45,3 mMol) der Titelverbindung aus Beispiel 16a) werden in 90 ml konz. Schwefelsäure eingetragen und 24 Stunden bei 90 °C gerührt. Man kühlt dann auf 0 °C ab und tropft 350 ml trockenen Ether zu. Der gebildete Niederschlag wird abgesaugt und in 50 ml 40 %iger Natronlauge gelöst und die Lösung 10 mal mit je 50 ml Dichlormethan extrahiert. Die organische Phase wird über Magnesiumsulfat getrocknet und im Vakuum eingedampft. Man erhält 6,23 g (69 % d. Th.) der Titelverbindung als weißes Pulver.

| | | | |
|---|---|---|---|
| Analyse: Ber.: | C 60,27 | H 10,62 | N 21,08 |
| Gef.: | C 60,03 | H 10,75 | N 20,95 |

### c) 3,6,9-Tris-(carboxymethyl)-14-oxa-3,6,9-triazabicyclo[9.2.1]tetradecan

6 g (30,1 mMol) der Titelverbindung aus Beispiel 16b) werden in 35 ml Wasser gelöst, mit 11,38 g (120,4 mMol) Chloressigsäure versetzt und diese Lösung mit 6N Kalilauge auf pH 9,5 eingestellt. Man erwärmt 12 Stunden auf 45 °C und hält während dieser Zeit den pH-Wert durch Zugabe weiterer Kalilauge bei 9,5 - 10. Anschließend kühlt man auf Raumtemperatur, versetzt langsam mit konz. Salzsäure bis pH 2 und dampft im Vakuum ein. Der Rückstand wird in 100 ml Wasser gelöst und diese Lösung auf eine Kationenaustauschersäule (IR 120) gegeben. Man wäscht die Säule zunächst mit viel Wasser. Dann eluiert man die gewünschte Substanz mit 0,5 N Ammoniaklösung, dampft die Lösung ein, löst den Rückstand in 100 ml Wasser und gibt die Lösung auf eine Anionenaustauschersäule (IRA 67). Zunächst wird mit Wasser gewaschen, anschließend eluiert man mit 0,5 N Ameisensäure. Die sauren Fraktionen werden eingedampft, der Rückstand in Methanol gelöst. Nach Zugabe von Aceton kristallisiert die Titelverbindung aus (5,74 g, 51 % der Theorie).

| | | | |
|---|---|---|---|
| Analyse: Ber.: | C 51,47 | H 7,29 | N 11,25 |
| Gef.: | C 51,60 | H 7,21 | N 11,38 |

### b) 14-Thia-3,6,9-triazabicyclo[9.2.1]tetradeca-1(13),11-dien

45 g (66,8 mMol) der Titelverbindung aus Beispiel 17a) werden in 130 ml konz. Schwefelsäure eingetragen und 24 Stunden bei 90 - 95 °C gerührt. Nach Abkühlen auf 0 °C tropft man 500 ml Ether zu, saugt vom gebildeten Niederschlag ab und löst den Niederschlag in 70 ml 40 %iger Natronlauge. Die Lösung wird fünfmal mit je 100 ml Dichlormethan extrahiert, über Magnesiumsulfat getrocknet und im Vakuum eingedampft. Den Rückstand kristallisiert man aus Ether/Hexan (3:1) um und erhält 7,8 g (55 % der Theorie) der Titelverbindung als weißes Pulver.

| | | | | |
|---|---|---|---|---|
| Analyse: Ber.: | C 56,83 | H 8,11 | N 19,88 | S 15,17 |
| Gef.: | C 56,59 | H 8,02 | N 20,12 | S 15,00 |

### c) 3,6,9-Tris-(carboxymethyl)-14-thia-3,6,9-triazabicyclo[9.2.1]tetradeca-1(13),11-dien

Man löst 7,5 g (35,5 mMol) der Titelverbindrnng aus Beispiel 17b) in 45 ml Wasser, versetzt mit 13,42 g (142 mMol) Chloressigsäure und stellt den pH-Wert mit 6 N Kalilauge auf 9,5 ein. Dann erwärmt man 12 Stunden auf 45 - 50 °C und hält während dieser Zeit den pH-Wert durch Zugabe weiterer Kalilauge bei 9,5 - 10. Nach Abkühlen auf 10 °C versetzt man mit konz. Salzsäure bis pH 2. Den dabei gebildeten Niederschlag isoliert man durch Absaugen, löst ihn in 100 ml Wasser und adsorbiert die Lösung an einer Kationenaustauschersäule (IR 120), wäscht die Säule mit 2 l Wasser, anschließend mit 0,5 N-Ammoniaklösung. Die Ammoniakfraktion wird im Vakuum eingedampft, der Rückstand in 100 ml Wasser gelöst und die Lösung an einen Anionenaustauscher (IRA 67) gebunden. Die Austauschersäule wird mit Wasser und 0,5 N Ameisensäure eluiert. Aus der sauren Fraktion erhält man die Titelverbindung durch Eindampfen im Vakuum. Zur weiteren Reinigung löst man Methanol und gibt gerade soviel Aceton zu, bis sich ein Niederschlag bildet. Man kühlt auf 0 °C, saugt vom Niederschlag ab und erhält 7,7 g (56,3 % der Theorie) der Titelverbindung als hellgelbes Pulver.

| | | | | |
|---|---|---|---|---|
| Analyse: Ber.: | C 49,86 | H 6,01 | N 10,90 | S 8,32 |
| Gef.: | C 49,71 | H 5,85 | N 10,80 | S 8,07 |

### d) Gadoliniumkomplex von 3,6,9-Tris-(carboxymethyl)-14-oxa-3,6,9-triazabicyclo-[9.2.1]tetradecan

3,73 g (10 mMol) der Titelverbindung aus Beispiel 16c) werden in 15 ml Wasser gelöst und 3 Stunden mit 1,81 g (5 mMol) Gadoliniumoxid bei 80 °C gerührt. Die erhaltene Lösung wird filtriert und nacheinander mit je 0,5 g Kationenaustauscher (IR 120) und Anionenaustauscher (IRA 67) ausgerührt, die Lösung erneut filtriert und einer Gefriertrocknung unterzogen. Man erhält 5,07 g (91 % d. Theorie) der Titelverbindung als weißes amorphes Pulver mit einem Wassergehalt von 5,4 %.

| | | | | |
|---|---|---|---|---|
| Analyse: Ber.: | C 36,42 | H 4,59 | N 7,96 | Gd 29,80 |
| Gef.: | C 36,30 | H 4,61 | N 7,82 | Gd 29,59 |
| (Wassergehalt berücksichtigt) | | | | |

### BEISPIEL 17

### a) 3,6,9-Tris(p-tolylsulfonyl)-14-thia-3,6,9-triazabicyclo[9.2.1]tetradeca-1(13),11-dien

Man löst 60,97 g (100 mMol) N,N',N''-Tris(p-tolylsulfonyl)-diethylentriamin-N,N''-dinatriumsalz in 800 ml Dimethylformamid und tropft bei 50 °C innerhalb von 90 Minuten 19,9 g (110 mMol) 2,5-Bis-chlormethylthiophen, gelöst in 330 ml Dimethylformamid, zu. Man rührt noch 90 Minuten bei 50 °C, tropft dann 1 l Wasser zu und saugt den gebildeten Niederschlag ab, wäscht mit Wasser und trocknet den Rückstand bei 50 °C im Vakuumtrockenschrank und kristallisiert aus Dioxan um. Man erhält 47,1 g (70 % der Theorie) der Titelverbindung als hellgelbes Pulver, Schmelzpunkt 265 - 268 °C.

| | | | | |
|---|---|---|---|---|
| Analyse: Ber.: | C 55,25 | H 5,24 | N 6,24 | S 19.03 |
| Gef.: | C 55,38 | H 5,44 | N 6,10 | S 19,01 |

### d) Gadoliniumkomplex von 3,6,9-Tris-(carboxymethyl)-14-thia-3,6,9-triazabicyclo[9.2.1]tetradeca-1(13),11-dien

2 g (5,19 mMol) der Titelverbindung aus Beispiel 17c) werden mit 941 mg (2,60 mMol) Gadoliniumoxid in 20 ml Wasser 4 Stunden auf 85 - 90 °C erhitzt. Die erhaltene Lösung wird filtriert und nacheinander mit je 0,26 g Kationenaustauscher (IR 120) und Anionenaustauscher (IRA 67) ausgerührt, die Lösung erneut filtriert und gefriergetrocknet. Man erhält 2,66 g (95 % der Theorie) der Titelverbindung als weißes amorphes Pulver, Wassergehalt 5,7 %.

| | | | | | |
|---|---|---|---|---|---|
| Analyse: Ber.: | C 35,61 | H 3,74 | Gd 29,14 | N 7,79 | S 5,94 |
| Gef.: | C 35,50 | H 3,51 | Gd 29,02 | N 7,98 | S 6,18 |
| (Wassergehalt bei der Berechnung berücksichtigt) | | | | | |

### BEISPIEL 18

### a) 13-Methoxy-3,6,9-tris(p-tolylsulfonyl)-3,6,9,15-tetraazabicyclo[9.3.1]-pentadeca-1(15),11,13-trien

Man löst 60,97 g (100 mMol) N,N',N''-Tris-(p-tolylsulfonyl)-diethylentriamin-N,N''-dinatriumsalz in 800 ml Dimethylformamid und tropft bei 50 °C innerhalb von 90 Minuten 47,76 g (100 mMol) 2,6-Bis-(p-tolylsulfonyloxymethyl)-4-methoxy-pyridin, gelöst in 400 ml Dimethylformamid. Man rührt noch 5 Stunden bei 90 °C, tropft dann 1,1 l Wasser zu, saugt den gebildeten Niederschlag ab, wäscht mit Wasser, trocknet das Produkt im Vakuumtrockenschrank und kristallisiert aus Isopropylalkohol um. Man erhält 43,3 g (62 % der Theorie) der Titelverbindung als weißes Pulver.

| | | | | |
|---|---|---|---|---|
| Analyse: Ber.: | C 56,71 | H 5,48 | N 8,016 | S 13,76 |
| Gef.: | C 56,90 | H 5,31 | N 8,00 | S 13,59 |

### b) 13-Methoxy-3,6,9,15-tetraazabicyclo[9,3,1]pentadeca-1(15),11,13-trien

30 g (42,9 mMol) der Titelverbindung aus Beispiel 18a) werden mit 100 ml konz. Schwefelsäure 24 Stunden bei 95 °C gerührt. Nach Abkühlen auf 0 °C tropft man 400 ml Ether zu, saugt vom gebildeten Niederschlag ab und löst in 60 ml 40 %iger Natronlauge. Die Lösung wird fünfmal mit je 75 ml Dichlormethan extrahiert, über Magnesiumsulfat getrocknet und im Vakuum eingedampft. Den Rückstand kristallisiert man aus Diisopropylether um und erhält 6,59 g (65 % der Theorie) der Titelverbindung als weißes Pulver.

| | | | |
|---|---|---|---|
| Analyse: Ber.: | C 60,99 | H 8,53 | N 23,71 |
| Gef.: | C 61,15 | H 8,40 | N 23,52 |

### c) 13-Methoxy-3,6,9-tris-(carboxymethyl)-3,6,9,15-tetraazabicyclo[9.3.1]-pentadeca-1(15),11,13-trien

6,2 g (26,2 mMol) der Titelverbindung aus Beispiel 18b) werden in 40 ml Wasser gelöst, mit 9,90 g (104,8 mMol) Chloressigsäure versetzt. Durch Zugabe von 6 N Kalilauge stellt man einen pH-Wert von 9,5 ein und erwärmt 8 Stunden auf 45 - 50 °C. Während dieser Zeit wird der pH-Wert durch Zugabe weiterer Kalilauge bei 9,5 - 10 gehalten. Man kühlt dann im Eisbad ab, versetzt mit konz. Salzsäure bis zu einem pH-Wert von 2. Es bildet sich ein Niederschlag, man saugt ab, löst den Rückstand unter leichtem Erwärmen in 80 ml Wasser und adsorbiert die Lösung an einer Kationenaustauschersäule (IR 120). Man eluiert die Säule zunächst mit reichlich Wasser, dann mit 0,5 N Ammoniaklösung. Das basische Eluat wird gesammelt und im Vakuum eingedampft. Den Rückstand löst man in 80 ml Wasser und adsorbiert die Lösung an einer Anionenaustauschersäule (IRA 67). Man eluiert zunächst mit Wasser, dann mit 0,5 N Ameisensäure. Die saure Fraktion wird im Vakuum eingedampft, der Rückstand in Methanol gelöst und durch Zugabe von Aceton die Titelverbindung ausgefällt. Man erhält 7,42 g (69 % der Theorie) als weißes Pulver.

| | | | |
|---|---|---|---|
| Analyse: Ber.: | C 52,68 | H 6,39 | N 13,65 |
| Gef.: | C 52,81 | H 6,22 | N 13,80 |

### d) Gadoliniumkomplex von 13-Methoxy-3,6,9-tris(carboxymethyl)-3,6,9,15-tetraazabicyclo[9.3.1]pentadeca-1(15),11,13-trien

5 g (12,18 mMol) der Titelverbindung aus Beispiel 18c) werden mit 2,21 g (6,09 mMol) Gadoliniumoxid in 60 ml Wasser 4 Stunden auf 85 - 90 °C erhitzt. Die Lösung wird filtriert und gefriergetrocknet. Man erhält 6,74 g (98 %) der Titelverbindung als weißes amorphes Pulver, Wassergehalt 4,1 %.

| | | | | |
|---|---|---|---|---|
| Analyse: Ber. : | C 38,29 | H 4,10 | Gd 27,85 | N 9,92 |
| Gef.: | C 38,41 | H 3,92 | Gd 27,60 | N 9,99 |
| (Wassergehalt bei der Berechnung berücksichtigt) | | | | |

### BEISPIEL 19

### a) 13-Chlor-3,6,9-tris-(tert.-butoxycarbonylmethyl)-3,6,9,15-tetraazabicyclo-[9.3.1]pentadeca-1(15),11,13-trien

Zu 7 g (29,08 mMol) der Titelverbindung aus Beispiel 5c) und 10,17 g (95,96 mMol) Natriumcarbonat in 200 ml Acetonitril gibt man 18,72 g (95,96 mMol) Bromessigsäure-tert.-butylester zu und rührt 24 Stunden bei Raumtemperatur.

Man dampft im Vakuum ein, nimmt den Rückstand mit 300 ml Wasser auf und extrahiert dreimal mit 200 ml Methylenchlorid. Nach Trocknen der organischen Phasen über Magnesiumsulfat wird im Vakuum eingeengt und das zurückbleibende Öl an Kieselgel chromatographiert (Laufmittel: Methylenchlorid/Ethanol = 15/1).

Ausbeute: 14,08 g (83 % der Theorie) eines farblosen Öls

| | | | | | |
|---|---|---|---|---|---|
| Analyse: Ber.: | C 59,73 | H 8,12 | N 9,61 | O 16,46 | Cl 6,08 |
| Gef.: | C 59,67 | H 8,25 | N 9,58 | | Cl 6,01 |

### b) 13-(N-Pyrrolidino)-3,6,9-tris-(tert.-butoxycarbonylmethyl)-3,6,9,15-tetraazabicyclo[9.3.1]pentadeca-1(15),11,13-trien

Zu 13,5 g (23,15 mMol) der Titelverbindung aus Beispiel 19a), 3,94 g (46,3 mMol) Pyrrolidinon und 612 mg (2,32 mMol) 18-Krone-6 in 200 ml wasserfreiem Dimethylformamid gibt man vorsichtig 1,11 g (46,3 mMol) Natriumhydrid (zuvor mit Pentan gewaschen) hinzu. Man rührt 72 Stunden bei 70 °C unter Stickstoff. Die Lösung wird auf Raumtemperatur abgekühlt und in 1,2 l Eiswasser gegossen. Anschließend wird dreimal mit 250 ml Essigester extrahiert. Die organische Phase wird über Magnesiumsulfat getrocknet und im Vakuum eingedampft. Der Rückstand an Kieselgel (Laufmittel: Methylenchlorid/Methanol: 13/1) chromatographiert.

Ausbeute: 5,7 g (39 % der Theorie) eines farblosen Öls, das beim Stehenlassen kristallisiert.

| | | | | |
|---|---|---|---|---|
| Analyse: Ber.: | C 62,73 | H 8,46 | N 11,09 | O 17,73 |
| Gef.: | C 63,68 | H 8,54 | N 11,01 | |

### c) 13-(N-Pyrrolidino)-3,6,9-tris-(carboxymethyl)-3,6,9,15-tetraazabicyclo-[9.3.1]pentadeca-1(15),11,13-trien

5,1 g (8,07 mMol) der Titelverbindung aus Beispiel 19b) wird in 50 ml Trifluoressigsäure gelöst und 6 Stunden bei Raumtemperatur gerührt. Man zieht das Solvens im Vakuum ab und reinigt wie in Beispiel 1d) beschrieben an einem Anionenaustauscher.

Kristallisation aus MeOH/Aceton liefert 2,88 g (77 % der Theorie) einer stark hygroskopischen Substanz.

| | | | | |
|---|---|---|---|---|
| Analyse: Ber.: | C 54,42 | H 6,31 | N 15,11 | O 24,17 |
| Gef.: | C 54,37 | H 6,42 | N 15,05 | |

### d) Gadolinium-Komplex von 13-(N-Pyrrolidino)3,6,9-tris-(carboxymethyl)-3,6,9,15-tetraazabicyclo[9.3.1]pentadeca-1(15),11,13-trien

2,5 g 15,4 mMol) der Titelverbindung aus Beispiel 19c) werden in 20 ml entionisiertem Wasser gelöst und 978 mg (2,7 mMol) Gadoliniumoxid zugefügt. Man rührt 3 Stunden bei 90 °C. Die Lösung wird filtriert und das Filtrat gefriergetrocknet.

Ausbeute: 3,32 g (100 % der Theorie) eines amorphen Pulvers, das laut Analyse 13,2 % Wasser enthält.

| | | | | | |
|---|---|---|---|---|---|
| Analyse: Ber.: | C 40,83 | H 4,24 | N 11,34 | O 18,13 | Gd 25,46 |
| Gef.: | C 40,74 | H 4,37 | N 11,28 | | Gd 25,41 |

### BEISPIEL 20

### a) 13-Azido-3,6,9-tris-(tert.-butoxycarbonylmethyl)-3,6,9,15-tetraazabicyclo-[9.3.1]pentadeca-1(15),11,13-trien

21 g (36,01 mMol) der Titelverbindung aus Beispiel 19a) werden in 200 ml Dimethylformamid gelöst und 7,02 g (108 mMol) Natriumazid sowie 951 mg (3,6 mMol) 18-Krone-6 zugesetzt. Man rührt 48 Stunden bei 90 °C.

Nach Abkühlen auf Raumtemperatur gießt man in 1,5 l Eiswasser und extrahiert dreimal mit 200 ml Essigester. Nach Trocknen der organischen Phase über Magnesiumsulfat wird eingedampft und das zurückbleibende Öl an Kieselgel (Laufmittel: Methylenchlorid/Ethanol = 15/1) chromatographiert.

Ausbeute: 10,83 g (51 % der Theorie) eines blaßgelben Öls.

| | | | | |
|---|---|---|---|---|
| Analyse: Ber.: | C 59,06 | H 8,03 | N 16,63 | O 16,28 |
| Gef.: | C 59,17 | H 8,05 | N 16,51 | |

### b) 13-Amino-3,6,9-tris-(tert.-butoxycarbonylmethyl)-3,6,9,15-tetraazabicyclo-[9.3.1]pentadeca-1(15),11,13-trien

10 g (16,96 mMol) der Titelverbindung aus Beispiel 20a) werden in 400 ml Ethanol gelöst und 1 g Pearlman-Katalysator (20 % Palladiumhydioxid auf Kohle) zugesetzt. Nach 24stündiger Hydrierung bei Normaldruck saugt man vom Katalysator ab und dampft im Vakuum ein. Das zurückbleibende Öl wird an Kieselgel (Laufmittel: Methylenchlorid/Methanol/Triethylamin = 10/1/0,05) chromatographiert. Man erhält 8,89 g (93 % der Theorie) eines leicht gelblichen Öls.

| | | | | |
|---|---|---|---|---|
| Analyse: Ber.: | C 61,78 | H 8,76 | N 12,42 | O 17,03 |
| Gef.: | C 61,67 | H 8,91 | N 12,35 | |

### c) 13-Amino-3,6,9-tris-(carboxymethyl)-3,6,9,15-tetraazabicyclo[9.3.1]pentadeca-1(15),11,13-trien

8,2 g (14,55 mMol) der Titelverbindung aus Beispiel 20b) werden in 100 ml Trifluoressigsäure gelöst und 6 Stunden bei Raumtemperatur gerührt. Nach Abdampfen des Lösungsmittels im Vakuum wird der Rückstand in 100 ml Wasser gelöst und über eine Säule, gefüllt mit Poly(4-vinyl-Pyridin), gegeben. Nach Eindampfen im Vakuum und Kristallisation aus Methanol/Aceton erhält man 5,24 g (91 % d. Theorie) stark hygroskopischen Feststoff.

| | | | | |
|---|---|---|---|---|
| Analyse: Ber.: | C 51,64 | H 6,37 | N 17,71 | O 24,28 |
| Gef.: | C 51,74 | H 6,31 | N 17,63 | |

### d) Gadolinium-Komplex von 13-Amino-3,6,9-tris-(carboxymethyl)-3,6,9,15-tetraazabicyclo[9.3.1]pentadeca-1(15),11,13-trien

4,8 g (12,14 mMol) der Titelverbindung aus Beispiel 20c) werden in 35 ml entionisiertem Wasser gelöst und 2,2 g (6,07 mMol) Gadoliniumoxid zugesetzt. Man rührt 3 Stunden bei 90 °C und hält den pH-Wert durch Zugabe von Essigsäure bei 5,5. Die Lösung wird filtriert und über eine Säule, gefüllt mit Poly(4-vinyl-pyridin), gegeben. Nach Behandlung mit Aktivkohle wird erneut filtriert und gefriergetrocknet.

Ausbeute: 6,07 g (91 % der Theorie) eines amorphen Pulvers, das laut Analyse 12,1 % Wasser enthält.

| | | | | | |
|---|---|---|---|---|---|
| Analyse: Ber.: | C 37,15 | H 4,06 | N 12,74 | O 17,47 | Gd 28,61 |
| Gef.: | C 37,08 | H 4,17 | N 12,68 | | Gd 28,54 |

### BEISPIEL 21

### a) 13-(Hydroxyacetamido)-3,6,9-tris-(tert.-butoxycarbonylmethyl)-3,6,9,15-tetraazabicyclo[9.3.1]pentadeca-1(15),11,13-trien

5,8 g (10,28 mMol) der Titelverbindung aus Beispiel 20b), 861 mg (11,32 mMol) Glykolsäure und 1,53 g (11,32 mMol) 1-Hydroxy-1H-benzotriazol Hydrat werden in 20 ml absolutem Dimethylformamid gelöst und auf 0 °C abgekühlt. Man gibt 2,36 g (11,32 mMol) Dicyclohexylcarbodiimid hinzu und rührt 1 Stunde bei 0 °C; dann über Nacht bei Raumtemperatur. Die Lösung wird in 150 ml Eiswasser gegeben und dreimal mit 150 ml Essigester extrahiert. Nach Trocknen der organischen Phase über Magnesiumsulfat wird im Vakuum eingedampft. Der Rückstand wird an Kieselgel (Laufmittel: Methylenchlorid/Methanol = 10/1) chromatographiert.

Ausbeute: 2,88 g (45 % der Theorie) eines farblosen Feststoffs.

| | | | | |
|---|---|---|---|---|
| Analyse: Ber.: | C 59,88 | H 8,27 | N 11,26 | O 20,59 |
| Gef.: | C 59,76 | H 8,35 | N 11,31 | |

### b) 13-(Hydroxyacetamido)-3,6,9-tris-(carboxymethyl)-3,6,9,15-tetraazabicyclo-[9.3.1]pentadeca-1(15),11,13-trien

2,7 g (4,34 mMol) der Titelverbindung aus Beispiel 21a) werden in 40 ml Trifluoressigsäure gelöst und 6 Stunden bei Raumtemperatur gerührt. Man dampft im Vakuum ein und reinigt den Rückstand wie in Beispiel 1d) beschrieben an einem Anionenaustauscher.

Kristallisation aus Isopropanol liefert 1,56 (79 % der Theorie) eines weißen Pulvers.

| | | | | |
|---|---|---|---|---|
| Analyse: Ber.: | C 50,32 | H 6,00 | N 15,45 | O 28,23 |
| Gef.: | C 50,24 | H 6,07 | N 15,49 | |

### c) Gadolinium-Komplex von 13-(Hydroxyacetamido)-3,6,9-tris-(carboxymethyl)-3,6,9,15-tetraazabicyclo[9.3.1]pentadeca-1(15),11,13-trien

1,45 g (3,2 mMol) der Titelverbindung aus Beispiel 21b) wird in 10 ml entionisiertem Wasser gelöst und 580 mg (1,6 mMol) Gadoliniumoxid zugesetzt. Man rührt 3 Stunden bei 90 °C. Die Lösung wird filtriert und das Filtrat gefriergetrocknet.

Ausbeute: 1,94 g (100 % der Theorie) eines amorphen Pulvers, das laut Analyse 11,5 % Wasser enthält

| | | | | | |
|---|---|---|---|---|---|
| Analyse: Ber.: | C 37,55 | H 3,98 | N 11,53 | O 21,06 | Gd 25,88 |
| Gef.: | C 37,48 | H 4,11 | N 11,48 | | Gd 25,79 |

### BEISPIEL 22

### a) 13-Chlor-3,6,9-tris-(p-tolylsulfonyl)-3,6,9-triazabicyclo[9.3.1.]pentadeca-1(15),11,13-trien

182,85 g (300 mMol) N,N',N''-Tris(p-tolylsulfonyl)-diethylentriamin-N-N''-dinatriumsalz werden in 2,4 l Dimethylformamid gelöst und auf 100 °C erwärmt. Hierzu tropft man innerhalb von 3 Stunden eine Lösung aus 63,15 g (300 mMol) 4-Chlor-2,6-bis(chlormethyl)-pyridin in 1 l Dimethylformamid. Man rührt über Nacht bei 100 °C.

In die noch heiße Lösung tropft man 3 l Wasser und kühlt auf Raumtemperatur ab. Der Niederschlag wird mit viel Wasser gewaschen und im Vakuum (60 °C) getrocknet. Kristallisation aus Acetonitril ergibt 128,7 g (61 % d. Theorie) der Titelverbindung als farbloses Pulver.

| | | | | | | |
|---|---|---|---|---|---|---|
| Analyse: Ber.: | C 54,65 | H 5,02 | N 7,97 | O 13,65 | S 13,68 | Cl 5,04 |
| Gef.: | C 54,61 | H 5,13 | N 7,91 | | S 13,65 | Cl 5,09 |

### b) 13-(N-Morpholino)-3,6,9-tris-(p-tolylsulfonyl)-3,6,9-triazabicyclo-[9.3.1]pentadeca-1(15),11,13-trien

126 g (179 mMol) der Titelverbindung aus Beispiel 22a) werden in 500 ml Dimethylsulfoxid gelöst und 87,12 g (1 mol) Morpholin zugesetzt. Die Lösung wird in einem Autoklaven 48 Stunden bei 140 °C und 10 bar gerührt. Man kühlt ab, gießt auf 3 l Eiswasser und saugt den Niederschlag ab. Nach Trocknen im Vakuum bei 60 °C, wird aus Aceton umkristallisiert. Man erhält 87,72 g (65 % der Theorie) als cremefarbenes Pulver.

| | | | | | |
|---|---|---|---|---|---|
| Analyse: Ber.: | C 57,35 | H 5,75 | N 9,29 | O 14,86 | S 12,76 |
| Gef.: | C 57,32 | H 5,84 | N 9,18 | | S 12,82 |

### c) 13-(N-Morpholino)-3,6,9,15-tetraazabicyclo[9.3.1]pentadeca-1(15),11,13-trien

86 g (114 mMol) der Titelverbindung aus Beispiel 22b) werden in 270 ml konz. Schwefelsäure eingetragen und 48 Stunden bei 100 °C gerührt. Man kühlt auf 0 °C und tropft 1,35 l absoluten Ether hinzu. Der Niederschlag wird abgesaugt und in 100 ml wäßriger Natronlauge (pH 12) suspendiert. Man extrahiert 7mal mit 150 ml Chloroform und trocknet die vereinigten organischen Phasen über Magnesiumsulfat. Nach Eindampfen im Vakuum erhält man 22,26 g (67 % der Theorie) eines gelblichen Öls, das beim Stehenlassen kristallisiert.

| | | | | |
|---|---|---|---|---|
| Analyse: Ber.: | C 61,82 | H 8,65 | N 24,04 | O 5,49 |
| Gef.: | C 61,89 | H 8,59 | N 24,13 | |

### d) 13-(N-Morpholino)-3,6,9-tris-(carboxymethyl)-3,6,9,15-tetraazabicyclo-[9.3.1]pentadeca-1(15),11,13-trien

10 g (34,3 mMol) der Titelverbindung aus Beispiel 22c) werden in 150 ml Wasser gelöst und 12,85 g (136 mMol) Chloressigsäure zugefügt. Man stellt mit 6 n Kalilauge auf pH 9,5. Es wird 12 Stunden bei 45 °C gerührt und der pH-Wert durch Zugabe von 6 n Kalilauge zwischen 9,5 - 10 gehalten. Man stellt mit konz. Salzsäure auf pH 2 und reinigt an Ionenaustauschern wie in Beispiel 1d) beschrieben. Kristallisation aus Methanol/Aceton liefert 9,9 g (62 % der Theorie) der Titelverbindung als stark hygroskopischen Feststoff.

| | | | | |
|---|---|---|---|---|
| Analyse: Ber.: | C 54,18 | H 6,71 | N 15,05 | O 24,06 |
| Gef.: | C 54,09 | H 6,82 | N 15,01 | |

### e) Gadolinium-Komplex von 13-(N-Morpholino)-3,6,9-tris-(carboxymethyl)-3,6,9,15-tetraazabicyclo[9.3.1]pentadeca-1(15),11,13-trien

9 g (19,33 mMol) der Titelverbindung aus Beispiel 22d) werden in 60 ml entionisiertem Wasser gelöst und 3,5 g (9,67 mMol) Gadoliniumoxid zugefügt. Man rührt 3 Stunden bei 90 °C und hält den pH-Wert durch Zugabe von Essigsäure bei 5,5. Die Lösung wird filtriert und über eine Säule, gefüllt mit Poly(4-vinyl-pyridin) gegeben. Nach Behandlung mit Aktivkohle wird erneut filtriert und gefriergetrocknet.

Ausbeute: 10,9 g (91 % der Theorie) eines amorphen Pulvers, das laut Analyse 9,87 % Wasser enthält.

| | | | | | |
|---|---|---|---|---|---|
| Analyse: Ber.: | C 40,70 | H 4,55 | N 11,30 | O 18,07 | Gd 25,37 |
| Gef.: | C 40,63 | H 4,64 | N 11,25 | | Gd 25,28 |

### BEISPIEL 23

### a) 13-Chlor-3,6,9-Tris-(benzyl)-3,6,9,15-tetraazabicyclo[9.3.1]pentadeca-1(15),11,13-trien

9,3 g (38,62 mMol) der Titelverbindung aus Beispiel 5c) und 21,36 g (154,5 mMol) Kaliumcarbonat werden in 200 ml Dimethylformamid gelöst und auf 70 °C erwärmt. Innerhalb 30 Minuten werden 26,43 g (154,5 mMol) Benzylbromid zugetropft und 24 Stunden bei 70 °C gerührt.

Man zieht das Solvens im Vakuum ab und nimmt den Rückstand in 250 ml 3 n Natronlauge auf. Es wird 5mal mit 150 ml Methylenchlorid extrahiert und die organischen Phasen über Magnesiumsulfat getrocknet. Nach Eindampfen im Vakuum wird an Kieselgel chromatographiert (Laufmittel: Isopropanol/Triethylamin = 20:1).

Ausbeute: 17,97 g (91 % der Theorie) eines zartgelben Öls.

| | | | | |
|---|---|---|---|---|
| Analyse: Ber.: | C 75,20 | H 6,90 | N 10,97 | Cl 6,93 |
| Gef.: | C 75,11 | H 6,98 | N 10,85 | Cl 7,06 |

### b) 13-Carboxy-3,6,9-Tris-(benzyl)-3,6,9,15-tetraazabicyclo[9.3.1]pentadeca-1(15),11,13-trien

Zu 1,95 g (79,44 mMol) Magnesiumspänen wird eine Lösung aus 17,5 g (34,24 mMol) der Titelverbindung aus Beispiel 23a) in 80 ml 1,2-Dimethoxyethan zugetropft und zum Sieden erhitzt. Hierzu tropft man über einen Zeitraum von 12 Stunden eine Lösung aus 6,43 g (34,24 mMol) 1,2-Dibromethan in 40 ml 1,2-Dimethoxyethan.
Man kühlt im Eisbad ab und schüttet die Lösung vorsichtig auf 10 g Trockeneis. Nach 3 Stunden Rühren bei Raumtemperatur fügt man vorsichtig 200 ml Wasser zu und bringt mit Salzsäure auf pH 4. Man dampft zur Trockne ein und kocht den Rückstand mit 200 ml Ethanol aus. Nach Abfiltrieren der Magnesiumsalze wird erneut zur Trockne eingedampft und der Rückstand an Kieselgel (Laufmittel: Chloroform/Methanol/Triethylamin = 20/15/1) chromatographiert.

Ausbeute: 5,16 g (29 % der Theorie) eines zartgelben Feststoffes.

| | | | | |
|---|---|---|---|---|
| Analyse: Ber.: | C 76,27 | H 6,79 | N 10,78 | O 6,16 |
| Gef.: | C 76,19 | H 6,88 | N 10,71 | |

### c) 13-(Morpholinocarbonyl)-3,6,9-Tris-(benzyl)-3,6,9,15-tetraazabicyclo-[9.3.1]pentadeca-1(15),11,13-trien

5,0 g (9,62 mMol) der Titelverbindung aus Beispiel 23b), 922 mg (10,58 mMol) Morpholin und 1,43 g (10,58 mMol) 1-Hydroxy-1H-benzotriazol Hydrat werden in 10 ml absolutem Dimethylformamid gelöst und auf 0 °C gekühlt. Man setzt 2,18 g (10,58 mMol) Dicyclohexylcarbodiimid hinzu und rührt 1 Stunde bei 0 °C; dann über Nacht bei Raumtemperatur.
Die Lösung wird in 180 ml Eiswasser gegossen und 3mal mit 150 ml Chloroform extrahiert. Nach Trocknen der organischen Phase über Magnesiumsulfat wird im Vakuum eingeengt. Der Rückstand wird an Kieselgel (Laufmittel: Chloroform/Methanol/Triethylamin = 20/5/1) chromatographiert.

Man erhält 4,22 g (88 % der Theorie) der Titelverbinduöng als farbloses Öl.

| | | | | |
|---|---|---|---|---|
| Analyse: Ber.: | C 75,48 | H 7,19 | N 11,90 | O 5,44 |
| Gef.: | C 75,37 | H 7,27 | N 11,83 | |

### d) 13-(Morpholinocarbonyl)-3,6,9,15-Tetraazabicyclo[9.3.1]pentadeca-1(15),11,13-trien

4,1 g (6,96 mMol) der Titelverbindung aus Beispiel 23c) werden in 250 ml Ethanol gelöst und 0,5 g Pearlman-Katalysator (20 % Palladiumhydroxid auf Kohle) zugesetzt. Nach 24stündiger Hydrierung im Autoklaven (50 °C und 3 bar Wasserstoffdruck) saugt man vom Katalysator ab und engt im Vakuum ein. Der Rückstand wird aus 30 ml Tetrahydrofuran umkristallisiert.

Ausbeute: 1,85 g (83 % der Theorie) der Titelverbindung als weißes kristallines Pulver.

| | | | | |
|---|---|---|---|---|
| Analyse: Ber.: | C 60,16 | H 7,89 | N 21,93 | O 10,02 |
| Gef.: | C 60,08 | H 7,97 | N 21,81 | |

### e) 13-(Morpholinocarbonyl)-3,6,9-tris-(carboxymethyl)-3,6,9,15-tetraazabicyclo[9.3.1]pentadeca-1(15),11,13-trien

1,6 g (5,0 mMol) der Titelverbindung aus Beispiel 23d) werden in 25 ml Wasser gelöst und 1,89 g (20 mMol) Chloressigsäure zugesetzt. Man stellt mit 6 n Kalilauge auf pH 9,5. Es wird 12 Stunden bei 45 °C gerührt und der pH-Wert durch Zugabe von 6 n Kalilauge zwischen 9,5 - 10 gehalten.

Nach Aufarbeitung an Ionenaustauschern wie in Beispiel 1d) beschrieben erhält man nach Kristallisation aus Methanol/Aceton 1,66 g (67 % d. Theorie) eines stark hygroskopischen Feststoffs.

| | | | | |
|---|---|---|---|---|
| Analyse: Ber.: | C 53,54 | H 6,33 | N 14,19 | O 25,94 |
| Gef.: | C 53,41 | H 6,47 | N 14,08 | |

### f) Gadolinium-Komplex von 13-(Morpholinocarbonyl)-3,6,9-tris(carboxymethyl)-3,6,9,15-tetraazabicyclo[9.3.1]pentadeca-1(15),11,13-trien

1,5 g (3,04 mMol) der Titelverbindung aus Beispiel 23e) werden in 10 ml entionisiertem Wasser gelöst und 551 mg (1,52 mMol) Gadoliniumoxid Zugesetzt. Man rührt 3 Stunden bei 90 °C. Die Lösung wird filtriert und das Filtrat gefriergetrocknet.

Ausbeute: 1,97 g (100 % der Theorie) eines weißen amorphen Pulvers, das laut Analyse 10,1 % Wasser enthält.

| | | | | | |
|---|---|---|---|---|---|
| Analyse: Ber.: | C 40,79 | H 4,36 | N 10,81 | O 19,76 | Gd 24,28 |
| Gef.: | C 40,71 | H 4,44 | N 10,89 | | Gd 24,17 |

### Beispiel für die NMR-Diagnostik in vivo:

Einer Nacktmaus Balb/c nu/nu, weiblich, 20g, mit subcutanem HT 29 Coloncarcinom wurden nach Voraufnahme im Kernspintomographen (Hersteller: General Electric, 2 Tesla) 0,1 mmol Gadolinium-Komplex von 3,6,9-Tris-(carboxymethyl)-3,6,9,15-tetraazabicyclo-[9.3.1]pentadeca-1(15),11,13-trien [Beispiel 1e)] pro kg i.v. in eine Caudalvene appliziert. Die Substanz war gelöst in bidestilliertem Wasser (pH 7,2). Es wurden Aufnahmen in Spin-Echo-Sequenz T_{R} = 400 msec., T_{E} = 30 msec gemacht.

Die Aufnahmen wurden vor sowie 1, 23 und 43 Minuten nach Applikation des Kontrastmittels im Bereich der Leber und des Tumors gemacht.

Es konnte gezeigt werden, daß die Signalintensität im Tumor anstieg und über den beobachteten Zeitraum nicht wieder abfiel. 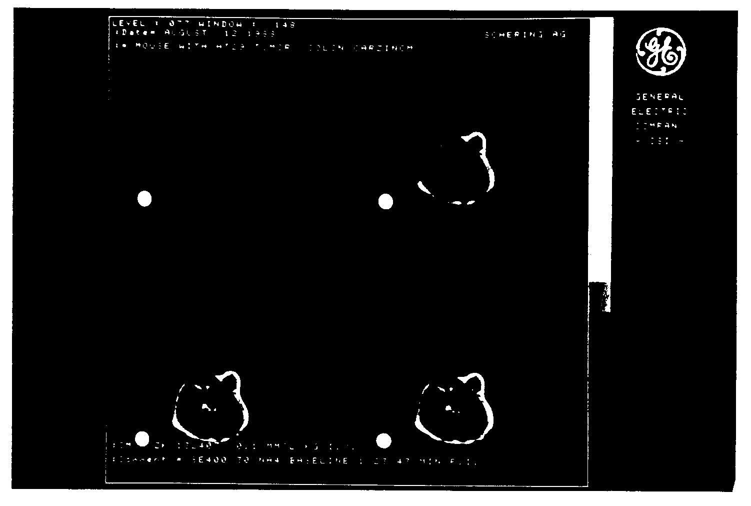
Die Abbildung zeigt eine Nacktmaus Balb/c nu/nu mit HT 29 Coloncarcinom im Transversalschnitt vor und nach i.v. Applikation des Kontrastmittels. Die Aufnahmen wurden in Spin-Echo-Sequenz T_{R} = 400 msec, T_{E} = 30 msec gemacht.

Links oben ist die Maus zu sehen vor Kontrastmittelapplikation. Die Aufnahme zeigt die Leber und den subcutanen Tumor. Die weiteren Aufnahmen wurden 1, 23 und 43 Minuten nach Applikation gemacht.

## Patentansprüche

1. Makrocyclische Verbindungen der allgemeinen Formel I 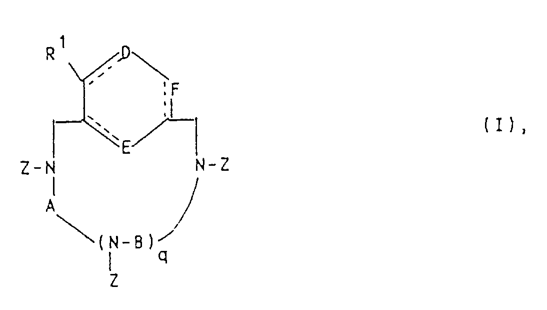 worin
.... für eine Einfach- oder Doppelbindung,
q für die Ziffern 0-5,
A und B, die gleich oder verschieden sind, jeweils für eine geradkettige oder verzweigte Alkylengruppe mit 2 bis 6 Kohlenstoffatomen,
D für ein Stickstoff-, Sauerstoffatom, die Gruppe =C=O, =NR², -CHR³- oder =CR³-,
E für ein Stickstoff-, Schwefel-, Sauerstoffatom, die 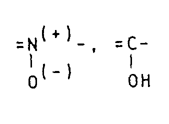 oder >NR⁴-Gruppe,
F für (-CHR⁸-)ₙ oder (=CR⁸)ₙ,
R¹ für ein Wasserstoff- oder Halogenatom oder eine C₁-C₆-Alkylgruppe,
Z für ein Wasserstoffatom oder die Gruppe -CH₂COOY mit Y in der Bedeutung eines Wasserstoffatoms und/oder eines Metallionenäquivalents eines Elements der Ordnungszahlen 21-29, 31, 32, 37-39, 42-44, 49 oder 51-83,
R² für ein Wasserstoffatom oder eine C₁-C₆-Alkylgruppe,
R³ für ein Wasserstoff- oder Halogenatom, eine Phenyl- oder eine C₁-C₆-Alkylgruppe, die gegebenenfalls durch eine oder mehrere Phenyl- und/oder Hydroxygruppe(n) substituiert ist, den Rest OR⁵, den Substituenten 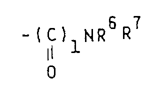 oder den Substituenten G,
R⁴ für eine Hydroxygruppe, für R² oder eine gegebenenfalls hydroxylierte oder carboxylierte C₁-C₆-Alkylgruppe,
R⁵ für einen gegebenenfalls durch 1 bis 3 Hydroxygruppe(n) substituierten C₁-C₆-Alkylrest,
R⁶ und R⁷ unabhängig voneinander für Wasserstoffatome, für den Rest R⁵, für gegebenenfalls durch 1 bis 3 Hydroxygruppen substituierte Phenyl- oder Benzylreste oder R⁶ und R⁷ gemeinsam mit dem Stickstoffatom für einem gesättigten oder ungesättigten, gegebenenfalls ein weiteres Stickstoff-, Sauerstoff-, Schwefelatom oder eine Carbonylgruppe enthaltenden 5- oder 6-Ring, der gegebenenfalls substituiert ist durch 1 bis 3 Reste R⁵, oder einer der Substituenten R⁶ oder R⁷ für den Rest 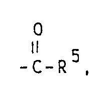
R⁸ für R¹ oder G,
l für die Ziffern 0 oder 1.
n für die Ziffern 0 oder 1,
G für einen über eine direkte Bindung, eine Bis(carbonylamino)gruppe (-NH-CO-CO-NH-) oder über eine C₁-C₂₀-Alkylengruppe, die gegebenenfalls an den Enden Carbonyl-(>CO) oder Carbonylamino(-NH-CO)-gruppen oder Sauerstoffatome trägt und gegebenenfalls ein oder mehrere Sauerstoffatom(e), Z-, acyl- oder hydroxyacylsubstituierte Iminogruppen oder ein bis zwei C-C-Doppel- und/oder C-C-Dreifachbindungen enthält, gebundenen zweiten Makrocyclus der allgemeinen Formel II 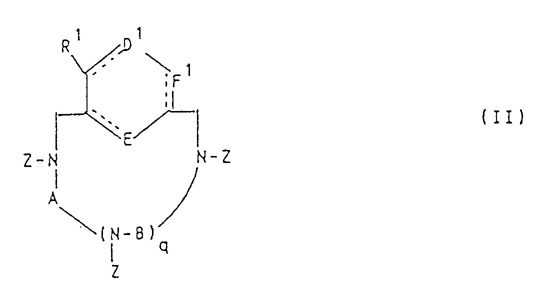 stehen,
worin
D¹ die gleiche Bedeutung wie D hat, mit der Ausnahme, daß D¹ nicht den Substituenten G enthält, oder für den Rest 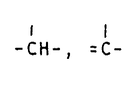 oder 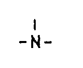 steht und
F¹ die gleiche Bedeutung wie F hat, mit der Ausnahme, daß F¹ nicht den Substituenten G enthält, oder für den Rest 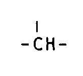 oder 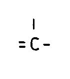 steht,
sowie deren Salze mit anorganischen und/oder organischen Basen, Aminosäuren oder Aminosäureamiden,
mit den Maßgaben, daß mindestens zwei der Substituenten Z für den Rest -CH₂COOY stehen, daß die makrocyclische Verbindung der allgemeinen Formel I nicht mehr als einen Rest G enthält und daß die allgemeine Formel I nicht für 3,6,9,12,18-Pentaazabicyclo[12.3.1]octadeca-1(18),14,16-trien-N-tetraessigsäure oder 3,6,9,15-Tetraazabicyclo[9.3.1]-pentadeca-1(15),11,13-trien-N-triessigsäure oder deren Cu-, Pb-, Co- und Sr-Komplexe steht.

2. Verbindungen gemäß Anspruch 1, dadurch gekennzeichnet, daß Y Wasserstoffatome darstellt.

3. Verbindungen gemäß Anspruch 1, dadurch gekennzeichnet, daß mindestens zwei der Substituenten Y Metallionenäquivalente mindestens eines Elements der Ordnungszahlen 21-29, 42, 44 oder 57-83 oder mindestens eines Radionuklids eines Elements der Ordnungszahlen 27, 29, 31, 32, 37-39, 43, 49, 62, 64, 70 oder 77 sind.

4. Verbindungen gemäß Anspruch 1, dadurch gekennzeichnet, daß G für eine über eine
-(CH₂)₁₋₆, -O-(CH₂)₁₋₆-O-, 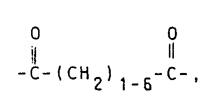 -C≡C-C≡C-, 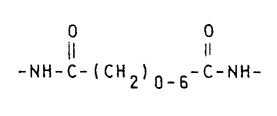 Gruppe verbundenen Ring der allgemeinen Formel II steht.

5. Pharmazeutische Mittel enthaltend mindestens eine physiologisch verträgliche makrocyclische Verbindung der allgemeinen Formel I 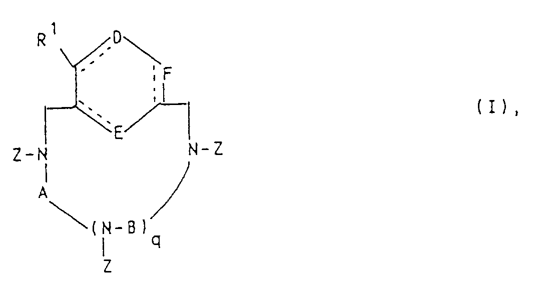 worin
.... für eine Einfach- oder Doppelbindung,
q für die Ziffern 0-5,
A und B, die gleich oder verschieden sind, jeweils für eine geradkettige oder verzweigte Alkylengruppe mit 2 bis 6 Kohlenstoffatomen,
D für ein Stickstoff-, Sauerstoffatom, die Gruppe =C=O, =NR², -CHR³ - oder =CR³-,
E für ein Stickstoff-, Schwefel-, Sauerstoffatom, die 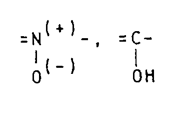 oder >NR⁴-Gruppe,
F für (-CHR⁸-)ₙ oder (=CR⁸)ₙ,
R¹ für ein Wasserstoff- oder Halogenatom oder eine C₁-C₆-Alkylgruppe,
Z für ein Wasserstoffatom oder die Gruppe -CH₂COOY mit Y in der Bedeutung eines Wasserstoffatoms und/oder eines Metallionenäquivalents eines Elements der Ordnungszahlen 21-29, 31, 32, 37-39, 42-44, 49 oder 57-83,
R² für ein Wasserstoffatom oder eine C₁-C₆-Alkylgruppe,
R³ für ein Wasserstoff- oder Halogenatom, eine Phenyl- oder eine C₁-C₆-Alkylgruppe, die gegebenenfalls durch eine oder mehrere Phenyl- und/oder Hydroxygruppe(n) substituiert ist, den Rest OR⁵, den Substituenten 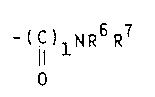 oder den Substituenten G,
R⁴ für eine Hydroxygruppe, für R² oder eine gegebenenfalls hydroxylierte oder carboxylierte C₁-C₆-Alkylgruppe,
R⁵ für einen gegebenenfalls durch 1 bis 3 Hydroxygruppe(n) substituierten C₁-C₆-Alkylrest,
R⁶ und R⁷ unabhängig voneinander für Wasserstoffatome, für den Rest R⁵, für gegebenenfalls durch 1 bis 3 Hydroxygruppen substituierte Phenyl- oder Benzylreste oder R⁶ und R⁷ gemeinsam mit dem Stickstoffatom für einen gesättigten oder ungesättigten, gegebenenfalls ein weiteres Stickstoff-, Sauerstoff-, Schwefelatom oder eine Carbonylgruppe enthaltenden 5- oder 6-Ring, der gegebenenfalls substituiert ist durch 1 bis 3 Reste R⁵, oder einer der Substituenten R⁶ oder R⁷ für den Rest 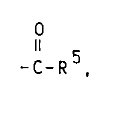
R⁸ für R¹ oder G,
l für die Ziffern 0 oder 1,
n für die Ziffern 0 oder 1
G für einen über eine direkte Bindung, eine Bis(carbonylamino)gruppe (-NH-CO-CO-NH-) oder über eine C₁-C₂₀-Alkylengruppe, die gegebenenfalls an den Enden Carbonyl-(>CO) oder Carbonylamino(-NH-CO)-gruppen oder Sauerstoffatome trägt und gegebenenfalls ein oder mehrere Sauerstoffatom(e), Z-, acyl- oder hydroxyacylsubstituierte Iminogruppen oder ein bis zwei C-C-Doppel- und/oder C-C-Dreifachbindungen enthält, gebundenen zweiten Makrocyclus der allgemeinen Formel II 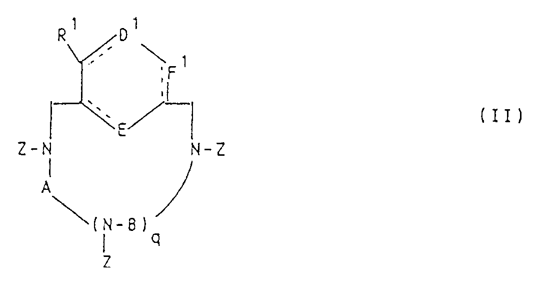 stehen,
worin
D¹ die gleiche Bedeutung wie D hat, mit der Ausnahme, daß D¹ nicht den Substituenten G enthält, oder für den Rest -CH-, =C- oder -N- steht un
F¹ die gleiche Bedeutung wie F hat, mit der Ausnahme, daß F¹ nicht den Substituenten G enthält, oder für den Rest -CH- oder =C- steht,
sowie deren Salze mit anorganischen und/oder organischen Basen, Aminosäuren oder Aminosäureamiden,
mit den Maßgaben, daß mindestens zwei der Substituenten Z für den Rest -CH₂COOY stehen und daß die makrocyclische Verbindung der allgemeinen Formel I nicht mehr als einen Rest G enthält, gegebenenfalls mit den in der Galenik üblichen Zusätzen.

6. Verwendung von mindestens einer physiologisch verträglichen Verbindung der allgemeinen Formel I 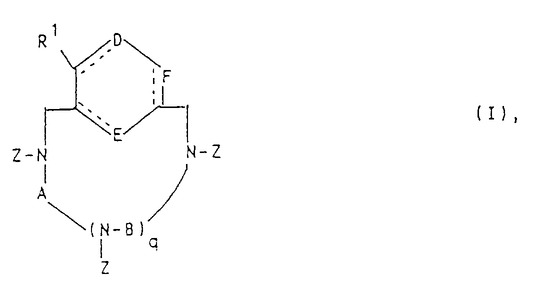 worin
.... für eine Einfach- oder Doppelbindung,
q für die Ziffern 0-5,
A und B, die gleich oder verschieden sind, jeweils für eine geradkettige oder verzweigte Alkylengruppe mit 2 bis 6 Kohlenstoffatomen,
D für ein Stickstoff-, Sauerstoffatom, die Gruppe =C=O, =NR², -CHR³ - oder =CR³-,
E für ein Stickstoff-, Schwefel-, Sauerstoffatom, die 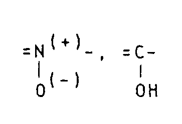 oder >NR⁴-Gruppe,
F für (-CHR⁸-)ₙ oder (=CR⁸)ₙ,
R¹ für ein Wasserstoff- oder Halogenatom oder eine C₁-C₆-Alkylgruppe,
Z für ein Wasserstoffatom oder die Gruppe -CH₂COOY mit Y in der Bedeutung eines Wasserstoffatoms und/oder eines Metallionenäquivalents eines Elements der Ordnungszahlen 21-29, 31, 32, 37-39, 42-44, 49 oder 57-83,
R² für ein Wasserstoffatom oder eine C₁-C₆-Alkylgruppe,
R³ für ein Wasserstoff- oder Halogenatom, eine Phenyl- oder eine C₁-C₆-Alkylgruppe, die gegebenenfalls durch eine oder mehrere Phenyl- und/oder und/oder Hydroxygruppe(n) substituiert ist, den Rest OR⁵, den Substituenten 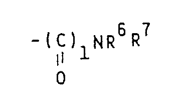 oder den Substituenten G,
R⁴ für eine Hydroxygruppe, für R² oder eine gegebenenfalls hydroxylierte oder carboxylierte C₁-C₆-Alkylgruppe,
R⁵ für einen gegebenenfalls durch 1 bis 3 Hydroxygruppe(n) substituierten C₁-C₆-Alkylrest,
R⁶ und R⁷ unabhängig voneinander für Wasserstoffatome, für den Rest R⁵, für gegebenenfalls durch 1 bis 3 Hydroxygruppen substituierte Phenyl- oder Benzylreste oder R⁶ und R⁷ gemeinsam mit dem Stickstoffatom für einen gesättigten oder ungesättigten, gegebenenfalls ein weiteres Stickstoff-, Sauerstoff-, Schwefelatom oder eine Carbonylgruppe enthaltenden 5- oder 6-Ring, der gegebenenfalls substituiert ist durch 1 bis 3 Reste R⁵, oder einer der Substituenten R⁶ oder R⁷ für den Rest 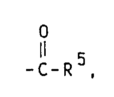
R⁸ für R¹ oder G,
l für die Ziffern 0 oder 1,
n für die Ziffern 0 oder 1,
G für einen über eine direkte Bindung, eine Bis(carbonylamino)gruppe (-NH-CO-CO-NH-) oder über eine C₁-C₂₀-Alkylengruppe, die gegebenenfalls an den Enden Carbonyl-(>CO) oder Carbonylamino(-NH-CO)-gruppen oder Sauerstoffatome trägt und gegebenenfalls ein oder mehrere Sauerstoffatom(e), Z-, acyl- oder hydroxyacylsubstituierte Iminogruppen oder ein bis zwei C-C-Doppel- und/oder C-C-Dreifachbindungen enthält, gebundenen zweiten Makrocyclus der allgemeinen Formel II 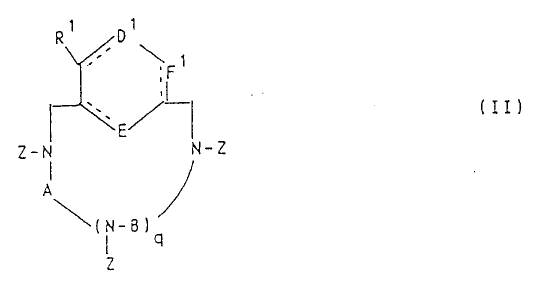 stehen,
worin
D¹ die gleiche Bedeutung wie D hat, mit der Ausnahme, daß D¹ nicht den Substituenten G enthält, oder für den Rest 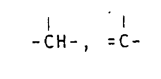 oder 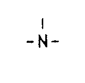 steht un
F¹ die gleiche Bedeutung wie F hat, mit der Ausnahme, daß F¹ nicht den Substituenten G enthält, oder für den Rest 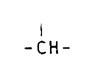 oder 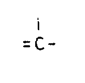 steht,
sowie deren Salze mit anorganischen und/oder organischen Basen, Aminosäuren oder Aminosäureamiden,
mit den Maßgaben, daß mindestens zwei der Substituenten Z für den Rest -CH₂COOY stehen, daß die makrocyclische Verbindung der allgemeinen Formel I nicht mehr als einen Rest G enthält und daß mindestens zwei der Substituenten Y Metallionenäquivalente mindestens eines Elements der Ordnungszahlen 21-29, 42, 44 oder 57-83 oder mindestens eines Radionuklids eines Elements der Ordnungszahlen 27, 29, 31, 32, 37-39, 43, 49, 62, 64, 70 oder 77 sind
für die Herstellung von Mitteln für die NMR-, Röntgen-, Radio-Diagnostik, Radio- oder Strahlen-Therapie.

7. Verfahren zur Herstellung von makrocyclischen Verbindungen der allgemeinen Formel I 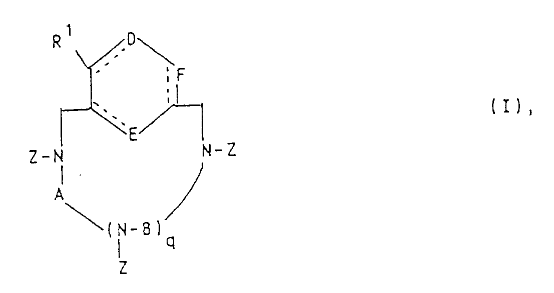 worin
.... für eine Einfach- oder Doppelbindung,
q für die Ziffern 0-5,
A und B, die gleich oder verschieden sind, jeweils für eine geradkettige oder verzweigte Alkylengruppe mit 2 bis 6 Kohlenstoffatomen,
D für ein Stickstoff-, Sauerstoffatom, die Gruppe =C=O, =NR², -CHR³- oder =CR³-,
E für ein Stickstoff-, Schwefel-, Sauerstoffatom, die 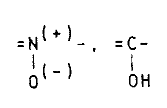 oder >NR⁴-Gruppe,
F für (-CHR⁸-)ₙ oder (=CR⁸)ₙ,
R¹ für ein Wasserstoff- oder Halogenatom oder eine C₁-C₆-Alkylgruppe,
Z für ein Wasserstoffatom oder die Gruppe -CH₂COOY mit Y in der Bedeutung eines Wasserstoffatoms und/oder eines Metallionenäquivalents eines Elements der Ordnungszahlen 21-29, 31, 32, 37-39, 42-44, 49 oder 57-83,
R² für ein Wasserstoffatom oder eine C₁-C₆-Alkylgruppe,
R³ für ein Wasserstoff- oder Halogenatom, eine Phenyl- oder eine C₁-C₆-Alkylgruppe, die gegebenenfalls durch eine oder mehrere Phenyl- und/oder Hydroxygruppe(n) substituiert ist, den Rest OR⁵, den Substituenten 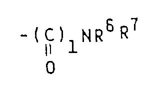 oder den Substituenten G,
R⁴ für eine Hydroxygruppe, für R² oder eine gegebenenfalls hydroxylierte oder carboxylierte C₁-C₆-Alkylgruppe,
R⁵ für einen gegebenenfalls durch 1 bis 3 Hydroxygruppe(n) substituierten C₁-C₆-Alkylrest,
R⁶ und R⁷ unabhängig voneinander für Wasserstoffatome, für den Rest R⁵, für gegebenenfalls durch 1 bis 3 Hydroxygruppen substituierte Phenyl- oder Benzylreste oder R⁶ und R⁷ gemeinsam mit dem Stickstoffatom für einen gesättigten oder ungesättigten, gegebenenfalls ein weiteres Stickstoff-, Sauerstoff-, Schwefelatom oder eine Carbonylgruppe enthaltenden 5- oder 6-Ring, der gegebenenfalls substituiert ist durch 1 bis 3 Reste R⁵, oder einer der Substituenten R⁶ oder R⁷ für den Rest 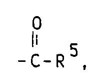
R⁸ für R¹ oder G,
l für die Ziffern 0 oder 1,
n für die Ziffern 0 oder 1,
G für einen über eine direkte Bindung, eine Bis(carbonylamino)gruppe (-NH-CO-CO-NH-) oder über eine C₁-C₂₀-Alkylengruppe, die gegebenenfalls an den Enden Carbonyl-(>CO) oder Carbonylamino(-NH-CO)-gruppen oder Sauerstoffatome trägt und gegebenenfalls ein oder mehrere Sauerstoffatom(e), Z-, acyl- oder hydroxyacylsubstituierte Iminogruppen oder ein bis zwei C-C-Doppel- und/oder C-C-Dreifachbindungen enthält, gebundenen zweiten Makrocyclus der allgemeinen Formel II 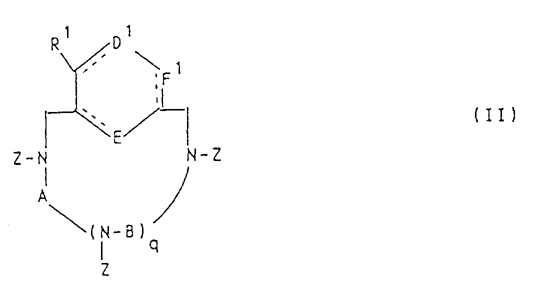 stehen,
worin
D¹ die gleiche Bedeutung wie D hat, mit der Ausnahme, daß D¹ nicht den Substituenten G enthält, oder für den Rest 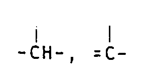 oder 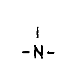 steht un
F¹ die gleiche Bedeutung wie F hat, mit der Ausnahme, daß F¹ nicht den Substituenten G enthält, oder für den Rest 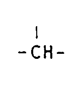 oder 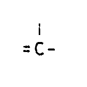 steht,
sowie deren Salze mit anorganischen und/oder organischen Basen, Aminosäuren oder Aminosäureamiden,
mit den Maßgaben, daß mindestens zwei der Substituenten Z für den Rest -CH₂COOY stehen, daß die makrocyclische Verbindung der allgemeinen Formel I nicht mehr als einen Rest G enthält und daß die allgemeine Formel I nicht für 3,6,9,12,18-Pentaazabicyclo[12.3.1]octadeca-1(18),14,16-trien-N-tetraessigsäure oder 3,6,9,15-Tetraazabicyclo[9.3.1]-pentadeca-1(15),11,13-trien-N-triessigsäure oder deren Cu-, Pb-, Co- und Sr-Komplexe steht,
dadurch gekennzeichnet, daß man in an sich bekannter Weise Verbindungen der allgemeinen Formel I' 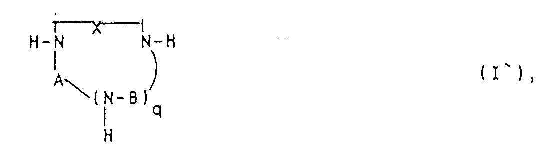 worin
X für 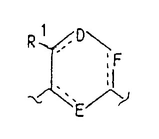 oder für einen in den gewünschten Ring umzuwandelnden 5- oder 6-Ring steht,
mit einer Halogenverbindung III
HalCH₂COOY' (III),
worin Hal für Chlor, Brom oder Jod und
Y' für ein Wasserstoffatom oder eine Säureschutzgruppe steht,
alkyliert und anschließend, gegebenenfalls nach Umwandlung von X in den gewünschten 5- oder 6-Ring des Endprodukts sowie gegebenenfalls nach Abspaltung der Schutzgruppen Y', gewünschtenfalls die so erhaltenen Komplexbildner der allgemeinen Formel I mit Y in der Bedeutung von Wasserstoff in an sich bekannter Weise mit mindestens einem Metalloxid oder Metallsalz eines Elements der Ordnungszahlen 21-29, 31, 32, 37-39, 42-44, 49 oder 57-83 umsetzt und anschließend, falls gewünscht, noch vorhandene acide Wasserstoffatome durch Kationen anorganischer und/oder organischer Basen, Aminosäuren oder Aminosäureamide substituiert.

8. Verfahren zur Herstellung der pharmazeutischen Mittel gemäß Anspruch 5, dadurch gekennzeichnet, daß man die in Wasser oder physiologischer Salzlösung gelöste oder suspendierte Komplexverbindung, gegebenenfalls mit den in der Galenik üblichen Zusätzen, in eine für die enterale oder parenterale Applikation geeignete Form bringt.

## Claims

1. Macrocyclic compounds of the general formula I 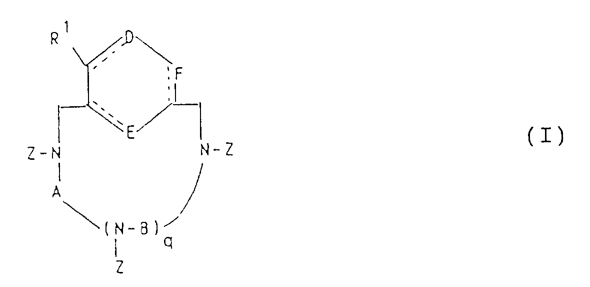 wherein
.... represents a single bond or a double bond,
q represents the numbers 0 to 5,
A and B, which are the same or different, each represent a straight-chained or branched alkylene group having from 2 to 6 carbon atoms,
D represents a nitrogen atom, an oxygen atom, the group =C=O, =NR², -CHR³- or =CR³-,
E represents a nitrogen atom, a sulphur atom, an oxygen atom, the group 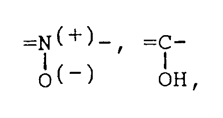 or >NR⁴,
F represents (-CHR⁸-)ₙ or (=CR⁸)ₙ,
R¹ represents a hydrogen atom or a halogen atom or a C₁-C₆-alkyl group,
Z represents a hydrogen atom or the group -CH₂COOY wherein Y represents a hydrogen atom and/or a metal ion equivalent of an element of atomic numbers 21-29, 31, 32, 37-39, 42-44, 49 or 57-83,
R² represents a hydrogen atom or a C₁-C₆-alkyl group,
R³ represents a hydrogen atom or a halogen atom, a phenyl group or a C₁-C₆-alkyl group optionally substituted by one or more phenyl and/or hydroxy groups, or represents the radical OR⁵, the substituent 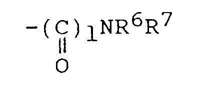 or the substituent G,
R⁴ represents a hydroxy group, R² or an optionally hydroxylated or carboxylated C₁-C₆-alkyl group,
R⁵ represents a C₁-C₆-alkyl radical optionally substituted by one or more phenyl and/or hydroxy groups,
R⁶ and R⁷, independently of each other, represent hydrogen atoms, the radical R⁵, or phenyl or benzyl radicals optionally substituted by from 1 to 3 hydroxy groups, or R⁶ and R⁷, together with the nitrogen atom, form a saturated or unsaturated 5- or 6-membered ring that optionally contains a further nitrogen, oxygen or sulphur atom or a carbonyl group and that is optionally substituted by from 1 to 3 radicals R⁵, or one of the substituents R⁶ and R⁷ represents the radical 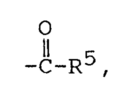
R⁸ represents R¹ or G,
l represents the number 0 or 1,
n represents the number 0 or 1,
G represents a second macrocycle of the general formula II that is bonded via a direct bond, a bis(carbonylamino) group (-NH-CO-CO-NH-) or via a C₁-C₂₀-alkylene group that optionally carries carbonyl (>CO) or carbonylamino (-NH-CO-) groups or oxygen atoms at the ends and that optionally contains one or more oxygen atoms, Z-, acyl- or hydroxyacyl-substitinted imino groups or one or two C-C-double and/or C-C-triple bonds: 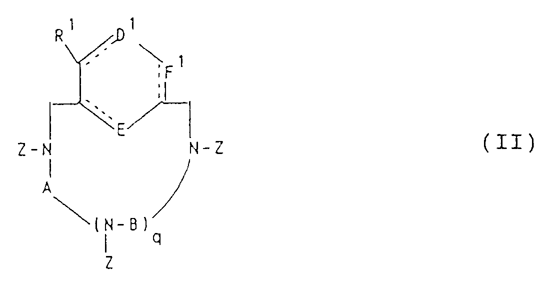
wherein
D¹ has the same meaning as D, except that D¹ does not contain the substituent G, or D¹ represents the radical 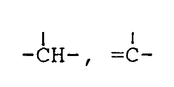 or 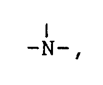 and
F¹ has the same meaning as F, except that F¹ does not contain the substituent G, or F¹ represents the radical 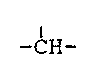 or 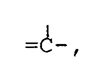
and their salts with inorganic and/or organic bases, amino acids or amino acid amides,
with the provisos that at least two of the substituents Z represent the radical -CH₂COOY, that the macrocyclic compound of the general formula I does not contain more than one radical G, and that the general formula I does not represent 3,6,9,12,18-pentaazabicyclo[12.3.1]octadeca-1(18),14,16-triene-N-tetraacetic acid or 3,6,9,15-tetraazabicyclo[9.3.1]pentadeca-1(15),11,13-triene-N-triacetic acid or their Cu, Pb, Co and Sr complexes.

2. Compounds according to claim 1, characterised in that Y represents hydrogen atoms.

3. Compounds according to claim 1, characterised in that at least two of the substituents Y are metal ion equivalents of at least one element of atomic numbers 21-29, 42, 44 or 57-83 or of at least one radionuclide of an element of atomic numbers 27, 29, 31, 32, 37-39, 43, 49, 62, 64, 70 or 77.

4. Compounds according to claim 1, characterised in that G represents a ring of the general formula II that is bonded via a group -(CH₂)₁₋₆-, -O-(CH₂)₁₋₆-O-, 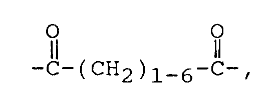 -C≡C-C≡C- or

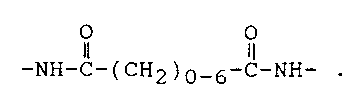

5. Pharmaceutical agents comprising at least one physiologically tolerable macrocyclic compound of the general formula I 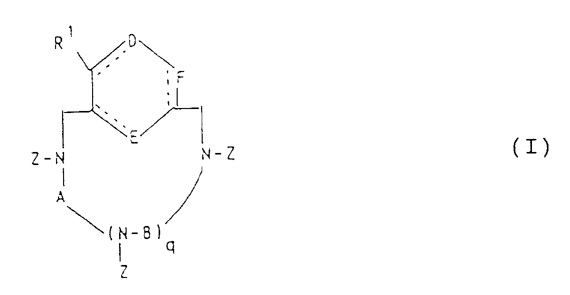 wherein
.... represents a single bond or a double bond,
q represents the numbers 0 to 5,
A and B, which are the same or different, each represent a straight-chained or branched alkylene group having from 2 to 6 carbon atoms,
D represents a nitrogen atom, an oxygen atom, the group =C=O, =NR², -CHR³- or =CR³-,
E represents a nitrogen atom, a sulphur atom, an oxygen atom, the group 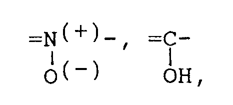 or >NR⁴,
F represents (-CHR⁸-)ₙ or (=CR⁸)ₙ,
R¹ represents a hydrogen atom or a halogen atom or a C₁-C₆-alkyl group,
Z represents a hydrogen atom or the group -CH₂COOY wherein Y represents a hydrogen atom and/or a metal ion equivalent of an element of atomic numbers 21-29, 31, 32, 37-39, 42-44, 49 or 57-83,
R² represents a hydrogen atom or a C₁-C₆-alkyl group,
R³ represents a hydrogen atom or a halogen atom, a phenyl group or a C₁-C₆-alkyl group optionally substituted by one or more phenyl and/or hydroxy groups, or represents the radical OR⁵, the substituent 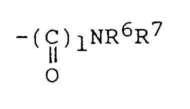 or the substituent G,
R⁴ represents a hydroxy group, R² or an optionally hydroxylated or carboxylated C₁-C₆-alkyl group,
R⁵ represents a C₁-C₆-alkyl radical optionally substituted by one or more phenyl and/or hydroxy groups,
R⁶ and R⁷, independently of each other, represent hydrogen atoms, the radical R⁵, or phenyl or benzyl radicals optionally substituted by from 1 to 3 hydroxy groups, or R⁶ and R⁷, together with the nitrogen atom, form a saturated or unsaturated 5- or 6-membered ring that optionally contains a further nitrogen, oxygen or sulphur atom or a carbonyl group and that is optionally substituted by from 1 to 3 radicals R⁵, or one of the substituents R⁶ and R⁷ represents the radical 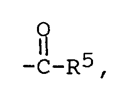
R⁸ represents R¹ or G,
l represents the number 0 or 1,
n represents the number 0 or 1,
G represents a second macrocycle of the general formula II that is bonded via a direct bond, a bis(carbonylamino) group (-NH-CO-CO-NH-) or via a C₁-C₂₀-alkylene group that optionally carries carbonyl (>CO) or carbonylamino (-NH-CO-) groups or oxygen atoms at the ends and that optionally contains one or more oxygen atoms, Z-, acyl- or hydroxyacyl-substituted imino groups or one or two C-C-double and/or C-C-triple bonds: 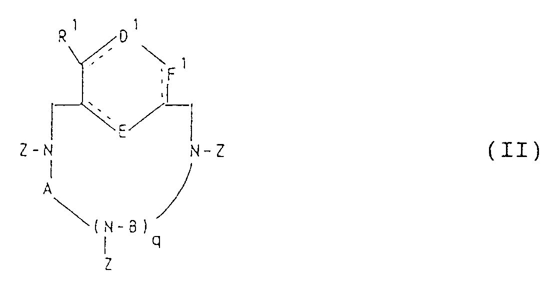
wherein
D¹ has the same meaning as D, except that D¹ does not contain the substituent G, or D¹ represents the radical 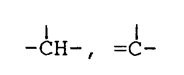 or 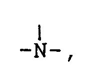 and
F¹ has the same meaning as F, except that F¹ does not contain the substituent G, or F¹ represents the radical 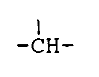 or 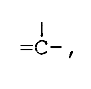
and their salts with inorganic and/or organic bases, amino acids or amino acid amides,
with the provisos that at least two of the substituents Z represent the radical -CH₂COOY, and that the macrocyclic compound of the general formula I does not contain more than one radical G,
optionally together with additives customary in galenic pharmacy.

6. Use of at least one physiologically tolerable compound of the general formula I 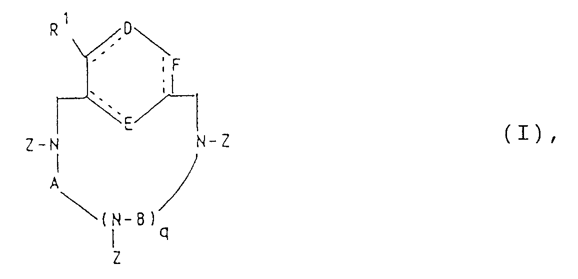 wherein
.... represents a single bond or a double bond,
q represents the numbers 0 to 5,
A and B, which are the same or different, each represent a straight-chained or branched alkylene group having from 2 to 6 carbon atoms,
D represents a nitrogen atom, an oxygen atom, the group =C=O, -NR², -CHR³- or =CR³-,
E represents a nitrogen atom, a sulphur atom, an oxygen atom, the group 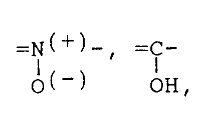 or >NR⁴,
F represents (-CHR⁸-)ₙ or (=CR⁸)ₙ,
R¹ represents a hydrogen atom or a halogen atom or a C₁-C₆-alkyl group,
Z represents a hydrogen atom or the group -CH₂COOY wherein Y represents a hydrogen atom and/or a metal ion equivalent of an element of atomic numbers 21-29, 31, 32, 37-39, 42-44, 49 or 57-83,
R² represents a hydrogen atom or a C₁-C₆-alkyl group,
R³ represents a hydrogen atom or a halogen atom, a phenyl group or a C₁-C₆-alkyl group optionally substituted by one or more phenyl and/or hydroxy groups, or represents the radical OR⁵, the substituent 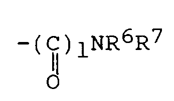 or the substituent G,
R⁴ represents a hydroxy group, R² or an optionally hydroxylated or carboxylated C₁-C₆-alkyl group,
R⁵ represents a C₁-C₆-alkyl radical optionally substituted by one or more phenyl and/or hydroxy groups,
R⁶ and R⁷, independently of each other, represent hydrogen atoms, the radical R⁵, or phenyl or benzyl radicals optionally substituted by from 1 to 3 hydroxy groups, or R⁶ and R⁷, together with the nitrogen atom, form a saturated or unsaturated 5- or 6-membered ring that optionally contains a further nitrogen, oxygen or sulphur atom or a carbonyl group and that is optionally substituted by from 1 to 3 radicals R⁵, or one of the substituents R⁶ and R⁷ represents the radical 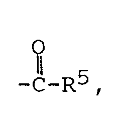
R⁸ represents R¹ or G,
l represents the number 0 or 1,
n represents the number 0 or 1,
G represents a second macrocycle of the general formula II that is bonded via a direct bond, a bis(carbonylamino) group (-NH-CO-CO-NH-) or via a C₁-C₂₀-alkylene group that optionally carries carbonyl (>CO) or carbonylamino (-NH-CO-) groups or oxygen atoms at the ends and that optionally contains one or more oxygen atoms, Z-, acyl- or hydroxyacyl-substituted imino groups or one or two C-C-double and/or C-C-triple bonds: 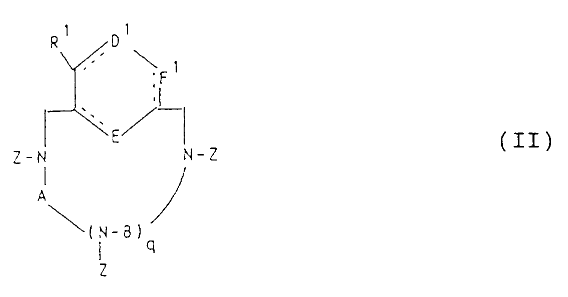
wherein
D¹ has the same meaning as D, except that D¹ does not contain the substituent G, or D¹ represents the radical 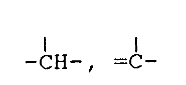 or 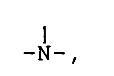 and
F¹ has the same meaning as F, except that F¹ does not contain the substituent G, or F¹ represents the radical 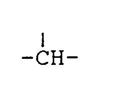 or 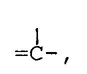
and their salts with inorganic and/or organic bases, amino acids or amino acid amides,
with the provisos that at least two of the substituents Z represent the radical -CH₂COOY, that the macrocyclic compound of the general formula I does not contain more than one radical G, and that at least two of the substituents Y are metal ion equivalents of at least one element of atomic numbers 21-29, 42, 44 or 57-83 or of at least one radionuclide of an element of atomic numbers 27, 29, 31, 32, 37-39, 43, 49, 62, 64, 70 or 77,
for the preparation of agents for NMR, X-ray or radiodiagnostics, radiotherapy or radiation therapy.

7. Process for the preparation of macrocyclic compounds of the general formula I 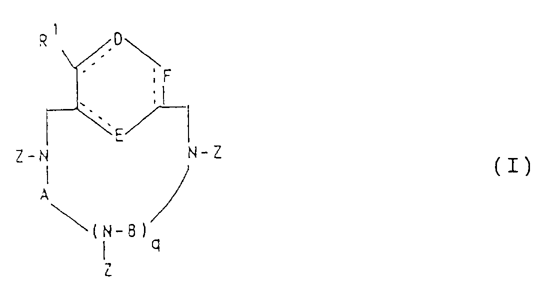 wherein
.... represents a single bond or a double bond,
q represents the numbers 0 to 5,
A and B, which are the same or different, each represent a straight-chained or branched alkylene group having from 2 to 6 carbon atoms,
D represents a nitrogen atom, an oxygen atom, the group =C=O, =NR², -CHR³- or =CR³-,
E represents a nitrogen atom, a sulphur atom, an oxygen atom, the group 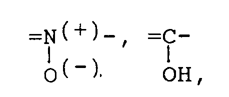 or >NR⁴,
F represents (-CHR⁸-)ₙ or (=CR⁸)ₙ,
R¹ represents a hydrogen atom or a halogen atom or a C₁-C₆-alkyl group,
Z represents a hydrogen atom or the group -CH₂COOY wherein Y represents a hydrogen atom and/or a metal ion equivalent of an element of atomic numbers 21-29, 31, 32, 37-39, 42-44, 49 or 57-83,
R² represents a hydrogen atom or a C₁-C₆-alkyl group,
R³ represents a hydrogen atom or a halogen atom, a phenyl group or a C₁-C₆-alkyl group optionally substituted by one or more phenyl and/or hydroxy groups, or represents the radical OR⁵, the substituent 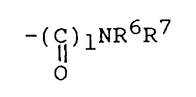 or the substituent G,
R⁴ represents a hydroxy group, R² or an optionally hydroxylated or carboxylated C₁-C₆-alkyl group,
R⁵ represents a C₁-C₆-alkyl radical optionally substituted by one or more phenyl and/or hydroxy groups,
R⁶ and R⁷, independently of each other, represent hydrogen atoms, the radical R⁵, or phenyl or benzyl radicals optionally substituted by from 1 to 3 hydroxy groups, or R⁶ and R⁷, together with the nitrogen atom, form a saturated or unsaturated 5- or 6-membered ring that optionally contains a further nitrogen, oxygen or sulphur atom or a carbonyl group and that is optionally substituted by from 1 to 3 radicals R⁵, or one of the substituents R⁶ and R⁷ represents the radical 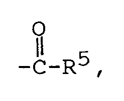
R⁸ represents R¹ or G,
l represents the number 0 or 1,
n represents the number 0 or 1,
G represents a second macrocycle of the general formula II that is bonded via a direct bond, a bis(carbonylamino) group (-NH-CO-CO-NH-) or via a C₁-C₂₀-alkylene group that optionally carries carbonyl (>CO) or carbonylamino (-NH-CO-) groups or oxygen atoms at the ends and that optionally contains one or more oxygen atoms, Z-, acyl- or hydroxyacyl-substituted imino groups or one or two C-C-double and/or C-C-triple bonds: 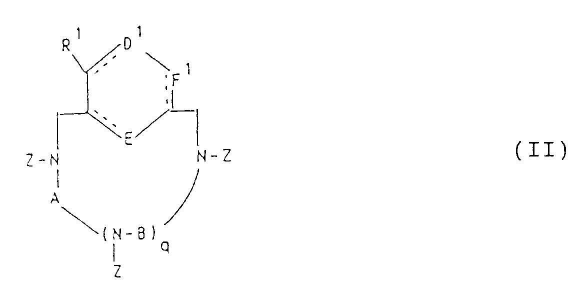 wherein
D¹ has the same meaning as D, except that D¹ does not contain the substituent G, or D¹ represents the radical 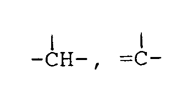 or 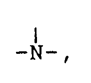 and
F¹ has the same meaning as F, except that F¹ does not contain the substituent G, or F¹ represents the radical 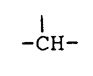 or 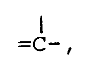
and their salts with inorganic and/or organic bases,
amino acids or amino acid amides,
with the provisos that at least two of the substituents Z represent the radical -CH₂COOY, that the macrocyclic compound of the general formula I does not contain more than one radical G, and that the general formula I does not represent 3,6,9,12,18-pentaazabicyclo[12.3.1]octadeca-1(18),14,16-triene-N-tetraacetic acid or 3,6,9,15-tetraazabicyclo[9.3.1]pentadeca-1(15),11,13-triene-N-triacetic acid or their Cu, Pb, Co and Sr complexes,
characterised in that compounds of the general formula I' 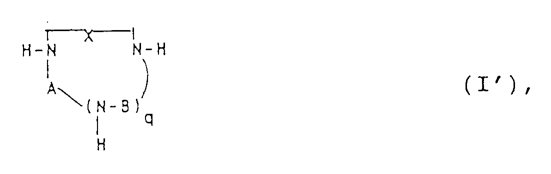 wherein
X represents 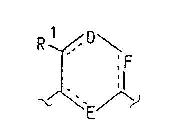 or a 5- or 6-membered ring for conversion into the desired ring,
are alkylated in a manner known per se with a halogen compound III
HalCH₂COOY' (III),
wherein Hal represents chlorine, bromine or iodine and Y' represents a hydrogen atom or an acid-protecting group,
and then, where appropriate after conversion of X into the desired 5- or 6-membered ring of the end product and, where appropriate, after removal of the protecting groups Y', the resulting complexing agents of the general formula I wherein Y represents hydrogen are, if desired, reacted with at least one metal oxide or metal salt of an element of atomic numbers 21-29, 31, 32, 37-39, 42-44, 49 or 57-83 and then, if desired, any acidic hydrogen atoms that are still present are substituted by cations of inorganic and/or organic bases, amino acids or amino acid amides.

8. Process for the preparation of the pharmaceutical agents according to claim 5, characterised in that the complex compound dissolved or suspended in water or physiological saline solution, optionally together with additives customary in galenic pharmacy, is brought into a form suitable for enteral or parenteral administration.

## Revendications

1. Composés macrocycliques répondant à la formule générale I 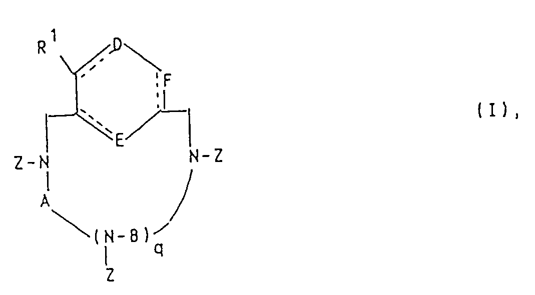 dans laquelle
.... représente une simple ou double liaison,
q représente les chiffres 0 à 5,
A et B, qui sont identiques ou différents, représentent chacun un groupe alkylène linéaire ou ramifié comportant 2 à 6 atomes de carbone,
D représente un atome d'azote, un atome d'oxygène, ou le groupe =C=O, =NR², -CHR³- ou =CR³-,
E représente un atome d'azote, de soufre ou d'oxygène ou le groupe 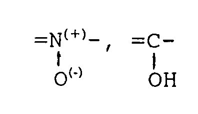 ou >NR⁴,
F représente (-CHR⁸-)ₙ ou (=CR⁸)ₙ,
R¹ représente un atome d'hydrogène ou d'halogène ou un groupe alkyle en C₁-C₆,
Z représente un atome d'hydrogène ou le groupe -CH₂COOY, où Y représente un atome d'hydrogène et/ou un équivalent d'ion métallique d'un élément des nombres atomiques 21-29, 31, 32, 37-39, 42-44, 49 ou 57-83,
R² représente un atome d'hydrogène ou un groupe alkyle en C₁-C₆,
R³ représente un atome d'hydrogène ou d'halogène ou un groupe phényle ou alkyle en C₁-C₆, qui est éventuellement substitué par un ou plusieurs groupes phényle et/ou hydroxy, le radical OR⁵, le substituant 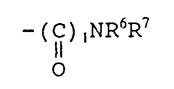 ou le substituant G,
R⁴ représente un groupe hydroxy, R² ou un groupe alkyle en C₁-C₆ éventuellement hydroxylé ou carboxylé,
R⁵ représente un radical alkyle en C₁-C₆ éventuellement substitué par un ou plusieurs groupes phényle et/ou hydroxy,
R⁶ et R⁷ représentent, indépendamment l'un de l'autre, des atomes d'hydrogène, le radical R⁵, des radicaux phényle ou benzyle éventuellement substitués par 1 à 3 groupes hydroxy, R⁶ et R⁷ pouvant former conjointement avec l'atome d'azote un noyau pentagonal ou hexagonal saturé ou insaturé, contenant éventuellement un autre atome d'azote, d'oxygène, ou de soufre ou un groupe carbonyle, ce noyau étant éventuellement substitué par 1 à 3 radicaux R⁵, un des substituants R⁶ ou R⁷ pouvant représenter le radical 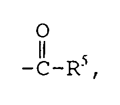
R⁸ représente R¹ ou G,
l représente les chiffres 0 ou 1,
n représente les chiffres 0 ou 1,
G représente un deuxième macrocycle qui est fixé par une liaison directe, un groupe bis(carbonylamino) (-NH-CO-CO-NH) ou un groupe alkylène en C₁-C₂₀ qui porte éventuellement aux extrémités des groupes carbonyle(>CO) ou carbonylamino(-NH-CO) ou des atomes d'oxygène et contient éventuellement un ou plusieurs atomes d'oxygène, Z, des groupes imino substitués par de l'acyle ou de l'hydroxyacyle ou une à deux doubles et/ou triples liaisons carbone-carbone, et qui répond à la formule générale II 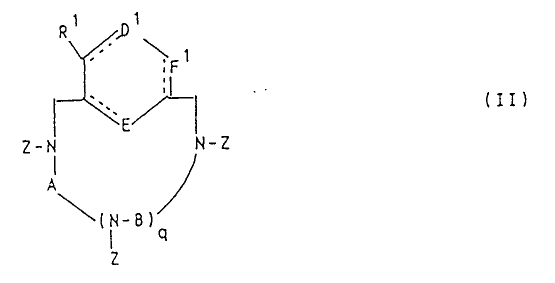
dans laquelle
D¹ a la même signification que D à l'exception que D¹ ne contient pas de substituant G, ou représente le radical 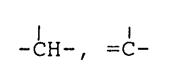 ou 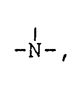 et
F¹ a la même signification que F, à l'exception que F¹ ne contient pas de substituant G, ou représente un radical 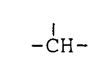 ou 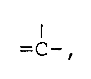
ainsi que leurs sels avec des bases inorganiques et/ou organiques, des aminoacides ou des amides d'aminoacides, à la condition qu'au moins deux des substituants Z représentent le radical -CH₂COOY, que le composé macrocyclique de la formule générale I ne contienne pas plus qu'un radical G et que la formule générale I ne représente pas de l'acide 3,6,9,12,18-pentaazabicyclo-[12.3.1]octadéca-1(18),14,16-triène-N-tétraacétique ou de l'acide 3,6,9,15-tétraazabicyclo(9.3.1]pentadéca-1(15),11,13-triène-N-triacétique ou leurs complexes de Cu, Pb, Co et Sr.

2. Composés suivant la revendication 1, caractérisés en ce que Y représente des atomes d'hydrogène.

3. Composés suivant la revendication 1, caractérisés en ce qu'au moins deux des substituants Y sont des équivalents d'ions métalliques d'au moins un élément des nombres atomiques 21-29, 42, 44 ou 57-83 ou d'au moins un radionucléïde d'un élément des nombres atomiques 27, 29, 31, 32, 37-39, 43, 49, 62, 64, 70 ou 77.

4. Composés suivant la revendication 1, caractérisés en ce que G représente un noyau de la formule générale II relié par l'intermédiaire d'un groupe -(CH₂)₁₋₆, -O-(CH₂)₁₋₆-O-, 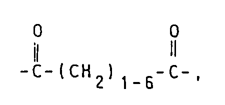 -C≡C-C≡C-,

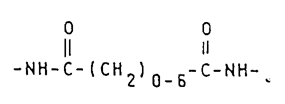

5. Produit pharmaceutique contenant au moins un composé macrocyclique physiologiquement compatible de la formule générale I 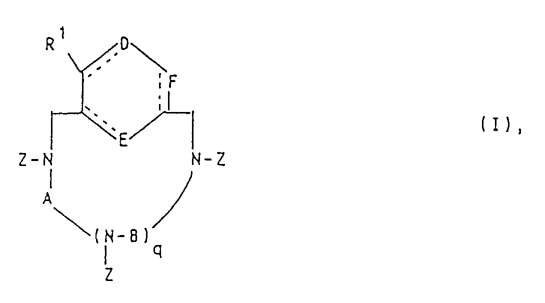 dans laquelle
.... représente une simple ou double liaison,
q représente les chiffres 0-5,
A et B, qui sont identiques ou différents, représentent chacun un groupe alkylène linéaire ou ramifié comportant 2 à 6 atomes de carbone,
D représente un atome d'azote, un atome d'oxygène, ou le groupe =C=O, =NR², -CHR³- ou =CR³-,
E représente un atome d'azote, de soufre ou d'oxygène ou le groupe 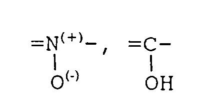 ou >NR⁴,
F représente (CHR⁸-)ₙ ou (=CR⁸)ₙ,
R¹ représente un atome d'hydrogène ou d'halogène ou un groupe alkyle en C₁-C₆,
Z représente un atome d'hydrogène ou le groupe -CH₂COOY, où Y a la signification d'un atome d'hydrogène et/ou d'un équivalent d'ion métallique d'un élément des nombres atomiques 21-29, 31, 32, 37-39, 42-44, 49 ou 57-83,
R² représente un atome d'hydrogène ou un groupe alkyle en C₁-C₆,
R³ représente un atome d'hydrogène ou d'halogène, un groupe phényle ou alkyle en C₁-C₆, qui est éventuellement substitué par un ou plusieurs groupes phényle et/ou hydroxy, le radical OR⁵, le substituant 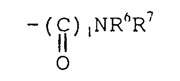 ou le substituant G,
R⁴ représente un groupe hydroxy, R² ou un groupe alkyle en C₁-C₆ éventuellement hydroxylé ou carboxylé,
R⁵ représente un radical alkyle en C₁-C₆ éventuellement substitué par un ou plusieurs groupes phényle et/ou hydroxy,
R⁶ et R⁷ représentent, indépendamment l'un de l'autre, des atomes d'hydrogène, le radical R⁵, ou des radicaux phényle ou benzyle éventuellement substitués par 1 à 3 groupes hydroxy, R⁶ et R⁷ pouvant former conjointement avec l'atome d'azote un noyau pentagonal ou hexagonal saturé ou insaturé, contenant éventuellement un autre atome d'azote, d'oxygène ou de soufre ou un groupe carbonyle, noyau qui peut éventuellement être substitué par 1 à 3 radicaux R⁵, un des substituants R⁶ ou R⁷ pouvant représenter le radical 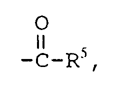
R⁸ représente R¹ ou G,
l représente les chiffres 0 ou 1,
n représente les chiffres 0 ou 1,
G représente un deuxième macrocycle lié par une liaison directe, un groupe bis(carbonylamino) (-NH-CO-CO-NH-) ou un groupe alkylène en C₁-C₂₀, qui porte éventuellement aux extrémités des groupes carbonyle (>CO) ou carbonylamino (-NH-CO) ou des atomes d'oxygène et contient éventuellement un ou plusieurs atomes d'oxygène, Z, des groupes imino substitués par de l'acyle ou de l'hydroxyacyle ou une à deux doubles et/ou triples liaisons carbone-carbone, et qui répond à la formule générale II 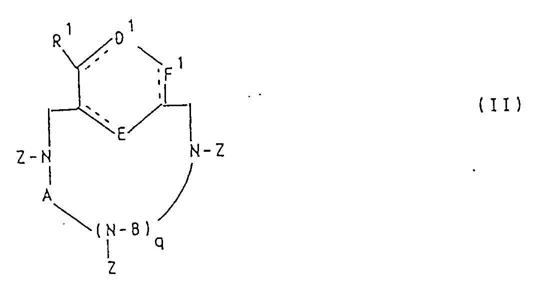
dans laquelle
D¹ a la même signification que D, à l'exception du fait que D¹ ne contient pas de substituant G, ou représente le radical -CH-, =C- ou -N-, et
F¹ a la même signification que F, à l'exception du fait que F¹ ne contient pas de substituant G, ou représente le radical -CH- ou =C-,
ainsi que leurs sels avec des bases inorganiques et/ou organiques, des aminoacides ou des amides d'aminoacides, à la condition qu'au moins deux des substituants Z représentent le radical -CH₂COOY et que le composé macrocyclique répondant à la formule générale I ne contienne pas plus qu'un radical G, éventuellement avec les additifs courants en pharmacie galénique.

6. Utilisation d'au moins un composé physiologiquement compatible répondant à la formule générale I 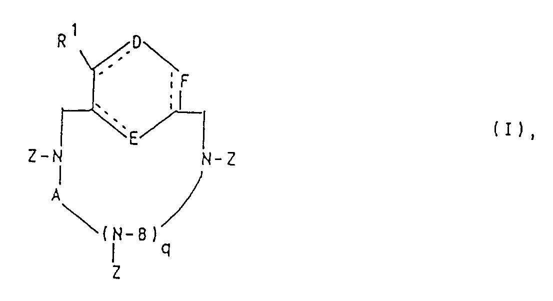 dans laquelle
.... représente une simple ou double liaison,
q représente les chiffres 0 à 5,
A et B, qui sont identiques ou différents, représentent chacun un groupe alkylène linéaire ou ramifié comportant 2 à 6 atomes de carbone,
D représente un atome d'azote ou d'oxygène ou le groupe =C=O, =NR², -CHR³- ou =CR³-,
E représente un atome d'azote, de soufre ou d'oxygène, ou le groupe 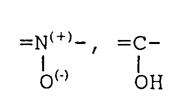 ou >NR⁴,
F représente (CHR⁸-)ₙ ou (=CR⁸)ₙ,
R¹ représente un atome d'hydrogène ou d'halogène ou un groupe alkyle en C₁-C₆,
Z représente un atome d'hydrogène ou le groupe -CH₂COOY, où Y a la signification d'un atome d'hydrogène et/ou d'un équivalent d'ion métallique d'un élément des nombres atomiques 21-29, 31, 32, 37-39, 42-44, 49 ou 57-83,
R² représente un atome d'hydrogène ou un groupe alkyle en C₁-C₆,
R³ représente un atome d'hydrogène ou d'halogène ou un groupe phényle ou alkyle en C₁-C₆, qui est éventuellement substitué par un ou plusieurs groupes phényle et/ou hydroxy, le radical OR⁵, le substituant 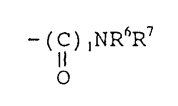 ou le substituant G,
R⁴ représente un groupe hydroxy, R² ou un groupe alkyle en C₁-C₆ éventuellement hydroxylé ou carboxylé,
R⁵ représente un radical alkyle en C₁-C₆ éventuellement substitué par un ou plusieurs groupes phényle et/ou hydroxy,
R⁶ et R⁷ représentent, indépendamment l'un de l'autre, des atomes d'hydrogène, le radical R⁵, des radicaux phényle ou benzyle éventuellement substitués par 1 à 3 groupes hydroxy, R⁶ et R⁷ pouvant former conjointement avec l'atome d'azote un noyau pentagonal ou hexagonal saturé ou insaturé, contenant éventuellement un autre atome d'azote, d'oxygène, ou de soufre ou un groupe carbonyle, ce noyau étant éventuellement substitué par 1 à 3 radicaux R⁵, un des substituants R⁶ ou R⁷ pouvant représenter le radical 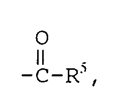
R⁸ représente R¹ ou G,
l représente les chiffres 0 ou 1,
n représente les chiffres 0 ou 1,
G représente un deuxième macrocycle lié par une liaison directe, un groupe bis(carbonylamino) (-NH-CO-CO-NH-) ou un groupe alkylène en C₁-C₂₀ qui porte éventuellement aux extrémités des groupes carbonyle (>CO) ou carbonylamino (-NH-CO) ou des atomes d'oxygène et contient éventuellement un ou plusieurs atomes d'oxygène, Z, des groupes imino substitués par de l'acyle ou de l'hydroxyacyle ou une à deux doubles et/ou triples liaisons carbone-carbone, et qui présentent la formule générale II 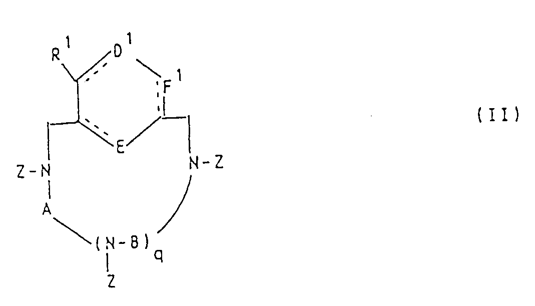
dans laquelle
D¹ a la même signification que D à l'exception du fait que D¹ ne contient pas de substituant G, ou représente le radical 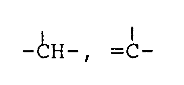 ou 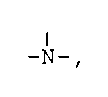 et
F¹ a la même signification que F, à l'exception du fait que F¹ ne contient pas de substituant G, ou représente le radical 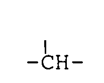 ou 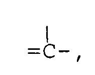
ainsi que de leurs sels avec des bases inorganiques et/ou organiques, des aminoacides ou des amides d'aminoacides,
à la condition qu'au moins deux des substituants Z représentent le radical -CH₂COOY, que le composé macrocyclique de la formule générale I ne contienne pas plus qu'un radical G et qu'au moins deux des substituants Y représentent des équivalents d'ions métalliques d'au moins un élément des nombres atomiques 21-29, 42, 44 ou 57-83 ou au moins d'un radionucléïde d'un élément des nombres atomiques 27, 29, 31, 32, 37-39, 43, 49, 62, 64, 70 ou 77,
pour la préparation de produits de diagnostic à RMN et aux rayons X, de radiodiagnostic, de radiothérapie ou de thérapie par des rayons.

7. Procédé de préparation de composes macrocycliques répondant à la formule générale I 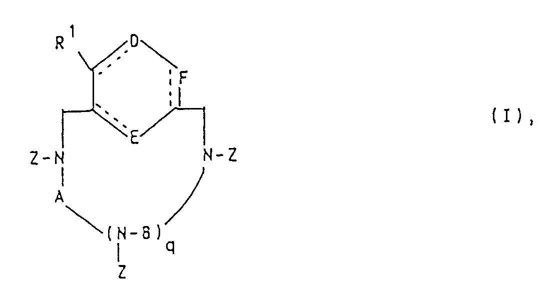 dans laquelle
.... représente une simple ou double liaison,
q représente les chiffres 0 à 5,
A et B, qui sont identiques ou différents, représentent chacun un groupe alkylène linéaire ou ramifié comportant 2 à 6 atomes de carbone,
D représente un atome d'azote ou d'oxygène ou le groupe =C=O, =NR², -CHR³- ou =CR³-,
E représente un atome d'azote, de soufre ou d'oxygène, ou le groupe 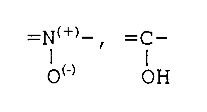 ou >NR⁴,
F représente (-CHR⁸-)ₙ ou (=CR⁸)ₙ,
R¹ représente un atome d'hydrogène ou d'halogène ou un groupe alkyle en C₁-C₆,
Z représente un atome d'hydrogène ou le groupe -CH₂COOY, où Y représente un atome d'hydrogène et/ou un équivalent d'ion métallique d'un élément des nombres atomiques 21-29, 31, 32, 37-39, 42-44, 49 ou 57-83,
R² représente un atome d'hydrogène ou un groupe alkyle en C₁-C₆,
R³ représente un atome d'hydrogène ou d'halogène ou un groupe phényle ou alkyle en C₁-C₆, qui est éventuellement substitué par un ou plusieurs groupes phényle et/ou hydroxy, le radical OR⁵, le substituant 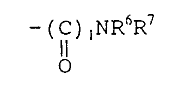 ou le substituant G,
R⁴ représente un groupe hydroxy, R² ou un groupe alkyle en C₁-C₆ éventuellement hydroxylé ou carboxylé,
R⁵ représente un radical alkyle en C₁-C₆ éventuellement substitué par un ou plusieurs groupes phényle et/ou hydroxy,
R⁶ et R⁷ représentent, indépendamment l'un de l'autre, des atomes d'hydrogène, le radical R⁵, des radicaux phényle ou benzyle éventuellement substitués par 1 à 3 groupes hydroxy, R⁶ et R⁷ pouvant former conjointement avec l'atome d'azote un noyau hexagonal ou pentagonal saturé ou insaturé, contenant éventuellement un autre atome d'azote, d'oxygène, ou de soufre ou un groupe carbonyle, ce noyau étant éventuellement substitué par 1 à 3 radicaux R⁵, un des substituants R⁶ ou R⁷ pouvant représenter le radical 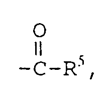
R⁸ représente R¹ ou G,
l représente les chiffres 0 ou 1,
n représente les chiffres 0 ou 1,
G représente un deuxième macrocycle qui est lié par une liaison directe, un groupe bis(carbonylamino) (-NH-CO-CO-NH-) ou un groupe alkylène en C₁-C₂₀ qui porte éventuellement aux extrémités des groupes carbonyle (>CO) ou carbonylamino (-NH-CO) ou des atomes d'oxygène et contient éventuellement un ou plusieurs atomes d'oxygène, Z, des groupes imino substitués par de l'acyle ou de l'hydroxyacyle ou une à deux doubles et/ou triples liaisons carbone-carbone, et qui répond à la formule générale II 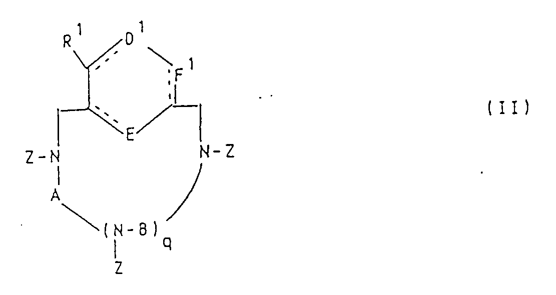
dans laquelle
D¹ a la même signification que D à l'exception du fait que D¹ ne contient pas de substituant G, ou représente le radical 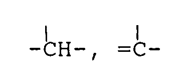 ou 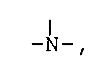 et
F¹ a la même signification que F, à l'exception du fait que F¹ ne contient pas de substituant G, ou représente le radical 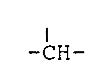 ou 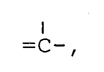
ainsi que de leurs sels avec des bases inorganiques et/ou organiques, des aminoacides ou des amides d'aminoacides,
à la condition qu'au moins deux des substituants Z représentent -CH₂COOY, que le composé macrocyclique répondant à la formule générale I ne contienne pas plus qu'un radical G et que la formule générale I ne représente pas de l'acide 3,6,9,12,18-pentaazabicyclo-[12.3.1]octadéca-1(18),14,16-triène-N-tétraacétique ou de l'acide 3,6,9,15-tétraazabicyclo[9.3.1]pentadéca-1(15),11,13-triène-N-triacétique ou leurs complexes de Cu, de Pb, de Co et de Sr,
caractérisé en ce qu'on alkyle d'une manière connue en soi des composés de la formule générale I' 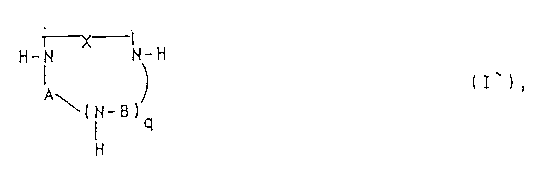 où
X représente 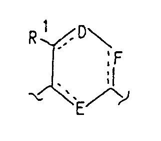 ou un noyau pentagonal ou hexagonal à transformer en le noyau souhaité,
avec un composé halogéné III
HalCH₂COOY' (III),
dans laquelle Hal représente du chlore, du brome ou de l'iode et
Y' représente un atome d'hydrogène ou un groupe de protection d'acide,
et ensuite, éventuellement après transformation de X en le noyau pentagonal ou hexagonal souhaité du produit final ainsi qu'éventuellement après séparation des groupes de protection Y', on fait réagir éventuellement les complexants ainsi obtenus de la formule générale I où Y a la signification de l'hydrogène, d'une manière connue en soi, avec au moins un oxyde métallique ou un sel métallique d'un élément des nombres atomiques 21-29, 31, 32, 37-39, 42-44, 49 ou 57-83, et ensuite, au cas où cela est nécessaire, on substitue des atomes d'hydrogène acide encore présents par des cations de bases inorganiques et/ou organiques, d'aminoacides ou d'amides d'aminoacides.

8. Procédé de préparation des produits pharmaceutiques suivant la revendication 5, caractérisé en ce qu'on amène le composé complexe dissous ou mis en suspension dans de l'eau ou une solution saline physiologique, éventuellement avec les additifs courants en pharmacie galénique, sous une forme appropriée pour l'application entérale ou parentérale.
